# EUROPEAN PATENT SPECIFICATION

(11) **EP 3 197 560 B1**
(45) Date of publication and mention of the grant of the patent: **06.03.2019**
(21) Application number: 15843248.4
(22) Date of filing: 25.09.2015
(51) Int. Cl.: A61Q 5/12, C11D 3/50, A61K 8/11, A61K 8/84, B01J 13/14

(54) **CAPSULE AGGREGATES**
KAPSELAGGREGATE
AGRÉGATS DE CAPSULE

(30) Priority: 26.09.2014 US 201462056106 P
(43) Date of publication of application: 02.08.2017
(73) Proprietor: International Flavors & Fragrances Inc., New York, NY 10019 (US)
(72) Inventor: SASAKI, Takashi, Morganville New Jersey 07751 (US); WANG, Chii-Fen, Princeton, New Jersey 08540 (US); PLUYTER, Johan Gerwin Lodewijk, Lindenhurst Illinois 60046 (US); LIN, Yuchuan, East Brunswick, New Jersey 08816 (US); KUNZEL, Crystal, Barnegat, New Jersey 08005 (US)
(74) Representative: Barker Brettell LLP
(86) International application number: PCT/US2015/052221
(87) International publication number: WO 2016/049456

(56) References cited:
- EP-A2- 1 719 554
- WO-A1-2011/056904
- WO-A1-2014/085286
- US-A1- 2013 330 292
- US-B2- 7 538 079
- US-B2- 7 915 214

## Description

### BACKGROUND

Nano- or micro-capsules are useful to deliver active materials to a target area in a time-delayed or controlled manner. In these capsules, an active material (e.g., a fragrance, flavor, and malodor counteracting agent) is encapsulated inside polymeric capsule walls.

Combined with cationic deposition aids, these capsules have been applied in many rinse-off products such as shampoos and conditioners. Cationic deposition aids in these products improve the deposition of capsules, usually negatively charged, onto hairs and skins. See US 20130330292, US 20130337023, and US 7,538,078. These cationic deposition aids bind to both the anionic capsules and target surfaces, and thus help the deposition of the capsules.

Nevertheless, the interactions between the anionic capsules and the cationic deposition aids can be weakened or even canceled out when both cationic and anionic ions are abundant in products such as hair conditioners. The weakened interactions lead to poor adhesion performance of the capsules. Further, these capsules are often found unstable in the products as a result of spontaneous, uncontrolled flocculation of the capsules. The two shortcomings result in poor capsule performance such as low fragrance intensity in ionic environment.

There is a need to improve the stability of capsules and their performance including fragrance intensity and adhesion to applied areas.

### SUMMARY OF THE INVENTION

This invention is based on an unexpected discovery that certain capsule aggregates possess desirable properties including improved deposition and high perceived olfactory intensity. These aggregates can be prepared by a controlled coacervation process.
Accordingly, one aspect of this invention relates to aggregates each containing two or more benefit particles, one or more binder polymers, and one or more deposition polymers. Each aggregate is neutral or carries one or more net positive charges. It typically has a particle size of 1 to 200 µm(e.g., 1 to 150 µm and 5 to 120 µm). In the aggregate, the polymer combination theoretical charge-to-weight ratio can be 0 to +0.2 (preferably 0 to +0.1; more preferably +0.01 to +0.1; and even more preferably, 0 to +0.02).
The polymer combination theoretical charge-to-weight ratio as used herein refers to the average theoretical charge per polymer molecular weight. For instance, a sodium alginate useful to prepare the aggregate is a copolymer of guluronic acid and mannuronic acid at a molar ratio of 1 : 1. It has a polymer combination theoretical charge-to-weight ratio of -0.00505 calculated as follows: [0.5 x (charges of guluronic acid) + 0.5 x (charges of mannuronic acid)] / (average monomer unit molecular weight), where each of guluronic acid mannuronic acid has a negative charge, and their average monomer unit molecular weight is 198. As another example, 90% hydrolyzed polyvinylformamide useful to prepare the aggregate contains 90% vinyl amine units and 10% vinyl formamide. It has an polymer combination theoretical charge-to-weight ratio of +0.0197 calculated as follows: [0.9 x (charges of vinyl amine) + 0.1 x (charges of vinyl formamide)] / (average monomer unit molecular weight), where vinyl amine has a charge of +1, vinyl formamide has a charge of 0, and the average monomer unit molecular weight is 0.9 x molecular weight of vinyl amine + 0.1 x molecular weight of vinyl formamide = 0.9 x 43 + 0.1 x 71 = 45.8. As still another example, a polymer mixture contains 0.01 grams of sodium alginate and 10 grams of 90% hydrolyzed polyvinyl formamide. The polymer mixture has a polymer combination theoretical charge-to-weight ratio of +0.196 calculated as follows: (0.01 x (-0.00505) + 10 x 0.0197)/(0.01 + 10) = +0.0196.
The benefit particles each have a particle size of 0.1 to 50 µm (e.g., 0.5 to 30 µm), and contain an active material and a polymeric material. The active material is selected from the group consisting of fragrances, flavoring agents, fungicide, brighteners, antistatic agents, wrinkle control agents, fabric softener actives, hard surface cleaning actives, skin and/or hair conditioning agents, antimicrobial actives, UV protection agents, insect repellants, animal/vermin repellents, flame retardants, and a mixture thereof.

Both the binder and deposition polymers have a molecular weight of 5,000 to 10,000,000 Daltons (preferably, 10,000 to 1,000,000 Daltons and, more preferably, 100,000 to 750,000 Daltons).

The binder polymers each have an anionic chemical group that is negatively charged or capable of being negatively charged at a pH of 2 or higher (e.g., 4 or higher, 6 or higher, 7 or higher, 2-8.5, 3-5, and 5-8). Examples of the anionic chemical group include a carboxyl group (-COOH), a phenolic group (Ar-OH, Ar being an aromatic group), a sulfonic group (-SO₃H), a sulfinic group (-SO₂H), and an organophosphorus group such as -PO(OH)₂, -P(O)(OR)OH, -OP(OH)₂, and -OP(O)(OR)OH, R being C₁-C₁₀ an aliphatic or heteroaliphatic group or an aromatic or heteroaromatic group. Suitable binder polymers include, but are not limited to, polymers and copolymers of acrylic acid, crotonic acid, methacrylic acid, maleic anhydride, ethylene maleic anhydride, an acrylate, a methacrylate, an acrylamide, a sulfonated monomer (i.e., a monomer having a sulfonate group -SO₂OH), a sulfated monomer (i.e., a monomer having a sulfate group -OSO₂OH), or a phenolic monomer (i.e., a monomer having phenolic group -PhOH, Ph is a substituted or unsubstituted phenyl group). Preferably, the binder polymer is a carboxymethyl cellulose, an alginic acid, xanthan gum, carrageenan, agar, carboxyl ethyl cellulose, gum arabic, bacterial alginate, fucogalactan, fucoidan, gellan gum, gum ghatti, gum karaya, gum tragacanth, pectin, propylene glycol alginate, psyllium seed gum, sodium alginate, welan gum, and a combination thereof.

The term "aliphatic" herein refers to a saturated or unsaturated, linear or branched, acyclic, cyclic, or polycyclic hydrocarbon moiety. Examples include, but are not limited to, alkyl, alkylene, alkenyl, alkenylene, alkynyl, alkynylene, cycloalkyl, cycloalkylene, cycloalkenyl, cycloalkenylene, cycloalkynyl, and cycloalkynylene moieties. The term "alkyl" or "alkylene" refers to a saturated, linear or branched hydrocarbon moiety, such as methyl, methylene, ethyl, ethylene, propyl, propylene, butyl, butylenes, pentyl, pentylene, hexyl, hexylene, heptyl, heptylene, octyl, octylene, nonyl, nonylene, decyl, decylene, undecyl, undecylene, dodecyl, dodecylene, tridecyl, tridecylene, tetradecyl, tetradecylene, pentadecyl, pentadecylene, hexadecyl, hexadecylene, heptadecyl, heptadecylene, octadecyl, octadecylene, nonadecyl, nonadecylene, icosyl, icosylene, triacontyl, and triacotylene. The term "alkenyl" or "alkenylene" refers to a linear or branched hydrocarbon moiety that contains at least one double bond, such as -CH=CH-CH₃ and -CH=CH-CH₂-. The term "alkynyl" or "alkynylene" refers to a linear or branched hydrocarbon moiety that contains at least one triple bond, such as -C≡C-CH₃ and -C≡C-CH₂-. The term "cycloalkyl" or "cycloalkylene" refers to a saturated, cyclic hydrocarbon moiety, such as cyclohexyl and cyclohexylene. The term "cycloalkenyl" or "cycloalkenylene" refers to a non-aromatic, cyclic hydrocarbon moiety that contains at least one double bond, such as cyclohexenyl cyclohexenylene. The term "cycloalkynyl" or "cycloalkynylene" refers to a non-aromatic, cyclic hydrocarbon moiety that contains at least one triple bond, cyclooctynyl and cyclooctynylene.

The term "heteroaliphatic" herein refers to an aliphatic moiety containing at least one heteroatom selected from N, O, P, B, S, Si, Sb, Al, Sn, As, Se, and Ge.

The term "aryl" herein refers to a C₆ monocyclic, C₁₀ bicyclic, C₁₄ tricyclic, C₂₀ tetracyclic, or C₂₄ pentacyclic aromatic ring system. Examples of aryl groups include, but are not limited to, phenyl, phenylene, naphthyl, naphthylene, anthracenyl, anthracenylene, pyrenyl, and pyrenylene. The term "heteroaryl" herein refers to an aromatic 5-8 membered monocyclic, 8-12 membered bicyclic, 11-14 membered tricyclic, and 15-20 membered tetracyclic ring system having one or more heteroatoms (such as O, N, S, or Se). Examples of heteroaryl groups include, but are not limited to, furyl, furylene, fluorenyl, fluorenylene, pyrrolyl, pyrrolylene, thienyl, thienylene, oxazolyl, oxazolylene, imidazolyl, imidazolylene, benzimidazolyl, benzimidazolylene, thiazolyl, thiazolylene, pyridyl, pyridylene, pyrimidinyl, pyrimidinylene, quinazolinyl, quinazolinylene, quinolinyl, quinolinylene, isoquinolyl, isoquinolylene, indolyl, and indolylene.

Unless specified otherwise, aliphatic, heteroaliphatic, oxyaliphatic, alkyl, alkylene, alkenyl, alkenylene, alkynyl, alkynylene, cycloalkyl, cycloalkylene, cycloalkenyl, cycloalkenylene, cycloalkynyl, cycloalkynylene, heterocycloalkyl, heterocycloalkylene, heterocycloalkenyl, heterocycloalkenylene, aryl, and heteroaryl mentioned herein include both substituted and unsubstituted moieties. Possible substituents on cycloalkyl, cycloalkylene, cycloalkenyl, cycloalkenylene, cycloalkynyl, cycloalkynylene, heterocycloalkyl, heterocycloalkylene, heterocycloalkenyl, heterocycloalkenylene, aryl, and heteroaryl include, but are not limited to, C₁-C₁₀ alkyl, C₂-C₁₀ alkenyl, C₂-C₁₀ alkynyl, C₃-C₂₀ cycloalkyl, C₃-C₂₀ cycloalkenyl, C₃-C₂₀ heterocycloalkyl, C₃-C₂₀ heterocycloalkenyl, C₁-C₁₀ alkoxy, aryl, aryloxy, heteroaryl, heteroaryloxy, amino, C₁-C₁₀ alkylamino, C₂-C₂₀ dialkylamino, arylamino, diarylamino, C₁-C₁₀ alkylsulfonamino, arylsulfonamino, C₁-C₁₀ alkylimino, arylimino, C₁-C₁₀ alkylsulfonimino, arylsulfonimino, hydroxyl, halo, thio, C₁-C₁₀ alkylthio, arylthio, C₁-C₁₀ alkylsulfonyl, arylsulfonyl, acylamino, aminoacyl, aminothioacyl, amido, amidino, guanidine, ureido, thioureido, cyano, nitro, nitroso, azido, acyl, thioacyl, acyloxy, carboxyl, and carboxylic ester. On the other hand, possible substituents on aliphatic, heteroaliphatic, oxyaliphatic, alkyl, alkylene, alkenyl, alkenylene, alkynyl, and alkynylene include all of the above-recited substituents except C₁-C₁₀ alkyl. Cycloalkyl, cycloalkylene, cycloalkenyl, cycloalkenylene, heterocycloalkyl, heterocycloalkylene, heterocycloalkenyl, heterocycloalkenylene, aryl, and heteroaryl can also be fused with each other.

Turning to the deposition polymers, each of them has a cationic chemical group that is positively charged or capable of being positively charged at a pH of 10 or lower (*e.g.,* 8 or lower, 7 or lower, 5 or lower, 2-8.5, 3-5, and 5-8). The cationic chemical group can be an amine group, a pyridine group, an imidazole group, an amide group, or an ethylenimine group. Examples of the deposition polymers include polymers and copolymers of one of the following monomers: diallyldimethylammonium chloride, methacrylamide propyltrimethylammonium chloride, N,N-dimethylaminoethyl methacrylate, vinyl pyridine, quaternized vinyl pyridine, vinyl amine, allyl amine, vinyl imidazoline, vinyl imidazole, vinyl imidazolinium, dimethylaminoethyl methacrylate, dimethylamino-propyl, methacryloylaminopropyl lauryldimonium chloride, amino-functionalized silicone, and ethylenimine.

In the aggregate, the benefit particles bind to each other through one of the binder polymers or one of the deposition polymers. The binding is either achieved via a covalent or non-covalent bond. One of the binder polymers also bonds to one of the deposition polymers via a covalent or non-covalent bond. In some embodiments, each of the benefit particles is negatively charged and bonds to one of the deposition polymers via one or more hydrogen bonds, one or more dipolar interactions, or one or more ionic interactions, and one of the binder polymers bonds to one of the deposition polymers via one or more ionic interactions. In certain embodiments, the benefit particles each are positively charged and bond to one of the binder polymers via ionic interactions, and one of the binder polymers bonds to the deposition polymers also via an ionic interaction.

The aggregate of this invention can further contain a salt that has a multivalent ion, e.g., a sulfate salt. The weight ratio between the salt and the capsules can be in the range of 0.00002 : 1 to 0.2 : 1 (*e.g.*, 0.00004 : 1 to 0.02 : 1, 0.00008 : 1 to 0.02 : 1, and 0.0001 : 1 to 0.01 : 1).

The aggregate of this invention can also contain a viscosity stabilizer such as cetyl trim ethyl ammonium-based surfactant (*e.g*., cetyl trimethyl ammonium chloride and cetyl trimethyl ammonium bromide), dodecyltrimethylammonium chloride, dodecyltrimethylammonium bromide, benzyldimethylhexadecylammonium chloride, benzyldimethylhexadecylammonium bromide, trimethyloctadecylammonium chloride, and trimethyloctadecylammonium bromide.

The covalent bond is typically via a cross-linker selected from the group consisting of a urea-formaldehyde precondensate, a melamine-formaldehyde precondensate, an aldehyde-based cross-linker, a water soluble carbodiimide, a polyfunctional aziridine, a polyfunctional epoxy, a polyfunctional oxazoline, a polyfunctional acrylate, a polyfunctional methacrylate, a polyfunctional cyano acrylate, a polyfunctional isocyanate, a polyfunctional acrylamide, a polyfunctional acetyl acetonate, a polyfunctional levulinate, and a combination thereof. The weight ratio of the cross-linker and the capsules is 0.00002 : 1 to 10 : 1 (*e.g.,* 0.0002 : 1 to 10 : 1, 0.002 : 1 to 5 : 1, and 0.01 : 1 to 5 : 1).

In any of the aggregates described above, each of the benefit particles can be a capsule that has a core containing an active material and a wall encapsulating the active material. The wall of the capsule is formed of a urea-formaldehyde polymer, a melamine-formaldehyde polymer, a phenolic-formaldehyde polymer, a urea-glutaraldehyde polymer, a melamine-glutaraldehyde polymer, a phenolic-glutaraldehyde polymer, polyurea, polyurethane, polyacrylate, polyamide, polyester, an epoxy cross-linked polymer, a polyfunctional carbodiimide cross-linked polymer, silica, a silica-derived material, and a combination thereof. In other embodiments, the binder polymers, the deposition polymers, or both can be a part of the capsule wall, i.e., embedded in the capsule wall. The embedding can be achieved by preparing the capsules from an emulsion that includes these polymers, e.g., as a dispersant.

Another aspect of this invention relates to a process of preparing an aggregate. The process includes the steps of: (a) providing a benefit particle dispersion containing benefit particles in water, each of the benefit particles containing an active material and a polymeric material immobilizing the active material, (b) adding one or more binder polymers and one or more deposition polymer to obtain a polymeric mixture, each of the binder polymers having an anionic chemical group that is negatively charged or capable of being negatively charged, and each of the deposition polymers having a cationic chemical group that is positively charged or capable of being positively; and (c) causing formation of the aggregate in the polymeric mixture induced by an coacervation event, the coacervation event being pH adjusting, heating, UV irradiating, sonicating, radio irradiating, ionizing, exposing to an enzyme or bacterium, addition of salt or non-solvent, a chemical reaction, or a combination thereof. Preferably, the coacervation event is pH adjusting by adding an acid or a base. Examples of the acid include lactic acid, hydrochloric acid, acetic acid, citric acid, sulfuric acid, nitric acid, or a combination. Suitable base include potassium hydroxide, sodium carbonate, sodium bicarbonate, ammonium hydroxide, triethylamine, pyridine, and a combination thereof. The benefit particles, binder polymers, and deposition polymers are defined above. The molar ratio between the cationic group and the anionic group can be 1 : 1 to 10000 : 1 (*e.g.,* 1 : 1 to 5000 : 1, 1.01 : 1 to 5000 : 1 and 1.01 : 1 to 3000 : 1), in which the aggregate has one or more positive charges

Also within the scope of this invention is an aggregate prepared by the process described above.

Yet within the scope of this invention is a consumer product containing any aggregate described above and, optionally, one or more different aggregate, a free active material, one or more free capsules containing an active material, or a combination thereof. The consumer product can be a shampoo, hair conditioner, personal wash, fabric detergent, fabric softener, or hard surface cleaner.

The details of one or more embodiments of the invention are set forth in the description and drawings below. Other features, objects, and advantages of the invention will be apparent from the description, drawings, and the claims.

### BRIEF DESCRIPTION OF THE DRAWINGS

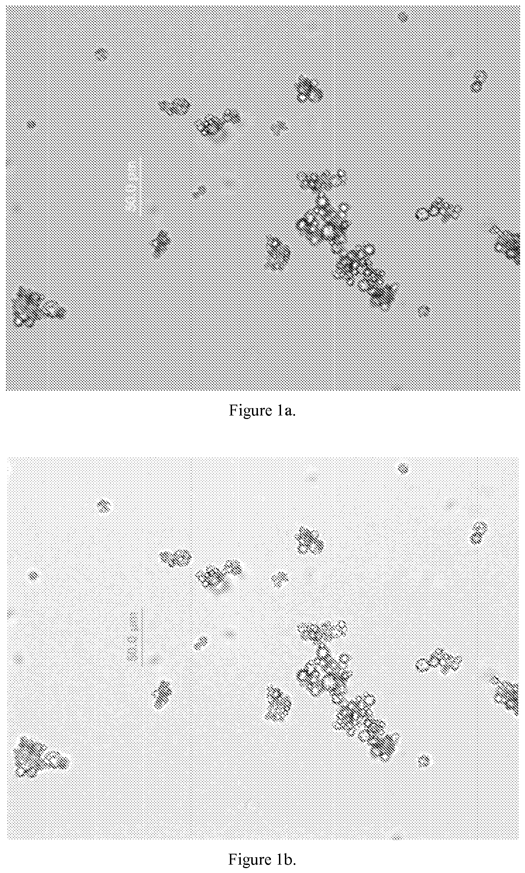

Figures 1(a)-(c) show the microscope images of two aggregates (Aggregates 1 and 2) of the invention and the polyurea (PU) capsule used to prepare the two aggregates: (a) Aggregate 1, (b) Aggregate 2, and (c) the PU capsule.

### DETAILED DESCRIPTION OF THE INVENTION

As described above, the aggregates each contain a plurality of benefit particles, one or more binder polymers, and one or more deposition polymers.

The binder polymer is capable of binding to the deposition polymer. The binding results in the formation of the aggregates induced by a coacervation event, which can be pH adjusting, heating, UV irradiating, sonicating, radio irradiating, ionizing, exposing to an enzyme or bacterium, addition of salt or non-solvent, a chemical reaction, or a combination thereof.

A skilled person in the art can choose a coacervation event and determine coacervation parameters to obtain aggregates having a desirable particle size and charges. The particle size herein refers to the longest dimension of an aggregate, e.g., the diameter of a spherical aggregate and the length of an angular aggregate.

The binder polymer has an anionic group that can bound to the benefit particle surface and/or the deposition polymer via a non-covalent or covalent interaction. Other than the polymers mentioned in the summary section above, additional suitable binder polymers include polymers or copolymer of anionic monomers such as sulfonated sulfonate and sulfonated naphthalene polycondensate. The content of the binder polymer can be from 0.001% to 15%, more preferably from 0.1% to 10% by the weight of the aggregate.

Non-covalent interactions between the binder polymer and the benefit particle surface and the deposition polymer include (i) electrostatic interactions such as ionic interactions, hydrogen bonds, and halogen bonds; (ii) Van der Waals forces such as dipole-dipole interactions, dipole-induced dipole interactions, and London dispersion forces; (iii) π-effects such as π-π interactions, cation-π and anion-π interactions, and polar-π interactions; and (iv) hydrophobic effect, i.e., the desire for hydrophobic polymers and/or capsules to aggregate in an aqueous environment.

The covalent interaction includes ether bonds (-O-), ester bonds (-COO-); carbon-carbon single bonds, carbon-carbon double bonds, thioether bonds (-S-), disulfide bonds (-S-S-), thioester bonds (-CO-S-, -CS-O-, and -CSS-), amine bonds including secondary, tertiary and quaternary amines, imine bonds (-CR=N-), hydroxylamine bonds (-O-NR-), amide bonds (-CONR-, R being H or a substitute such as an aliphatic, heteroaliphatic, aryl, or heteroaryl group), urea bonds (-NR-CO-NR'-, R', independently from R, being H or a substitute such as an aliphatic, heteroaliphatic, aryl, or heteroaryl group), carbamate bonds (-OCONR-), organic carbonate bonds (-OCOO-), sulfoxide bonds (-SO-), sulfonyl bonds (-SO₂-), sulfonamide bonds (-SO₂NR-), and organophosphate bonds.

Some covalent interactions among the capsules, the binder polymers, and the deposition polymers are through covalent crosslinkers. Suitable crosslinkers include melamine-formaldehyde precondensates (which can have any degree of methylol, alkoxy, and imino content so long as water solubility and weak acid curing is maintained, such as methylated high imino melamine crosslinkers Cymel 328, 385, and 373, commercially available from Cytec Industries); urea-formaldehyde precondensates; aldehydic crosslinkers containing formaldehyde, glutaraldehyde and acetaldehyde; water soluble carbodiimides such as N-Cyclohexyl-N'-(2-morpholinoethyl)carbodiimide metho-p-toluenesulfonate, N-(3 -Dimethylaminopropyl)-N'-ethylcarbodiimide, polymeric carbodiimides Zoldine XL-29SE and UCARlink (both commercially available from the Dow Chemical); polyfunctional aziridines (the aziridine crosslinkers are preferred to be used with nucleophilic deposition aids containing O, N, or S containing molecules such as alcohols, amines, and thiols); polyfunctional epoxy-based materials such as 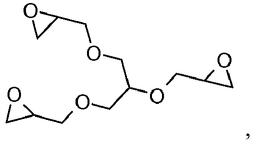 polyalkylene oxide including ethylene glycol diglycidyl ether, 1,4-butanediol diglycidyl ether (e.g., HELOXY Modifier 67 commercially available from Hexion), trimethylolpropane triglycidyl ether (e.g., HELOXY Modifier 48 commercially available from Hexion), neopentyl glycol diglycidyl ether (e.g., HELOXY Modifier 68 commercially available from Hexion), cyclohexane dimethanol diglycidyl ether (e.g., HELOXY Modifier 107 commercially available from Hexion), and glycerol triglycidyl ether (commercially available from Polysciences), a polymeric crosslinker containing an epoxy reactive group, such as Resin 4190, Resin 2023, and Kymene 557H (reaction products of epichlorohydrin and polyamide; commercially available from Hercules); reactive silicones containing a glycidyl ether group; and polyfunctional oxazoline crosslinkiers such as EPOCROS WS and K series (commercially available from Nippon Shokubai) and the APR-200 series (commercially available from Advanced Polymer Inc.).

In some embodiments, anionic polymers in the deposition polymer blend with cationic functional polymers can be used to bind the capsules, binder polymers, deposition polymers via ionic interactions. One example is a water-soluble polymer containing one or more carboxylic acid groups. Common carboxylic acid-containing polymers are copolymers based on acrylic acid having the structure shown below: 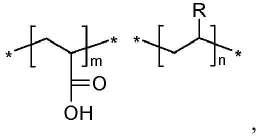 wherein R can be almost any functional group based on alkyl or aromatic carbon, or heteroatoms such as N, O, or S. Other anionic polymers suitable for ionic crosslinking include acrylic acid and methacrylic acid based polymers such as Acrylidone 1005 (polymer/copolymer of N-vinylpyrrolidone and acrylic acid; commercially available from ISP), Acudyne SCP (an anionic terpolymer of acrylamide, acrylamidomethyl propanesulfonic acid sodium salt, and methacrylic acid; commercially available from Rohm & Haas), Amphomer LV-71 (an octylacrylamide/ acrylates/butylaminoethyl methacrylate copolymer; commercially available from the National Starch), Aquaflex XL-30 (an isobutylene/dimethylaminopropyl maleimide /ethoxylated maleimide/maleic acid copolymer; commercially available from ISP), Easysperse (a poly(methylvinylether/maleic acid)- butyl ethyl ester; commercially available from ISP), Fixomer A-30 (a methacrylic acid/sodium acrylamidomethyl propane sulfonate copolymer; commercially available from Nalco), I-Rez 160 (an isobutylene/maleic anhydride copolymer; commercially available from Ashland), the Merquat series such as 2001 (an amphoteric terpolymer consisting of acrylic acid, methacrylamidopropyl trimethyl ammonium chloride and methyl acrylate), 2003PR (an ampholytic terpolymer consisting of methacryla-midopropyl trimethyl ammonium chloride, acrylamide and acrylic acid) and Merquat Plus 3330 / Merquat Plus 3331 / Merquat 3333 (amphoteric terpolymers consisting of acrylic acid, diallyl dimethylammonium chloride and acrylamide; commercially available from Lubrizol), Scripset 520 (copolymer of styrene and maleic anhydride; commercially available from Hercules), and Superfloc A2875M and A-370M (copolymer of acrylic acid and acrylamide; commercially available from Kemira), Gantrez S-95 and S-97 copolymers of methyl vinyl ether/maleic acid; commercially available from Ashland), ethyl, butyl and isopropyl esters of methylvinyl ether/maleic acid copolymers such as Gantrez SP-215, ES-225, A-425, ES-425, ES-435 and ES-335 (commercially available from Ashland), Zemac E400 (an alternating copolymer of ethylene and maleic anhydride; commercially available from Vertellus), polymers containing sulfonate or sulphate groups such as polystryrene sulfonate (Flexan II, commercially available from AkzoNobel), formaldehyde-naphthalene polycondensates (e.g., Morwet D425, commercially available from AkzoNobel), carrageenan (Ticaloid products from TIC Gums Inc.), and amino methyl propyl sulfonate monomer based polymers such as Acumer 3100 (commercially available from DOW Chemical Corp) and Aquatreat AR 546 (commercially available from AkzoNobel Corp).

Bio-based polymers can also be used for ionic crosslinking. Examples include polysaccharides (alcohol and carboxylic acid containing) such as alginate and xanthan, and (ii) proteins containing carboxylic acid, amine and thiol functional groups via, for instance, glutamic acid, aspartic acid, lysine and cysteine.

Other ionic crosslinking polymer include amine-containing polymers such as polyvinyl amine, polyethylene imine, ethoxylated polyethylene imine and polyamido amines, alcohol-containing polymers such as poly vinyl alcohol, and acrylics polymers containing functional monomers such as hydroxyethyl acrylate.

When the benefit particle is a capsule, the binder polymer can a part of the capsule wall, i.e., embedded in the wall. The embedding occurs when the binder polymer is used during the preparation of the capsules. See, e.g., US Patent Application Publication 2013/0337023. As described in US 2013/0337023, a polyurea capsule is prepared by emulsifying an oil phase into an aqueous phase in the presence of a dispersant and subsequently forming a capsule wall through an interfacial polymerization between a polyisocyanate and an amine cross-linker. When the binder polymer is used as the dispersant, it can become a part of the capsule wall.

In some embodiments, the binder polymer is not a part of the capsule wall but bonds to or coats the capsule surface via a non-covalent or covalent interaction described above.

Turning to the deposition polymer, it has a cationic group that is positively charged or capable of being positively charged. The deposition polymer improves delivery efficiency of fragrance-containing aggregates onto a substrate, such as a skin and hair. In this invention, it also facilitates the formation of aggregates by binding to benefit particles (e.g., capsules) and/or the binder polymer that is attached to one of the benefit particles. Suitable deposition polymers include cationic or amphoteric polymers and copolymers prepared from the following monomers: diallyl dimethyl ammonium chloride (DADMAC), methacrylamidopropyltrimethylammonium chloride (MAPTAC), 2-(dimethylamino) ethyl methacrylate (DAMEMA), vinyl pyridine, quaternized vinyl pyridine, vinyl amine, vinyl imidazoline, vinyl imidazole, vinyl imidazolinium, dimethylaminoethyl methacrylate, dimethylaminopropyl, methacrylamide, methacryloylaminopropyl lauryldimonium chloride (MAPLDAC). Other useful deposition polymers are cationic starches such as Hi-CAT CWS42 (commercially available from Roquette America, Inc.), cationic guars such as Jaguar C-162, cationic amino resins, cationic urea resins, hydrophobic quaternary amines.

The content of the deposition polymer can be from 0.01% to 15%, more preferably from 0.1 % to 10% by the weight of the aggregate.

The combined content of the binder and deposition polymers is preferably in the range of 0.01 % to 15% (e.g., 0.1% to 10%). The weight ratio of the deposition polymer to the binder polymer can be 1 : 500 to 500 : 1 (e.g., 1 : 100 to 100 : 1, 1 : 50 to 50 : 1).

Like the binder polymer, the deposition polymer can be a part of the capsule wall when it is added during the preparation of the capsules, or can be a separate part when it is added after the capsules have been prepared.

Additional capsule deposition aid from 0.01 to 25%, more preferably from 5 to 20% can be included by weight of the capsule. The additional capsule deposition aid can be added during the preparation of the capsules, after the capsules have been made, or after the aggregates have been formed.

These additional deposition aids, together with the deposition polymers, are used to aid in deposition of capsules to surfaces such as fabric, hair or skin. These include anionically, cationically, nonionically, or amphoteric water-soluble polymers. Those skilled in the art would appreciate that the charge of these polymers can be adjusted by changing the pH, depending on the product in which this technology is to be used. Any suitable method for coating the deposition aids onto the encapsulated fragrance materials can be used. The nature of suitable polymers for assisted capsule delivery to interfaces depends on the compatibility with the capsule wall chemistry since there has to be some association to the capsule wall. This association can be through physical interactions, such as hydrogen bonding, ionic interactions, hydrophobic interactions, electron transfer interactions or, alternatively, the polymer coating could be chemically (covalently) grafted to the capsule or particle surface. Chemical modification of the capsule or particle surface is another way to optimize anchoring of the polymer coating to capsule or particle surface. Furthermore, the capsule and the polymer need to be compatible with the chemistry (polarity, for instance) of the desired interface. Therefore, depending on which capsule chemistry and interface (e.g., cotton, polyester, hair, skin, wool), the polymer can be selected from one or more polymers with an overall zero (amphoteric: mixture of cationic and anionic functional groups) or net positive charge, based on the following polymer backbones: polysaccharides, polypeptides, polycarbonates, polyesters, polyolefinic (vinyl, acrylic, acrylamide, poly diene), polyester, polyether, polyurethane, polyoxazoline, polyamine, silicone, polyphosphazine, olyaromatic, poly heterocyclic, or polyionene, with molecular weight (MW) ranging from about 1 ,000 to about 1000,000,000, preferably from about 5,000 to about 10,000,000. As used herein, molecular weight is provided as weight average molecular weight.

Particular examples of cationic polymers that can be used include, e.g., polysaccharides such as guar, alginates, starch, xanthan, chitosan, cellulose, dextrans, arabic gum, carrageenan, and hyaluronates. These polysaccharides can be employed with cationic modification and alkoxy-cationic modifications such as cationic hydroxyethyl or cationic hydroxypropyl. For example, cationic reagents of choice are 3 -chloro-2-hydroxypropyl trimethylammonium chloride or its epoxy version. Another example is graft-copolymers of polyDADMAC on cellulose. Alternatively, polysaccharides can be employed with aldehyde, carboxyl, succinate, acetate, alkyl, amide, sulfonate, ethoxy, propoxy, butoxy, and combinations of these functionalities; or any hydrophobic modification (compared to the polarity of the polysaccharide backbone). The above modifications can be in any ratio and the degree of functionalization can be up to complete substitution of all functionalizable groups, as long as the theoretical net charge of the polymer is zero (mixture of cationic and anionic functional groups) or preferably positive. Furthermore, up to 5 different types of functional groups may be attached to the polysaccharides. Also, polymer graft chains may be differently modified to the backbone. The counterions can be any halide ion or organic counter ion. See U.S. Pat. Nos. 6,297,203 and 6,200,554.

Another source of cationic polymers contain protonatable amine groups so that the overall net charge is zero (amphoteric: mixture of cationic and anionic functional groups) or positive. The pH during use will determine the overall net charge of the polymer. Examples include silk protein, zein, gelatin, keratin, collagen and any polypeptide, such as polylysine.

Further cationic polymers include polyvinyl polymers with up to 5 different types of monomers can be used. The monomers of such polymer have the generic formula:

-C(R₂)(R₁)-CR₂R₃-

wherein, R₁ is H, C₁-C₂₅ alkane, C₁-C₂₅ alkene (in which the number of double bonds ranges from 1-5), C₁-C₂₅ alkoxylated fatty alcohol, or a liquid crystalline moiety that can provide the polymer with thermotropic liquid crystalline properties;
R₂ is H or CH₃; and
R₃ is -Cl, -NH₂ (*i.e.,* polyvinyl amine or its copolymers with N-vinyl formamide.

Such polyvinyl polymers are sold under the name LUPAMIN 9095 by BASF Corporation. Further suitable cationic polymers containing hydroxylalkylvinylamine units, as disclosed in U.S. Pat. No. 6,057,404.

Another class of materials are polyacrylates with up to 5 different types of monomers. Monomers of polyacrylates have the generic formula:

-CH(R₁-C(R₂)(CO-R₃-R₄)-

wherein, R₁ is H, C₁-C₂₅ alkane, C₁-C₂₅ alkene (in which the number of double bonds ranges from 1-5), C₁-C₂₅ alkoxylated fatty alcohol, or a liquid crystalline moiety that can provide the polymer with thermotropic liquid crystalline properties;
R₂ is H or CH₃;
R₃ is a C₁-C₂₅ alkyl alcohol or an alkylene oxide with any number of double bonds, or R₃ may be absent such that the C=O bond is (via the C-atom) directly connected to R₄; and
R₄ is -NH₂, -NHR₁, -NR₁R₂, -NR₁R₂R₆ (where R₆ = R₁, R₂, or -CH₂-COOH or its salt), -NH-C(O)-, sulfobetaine, betaine, polyethylene oxide, poly(ethyleneoxide/ propylene oxide/butylene oxide) grafts with any end group, H, OH, styrene sulfonate, pyridine, quaternized pyridine, alkyl-substituted pyrrolidone or pyridine, pyridine-N-oxide, imidazolinium halide, imidazolium halide, imidazol, piperidine, -OR₁, -OH, - COOH alkali salt, sulfonate, ethoxy sulphate, pyrrolidone, caprolactam, phenyl-R₄ or naphthalene-R₅, where R₄ and R₅ are R₁, R₂, R₃, sulfonic acid or its alkali salt or organic counter ion. Also, glyoxylated cationic polyacrylamides can be used. Typical polymers of choice are those containing the cationic monomer dimethylaminoethyl methacrylate (DMAEMA) or methacrylamidopropyl trimethyl ammonium chloride (MAPTAC). DMAEMA can be found in GAFQUAT and GAFFIX VC-713 polymers from ISP. MAPTAC can be found in BASF's LUVIQUAT PQ11 PN and ISP's GAFQUAT HS100.

Another group of polymers that can be used are those that contain cationic groups in the main chain or backbone. Included in this group are:
i) polyalkylene imines such as polyethylene imine, commercially available as LUPASOL from BASF. Any molecular weight and any degree of crosslinking of this polymer can be used in the present invention;
ii) ionenes as disclosed in U.S. Pat. No. 4,395,541 and U.S. Pat. No. 4,597,962;
iii) adipic acid/dimethyl amino hydroxypropyl diethylene triamine copolymers, such as CARTARETIN F-4 and F-23, commercially available from Sandoz;
iv) polymers of the general formula: -[N(CH₃)₂-(CH₂)ₓ-NH-(CO)-NH-(CH₂)_{y}-N(CH₃)₂)-(CH₂)_{z}-O-(-CH₂)ₚ]ₙ-, with x, y, z, p = 1-12, and n according to the molecular weight requirements. Examples are Polyquaternium-2 (MIRAPOL A-15), Polyquaternium-17 (MIRAPOL AD-1), and Polyquaternium-18 (MIRAPOL AZ-1). Other polymers include cationic polysiloxanes and cationic polysiloxanes with carbon-based grafts with a net theoretical positive charge or equal to zero (mixture of cationic and anionic functional groups). This includes cationic end-group functionalized silicones (*i.e.,* Polyquaternium-80). Silicones with general structure: -Si(R₁)(R₂)-O-]ₓ-[Si(R₃)(R₂)-O-]_{y}- where R₁ is any alkane from C₁-C₂₅ or H with number of double bonds from 0-5, aromatic moieties, polysiloxane grafts, or mixtures thereof. R₁ can also be a liquid crystalline moiety that can provide the polymer with thermotropic liquid crystalline properties. R₂ can be H or CH₃; and R₃ can be -R₁-R₄, where R₄ can be -NH₂, -NHR₁, -NR₁R₂, -NR₁R₂R₆ (where R₆ = R₁, R₂, or -CH₂-COOH or its salt), -NH-C(O)-, -COOH, -COO- alkali salt, any C₁-C₂₅ alcohol, -C(O)-NH₂ (amide), -C(O)-N(R₂)(R₂')(R₂"), sulfobetaine, betaine, polyethylene oxide, poly(ethyleneoxide/propylene oxide/butylene oxide) grafts with any end group, H, -OH, styrene sulfonate, pyridine, quaternized pyridine, alkyl-substituted pyrrolidone or pyridine, pyridine-N-oxide, imidazolinium halide, imidazolium halide, imidazol, piperidine, pyrrolidone, caprolactam, sulfonate, ethoxysulphate phenyl-R₅ or naphthalene-R₆ where R₅ and R₆ are R₁, R₂, R₃, sulfonic acid or its alkali salt or organic counter ion. R₃ can also be -(CH₂)ₓ-O-CH₂-CH(OH)-CH₂-N(CH₃)₂-CH₂-COOH and its salts. Any mixture of these R₃ groups can be selected. X and y can be varied as long as the theoretical net charge of the polymer is zero (amphoteric) or positive. In addition, polysiloxanes containing up to 5 different types of monomeric units may be used. Examples of suitable polysiloxanes are found in U.S. Pat. Nos. 4,395,541 4,597,962 and 6,200,554. Another group of polymers that can be used to improve capsule/particle deposition are phospholipids that are modified with cationic polysiloxanes. Examples of these polymers are found in U.S. Pat. No. 5,849,313, WO Patent Application 95/18096A1 and European Patent No. 0737183B1.

Furthermore, copolymers of silicones and polysaccharides and proteins can be used (e.g., those commercially available as CRODASONE brand products).

Another class of polymers includes polyethylene oxide-co-propyleneoxide-co-butylene oxide polymers of any ethylene oxide/propylene oxide/butylene oxide ratio with cationic groups resulting in a net theoretical positive charge or equal to zero (amphoteric). Examples of such polymers are the commercially available TETRONIC brand polymers.

Suitable polyheterocyclic (the different molecules appearing in the backbone) polymers include the piperazine-alkylene main chain copolymers disclosed by Kashiki and Suzuki (1986) Ind. Eng. Chem. Fundam. 25:120-125.

Table 1 below shows polyquaternium polymers that can be used as deposition aids in this invention.

**TABLE 1. Deposition Aids -- Cationic Polyquaternium Polymers**

| Polyquaternium | Description | Commercial Products |
|---|---|---|
| 1 | Ethanol, 2,2',2"-nitrilotris-, polymer with 1,4-dichloro-2-butene and N,N,N',N'-tetramethyl-2-butene-1,4-diamine | Polyquad (Alcon) |
| 2 | Poly[bis(2-chloroethyl) ether-alt-1,3-bis[3- (dimethylamino)propyl]urea] | Mirapol A-15 |
| 4 | Hydroxyethyl cellulose dimethyl diallylammonium chloride copolymer; Diallyldimethylammonium chloride-hydroxyethyl cellulose copolymer | Celquat L-200, H-100, L-200 |
| 5 | Copolymer of acrylamide and quaternized dimethylammoniumethyl methacrylate | Merquat 5, RETEN (Hercules) |
| 6 | Poly(diallyldimethylammonium chloride) | Merquat 100, 106, Mirapol 100 |
| 7 | Copolymer of acrylamide and diallyldimethylammonium chloride | Merquat 550, 550L, 550PR, S, 7SPR, 740, 2200, Mirapol 550, Polyquart 770/NA, Conditioneze 7 |
| 8 | Methyl and Stearyl Dimethylaminoethyl Methacrylate Quaternized with Dimethyl Sulfate | |
| 9 | Polydimethylaminoethyl Methacrylate Quaternized with Methyl Bromide | |
| 10 | Quaternized hydroxyethyl cellulose | Merquat 10, Celquat SC-230M, SC-240C, SC-140C, Ucare Polymer |
| 11 | Copolymer of vinylpyrrolidone and quaternized dimethylaminoethyl methacrylate | Luviquat PQ 11PN, Gafquat 775N, 440, 734, 775 |
| 12 | 2-Propenoic Acid, 2-Methyl-, Decahydro-1,4-Dimethyl-7-(1-Methylethyl)-1-Phenanthrenyl)Methyl Ester, Polymer with 2-(Diethylamino)Ethyl 2-Methyl-2-Propenoate and Ethyl 2-Methyl-2-Propenoate, compd. with Dimethyl Sulfate | |
| 13 | 2-Propenoic Acid, 2-Methyl-, 2-(Diethyl-amino)Ethyl Ester, Polymer with Ethyl 2-Methyl-2-Propenoate and 9-Octadecenyl 2-Methyl-2-Propenoate, compd. with Dimethyl Sulfate | |
| 14 | Ethanaminium, N,N,N-Trimethyl-2-[(2-Methyl-1-Oxo-2-Propenyl)Oxy]-, Methyl Sulfate, Homopolymer | |
| 15 | Ethanaminium, N,N,N-Trimethyl-2-[(2-Methyl-1-Oxo-2-Propenyl)Oxy]-, Chloride, Polymer with 2-Propenamide | Rohagit KF 720F (Rohm GmbH) |
| 16 | Copolymer of vinylpyrrolidone and quaternized vinylimidazole | Luviquat FC 370, HM 552, Style, FC 550, Excellence |
| 17 | Poly(Oxy-1,2-Ethanediyl (Dimethyliminio)-1,3-Propanediylimino(1,6-Dioxo-1,6-Hexanediyl)Imino-1,3-Propanediyl-(Dimethyliminio)-1,2-Ethanediyl Dichloride | Mirapol AD |
| 18 | Poly[oxy-1,2-ethanediyl(dimethyliminio)-1,3-propanediylimino-(1,6-dioxo-1,6-heptanediyl)imino-1,3-propanediyl-(dimethyliminio)-1,2-ethanediyl dichloride] | Luviquat 500 |
| 19 | Ethenol, polymer with aminomethyloxirane | Arlatone PQ-220 (ICI Americas) |
| 20 | Ethenyl octadecyl ether, polymer with aminomethyloxirane | Arlatone PQ-225 |
| 22 | Copolymer of Acrylic Acid and Diallyldimethylammonium Chloride | Merquat 280, 281, 280SD, 295 |
| 24 | Cellulose, 2-[2-Hydroxy-3-(Trimethylammonio)Propoxy]Ethyl Ether, Chloride (Similar to PQ-10) | Quatrisoft Polymer LM-200 (Dow Chemical) |
| 27 | Hexanediamide, N,N'-bis(3-(Dimethylamino)Propyl)-, Polymer with N,N'-bis(3-Dimethylamino)Propyl Urea and 1,1'-Oxybis(2-Chloroethane), Block | |
| 28 | Copolymer of vinylpyrrolidone and methacrylamidopropyl trimethylammonium | Gafquat HS-100, Conditioneze NT-10 |
| 29 | Chitosan, 2,3-Dihydroxypropyl-2-Hydroxy-3-(Trimethylammonio)Propyl Ether, Chloride | Quaternized Chitosan |
| 30 | Ethanaminium, NCarboxymethyl)-N,N-Dimethyl-2-((2-Methyl-1-Oxo-2-Propenyl)Oxy)-, Inner Salt, Polymer with Methyl 2-Methyl-2-Propenoate | Mexomere PX (Chimex) |
| 31 | 2-Propenenitrile, Homopolymer, Hydrolyzed, Block, Reaction Products with N,N-Dimethyl-1,3-Propanediamine, Di-Et Sulfate-Quaternized | Hypan QT100 (Lipo) |
| 32 | Poly(acrylamide 2-methacryloxyethyl-trimethyl ammonium chloride) | Cosmedia CTC (Cognis GmbH) - PQ-32 + other, Salcare SC92 (Ciba Corp.) PQ-32 + other |
| 33 | Ethanaminium, N,N,N-Trimethyl-2-[1-Oxo-2-Propenyl)Oxy]-, Chloride, Polymer with 2-Propenamide | Lanoquat DES-50, Ultimer CG-200 (Nalco), Sepigel Quat33 (Seppic) - PQ-33 + other |
| 34 | Poly(diethyliminio-1,3-propanediyldi-methyliminio-1,3-propanediyl dibromide) | Mexomere PAK (Chimex) |
| 35 | Ethanaminium, N-carboxymethyl-N,N-dimethyl-2-(2-methyl-1-oxo-2-propenyloxy)-, inner salt, polymer with N,N,N-trimethyl-2-(2-methyl-1-oxo-2-propenyloxy)ethanaminium methyl sulfate | Plex 3074 L (Rohm GmbH) |
| 36 | 2-Propenoic Acid, 2-Methyl-,2-(Dimethylamino)Ethyl Ester, Polymer with Methyl 2-Methyl-2-Propenoate, compd. with Dimethyl Sulfate | Plex 4739L (Rohm GmbH) |
| 37 | N,N,N-Trimethyl-2-[(Methyl-1-Oxo-2-Propenyl)Oxy]Ethanaminium Chloride, Homopolymer | Ultragel 300 (Cognis), Synthalen CN, CR, CU (3V Group), Syntran PC 5320 (Interpolymer) |
| 39 | 2-Propen-1-aminium, N,NDimethyl-N-2-Propenyl-, Chloride, Polymer with 2-Propenamide and 2-Propenoic Acid | Merquat 3940, PLUS-3330, PLUS-3331, 3331PR |
| 42 | Poly[oxyethylene(dimethyliminio)ethylene (dimethylimino)ethylene dichloride] | Busan 1507 (Buckman Labs) |
| 43 | polymeric quaternary ammonium salt formed from acrylamide, acrylamidopropyltrimonium chloride, 2-amidopropylacrylamide sulfonate, and DMAPA monomers | Genamin PQ 43 (Clariant Functional Chemicals), Bozequat 4000 (Clariant) |
| 44 | Poly(2-oxopyrrolidin-1-ylethylene, 3-methylimidazolium-1-ylethylene methyl sulfate) | Luviquat Ultracare, MS 370 (BASF), Softenol PQ44 (Zdchimmer & Schwarz Italianat S.p.A) |
| 45 | Glycine, N-methyl-N-[2-[(2-methyl-1-oxo-2-propenyl)oxy]ethyl]-, polymer with 2-(dimethylamino)ethyl 2-methyl-2-propenoate, compound with dimethyl sulfate | Plex 3073L (Rohm GmbH) |
| 46 | 1H-Imidazolium, 1-Ethenyl-3-Methyl-, Methyl Sulfate, Polymer with 1-Ethenyl-hexahydro-2H-Azepin-2-one and 1-Ethenyl-2-Pyrrolildinone | Luviquat Hold |
| 47 | 1-Propanaminium, N,N,NTrimethyl-3 -((2-Methyl-1-Oxo-2-Propenyl)Amino)-, Chloride, Polymer with Methyl 2-Propenoate and 2-Propenoic Acid | Merquat 2001, 2001N |
| 48 | Polymeric quaternary ammonium salt of formed from methacryloyl ethyl betaine, 2-hydroxyethyl methacrylate and methacryloyl ethyl trimethyl ammonium chloride | Plascize L-450 (Goo Chemical) |
| 49 | polymeric quaternary ammonium salt formed by the reaction of methacryloyl ethyl betaine, PEG-9 methacrylate and methacryloyl ethyl trimethyl ammonium chloride | Plascize L-440 (Goo Chemical) |
| 50 | Carboxylatoethyldimethylammonioethyl 2-methyl-2-propenoate homopolymer | Plascize L-401 (Goo Chemical) |
| 51 | 3,5,8-Triox-4-Phosphaundec-10-en-1-aminium, 4-Hydroxy-N,N,N,10-Tetramethyl-9-Oxo, Inner Salt, 4-Oxide, Polymer with Butyl 2-Methyl-2-Propenoate | Lipidure PMB (NOF) |
| 53 | Acrylic Acid/Acrylamide/Methacrylamidopropyltrimonium Chloride Copolymer | Merquat 2003PR |
| 54 | Aspartic acid, polymer with C6-18 alkylamine, 3-dimethylaminopropylamine and sodium chloroacetate | Quilty-Hy (Mitsui) |
| 55 | 1-Dodecanaminium, N,NDimethyl-N-[3-[(2-Methyl-1-Oxo-2-Propenyl)-AminoPropyl]-, Chloride, Polymer with N-[3-(Dimethylamino)Propyl]-2-Methyl-2-Propenamide and 1-Ethenyl-2-Pyrrolidinone | Styreze W |
| 56 | 5-Isocyanato-1-(isocyanato methyl)-1,3,3-trimethylcyclohexane, polymer with 1,3-butanediol and bis(2-hydroxyethyl)dimethylammonium methyl sulfate | Hairrol UC-4 (Sanyo Chemical) |
| 57 | 12-Hydroxy-9(Z)-octadecenamidopropyl-trimethylammonium chloride, polymers with ricinus communis (castor) oil, isooctdecanoic acid and butandioic acid | Zenigloss Q (Zenitech) |
| 58 | 2-Propenoic Acid, Methyl Ester, Polymer with 2,2-Bis[(2-Propenyloxy)Methyl]-1-Butanol and Diethenylbenzene, Reaction Products with N,NDimethyl-1,3-Propanediamine, Chloromethane-Quaternized | Lowenol Conditioner PWW (Lowenstein)-PQ-58 and Wheat Protein |
| 59 | Poly(20,25-dioxo-2,5,10,15,18-pentamethyl-10-(2-hydroxy-3-(3-(3-phenyl-2-propenamido)propyldimethylammonio)propyl)-10-azonia-1,4,7,13,16,19-hexaoxa-pentacosanediyl) chloride | Crodasorb UV-HPP (Croda, Inc.) - PQ-59 and Butylene Glycol |
| 60 | 9-Octadecenoic Acid, 12-Hydroxy-, [(2-Hydroxyethyl)-Imino]Di-2,1-Ethanediyl Ester, Polymer with 5-Isocyanato-1-(Isocyanatomethyl)-1,3,3-Trimethylcyclohexane, Compd. with Diethyl Sulfate | Polylipid PPI-RC (Alzo/Bernel) - PQ-60 and Propylene Glycol |
| 61 | 2-Methyl-2-propenoyloxyethyl N,N,N-trimethylammonioethyl phosphate inner salt, polymer with octadecyl 2-methyl-2-propenoate | Lipidure-S (NOF) |
| 62 | Polymeric quaternary ammonium salt of butyl methacrylate, polyethylene glycol methyl ether methacrylate, ethylene glycol dimethacrylate and 2-methacryloylethyl trimonium chloride with 2,2'-azobis(2-methyl propionamidine)dihydrochloride | Nanoaquasome (Amore Pacific/Kyung-do) |
| 63 | polymeric quaternary ammonium salt formed by acrylamide, acrylic acid and ethyltrimonium chloride acrylate | Finquat (Innospec), Octacare PQ63 (Innospec Edison, NJ), OF-308 (WSP Chemical & Technology) |
| 64 | 2-Methyl-2-propenoyloxyethyl N,N,N-trimethylammonioethyl phosphate inner salt, polymer with 2-hydroxy-3-(2-methyl-2-propenoyl)oxypropyltrimethylammonium chloride | Lipidure-C (NOF) |
| 65 | 2-Methyl-2-propenoyloxyethyl N,N,N-trimethylammonioethyl phosphate inner salt, polymer with butyl 2-methyl-2-propenoate and sodium 2-methyl-2-propenoate | Lipidure-A (NOF) |
| 66 | 5-Isocyanato-1-(isocyanato methyl)-1,3,3-trimethylcyclohexane, polymer with di(hydroxypolymethylene) benzene-dicarboxylate and ethylbis(2-hydroxyethyl)methylammonium ethyl sulfate | WBR-2925C (Taisei) - PQ-66 and Methyl Pyrrolidone |
| 67 | 2-Hydroxyethyl cellulose ether, reaction products with N,N,N-trimethyl-N-oxiranylmethylammonium chloride and N-dodecyl-N,N-dimethyl-N-oxiranylmethylammonium chloride | Softcat (Dow Chemical) |
| 68 | 1-Ethenyl-2-pyrrolidinone, polymer with 1-ethenylimidazole and 1-ethenyl-3 - methylimidazolium methyl sulfate | Luviquat Supreme |
| 69 | polymeric quaternary ammonium salt composed of vinyl caprolactam, vinylpyrrolidone, dimethylaminopropyl methacrylamide (DMAPA), and methacryloylaminopropyl lauryldimonium chloride | Aquastyle 100, 300 (ISP) |
| 70 | polymeric quaternary ammonium salt consisting of an ethoxylated, propoxylated stearyl amine condensed with adipic acid and dilinoleic acid and quaternized with dimethyl sulfate | Lustreplex (Croda) |
| 71 | | ColaMoist 300P (Colonial Chemical Inc) |
| 72 | polymeric quaternary ammonium salt of hydroxethylcellulose reacted with a coco-alkyl dimethyl ammonium substituted epoxide | Mirustyle CP (Croda) |
| 73 | polymeric quaternary ammonium salt consisting of propyltrimonium chloride acrylamide, ethyltrimonium chloride methacrylate and dimethylacrylamide monomers; Propanaminium, N,N,N-trimethyl-3-(2-propenamido)-, chloride, polymer with N,N,N-trimethyl-2-(2-methyl-2-propenoyloxy)ethanaminium chloride and N,N-dimethyl-2-propenamide | Diaformer C-802, C-823 (Mitsubishi Chem), Diasleek C-802, C-823 (Mitsubishi Chem) |
| 74 | | Mirapol PB 20 (Rhodia) Polycare Boost (Rhodia) |
| 75 | O-(2-Hydroxy-2-trimethylammonio-propyl)starch chloride, reaction products with O-(3-dodecyldimethylammonio-2-hydroxypropyl)starch chloride | Amylomer Cat 220EMU (Grafe Chemie) |
| 76 | | Mirapol AT-1 (Rhodia) |
| 77 | Cocoglucoside Crosspolymer Hydroxypropyltrimonium Chloride | Colonial Poly SugaQuat TM-8610P (Colonial Chemical Inc) |
| 78 | Decylglucoside Crosspolymer Hydroxypropyl Laurdimonium Chloride | Colonial Poly SugaQuat L-1010P (Colonial Chemical Inc) |
| 79 | Decylglucoside Crosspolymer Hydroxypropyl Steardimonium Chloride | Colonial Poly SugaQuat S-1010P (Colonial Chemical Inc) |
| 80 | Laurylglucoside Crosspolymer Hydroxypropyl Laurdimonium Chloride | Colonial Poly SugaQuat L-1210P (Colonial Chemical Inc) |
| 81 | Laurylglucoside Crosspolymer Hydroxypropyl Steardimonium Chloride | Colonial Poly SugaQuat S-1210P (Colonial Chemical Inc) |
| 82 | Laurylglucoside Crosspolymer Hydroxypropyltrimonium Chloride | Colonial Poly SugaQuat TM-1218P (Colonial Chemical Inc) |
| 84 | polymeric quaternary ammonium salt of acrylamidopropyltrimethylammonium chloride, trimethylaminoethyl methacrylate, dimethylacrylamide and hydroxyethylmethacrylate | Diasleek C-824 (Mitsubishi Chemical) |
| 85 | polymeric quaternary ammonium salt of acrylamidopropyltrimethylammonium chloride, dimethylacrylamide and hydroxyethylmethacrylate | Diasleek C-825 (Mitsubishi Chemical) |
| 86 | polymeric quaternary ammonium salt of vinylpyrrolidone, 1-methyl-3-vinylimidazoline chloride, vinylimidazole and methacrylic acid | Luvigel Advanced (BASF) |
| 87 | polymeric quaternary ammonium salt of vinylpyrrolidone, vinylimidazole and diallyldimethyl ammonium chloride | Luviquat Sensation (BASF) |
| 88 | Poly(Dilinoleyldimonium hydroxypropyl)chlorides) | ColaQuat PDQ (Colonial Chemical Inc) |
| 89 | polymeric quaternary ammonium salt prepared by the reaction of t-butyl acrylate, vinyl pyrolidone, dimethylaminopropyl methacrylamide, methacrylic acid and ethyldimethyl[2-[(2-methyl-1-oxoallyl)oxy]ammonium ethyl sulfate, neutralized with orthophosphoric acid | (BASF) |
| 90 | polymeric quaternary ammonium salt of acrylamide and hydroxyethylcellulose quaternized with diallyldimethyl ammonium chloride | Hymoquat AK325R (Hymo Corporation) |
| 91 | polymeric quaternary ammonium salt of hydroxypropyl methacrylate and polyethylene glycol methacrylate quaternized with ethyltrimonium chloride methacrylate | Syntran 5500 (Interpolymer) - PQ-91 and PA |
| 92 | GLYCERYLAMIDOETHYL METHACRYLATE/STEARYL METHACRYLATE COPOLYMER | Ceracute-G (NOF) |
| 94 | polymeric quaternary ammonium salt consisting of acrylamide, dimethyl diallyl ammonium chloride and methacrylamidopropyltrimonium chloride monomers | (Toho) |
| 95 | copolymer of Zea Mays (Corn) Starch, Acrylic Acid and acrylamidopropyltrimethylammonium chloride monomers | Polyquart Ecoclean (Cognis) |
| 98 | | (Cognis GmbH) |
| 101 | | Deposilk Q1 (Air Products) |

Other suitable deposition aids include those described in US 2013/0330292, US 2013/0337023, US 2014/0017278.

The capsules useful in this invention can be selected from the following: (i) urea-formaldehyde, melamine-formaldehyde phenolic-formaldehyde, urea-glutaraldehyde, melamine-glutaraldehyde, phenolic-glutaraldehyde, and any combination thereof; (ii) polyurea (isocyanate-based), polyurethane, and any combination thereof; (iii) polyacrylate core-shell capsules, polyurea/polyurethane-acrylic hybrid core-shell capsules, and any combination thereof; (iv) polyamide-based and/or polyester-based capsules; (v) capsules produced using epoxy-crosslinkers; (vi) capsules based on silica and silica-derived materials which are typically produced using sol-gel processes. Some of these capsules are described in greater detail below.

Other than capsules, polymeric particles can also be used to prepare the aggregates of this invention. These particles include those loaded with fragrances onto polymers such as polyacrylates, polyureas (isocyanate-based), polyurethanes, acrylate-based hydrogel particlates, polyurea/polyurethane-acrylic hybrid core-shell particles, polyamides, polyesters, epoxy-based and epoxy crosslinked polymers, ethyl and other alkyl celluloses, polyolefins, and any combination thereof. For examples of these particles, see US Patent Application Publication 20050113267.

The aggregates each can contain multiple different capsules/polymeric particles, e.g., capsules containing different fragrances, and/or capsules having different wall materials, different particle sizes, or different wall thickness or density.

In some embodiments, salts (organic or inorganic) are included in aggregates to induce coacervation and/or improve aggregate stability. Examples include metal fluorides, chlorides, bromides, iodides, acetyl acetonates, nitrates, nitrites, sulfates, hydrogen sulfates, sulfites, bisulfites, carbonates, bicarbonates, borates, phosphates, hydrogen ammonium phosphates, dihydrogen ammonium phosphates, oxide bis(2,4-pentanadionate), sulfate oxides, silicates, antimonates, arsenates, germanates, carboxylates, alkoxides, enolates, phenoxides, and a combination thereof. Metals include, but are not limited to, alkali, alkaline, transition, and post-transition metals, e.g., lithium, sodium, potassium, phosphates, cesium, beryllium, magnesium, calcium, aluminum, titanium, manganese, iron, copper, nickel, zinc, gallium, indium, tin, lead, and bismuth. In addition to metallic salts, ammonium salts are also useful, which contain ammonium ions (NH₄⁺), or primary, secondary, tertiary, or quaternary ammonium cations. The salt can include a multivalent ion, i.e., a cation or anion that has two or more valences. Examples of multivalent ions include cations of Ca, Mg, Al, Fe, Mn, Zn, Co, Cu, Ni, Ti, Cr, and V, anions of sulfate, phosphates, multivalent acids (citric, resorcinol, oligomeric acrylic acids, and boric acid). Chemicals having multifunctional strong hydrogen bonding groups are also suitable to include in the aggregate to induce coacervation and/or improve aggregate stability. These chemicals include pyrrolidones and N-oxide pyridines.

To prepare aggregates of the invention, the benefit particles (e.g., capsules), the deposition polymers, and the binder polymers are mixed and coacervate induced by a coacervation event under controlled conditions. As an illustration, coacervation is achieved by adding an acid solution to the mixture at a pre-determined rate under agitation. To prepare an aggregate having a desirable particle size, stability, and fragrance release profile, a skilled person in the art can readily pick a suitable capsule, binder polymer, deposition polymer, and coacervation event such as pH adjustment using a certain acid. The skilled artisan can also determine the weight ratios among these materials and their contents in the aggregate. Further, it is also important to control the duration and intensity of the coacervation event, e.g., the acid solution concentration and the rate of adding the acid solution to the mixture to induce coacervation.

Unexpectedly, the aggregates prepared by a method of controlled coacervation deliver and deposit active materials more efficiently and have improved stability especially in an ionic environment, e.g., a hair conditioner.

Also within the scope of this invention are methods of preparing the aggregates (see the Summary section) and the aggregates prepared by the methods.

Described below are capsules suitable for preparing the capsule compositions and aggregates of this invention.

### Core-Shell Encapsulation Systems.

The capsules can be prepared following encapsulation procedures known in the art, see for example US Patent Nos. 2,800,457, 3,870,542, 3,516,941, 3,415,758, 3,041,288, 5,112,688, 6,329,057, and 6,261,483. Wall forming materials include melamine formaldehyde, polyurethane, polysiloxanes, polyurea, polyamide, polyimide, polyvinyl alcohol, polyanhydride, polyolefin, polysulfone, polysaccharide, protein, polylactide (PLA), polyglycolide (PGA), polyorthoester, polyphosphazene, silicone, lipid, modified cellulose, gums, polystyrene, and polyesters or combinations of these materials. Other polymeric materials that are functional are ethylene maleic anhydride copolymer, styrene maleic anhydride copolymer, ethylene vinyl acetate copolymer, and lactide glycolide copolymer. Biopolymers that are derived from alginate, chitosan, collagen, dextran, gelatin, and starch can also be used as the encapsulating materials. Additionally, capsules can be made via the simple or complex coacervation of gelatin. Preferred encapsulating wall polymers include those formed from isocyanates, acrylates, acrylamide, acrylate-co-acrylamide, hydrogel monomers, sol-gel precursors, gelatin, melamine-formaldehyde or urea-formaldehyde condensates, as well as similar types of aminoplasts.

### Polyurea/Polyurethane Capsules.

Polyurea capsules can be prepared using multi-functional isocyanates and multi-functional amines. See WO 2004/054362; EP 0 148149; EP 0 017 409 B1; US Patent Nos. 4,417,916, 4,124,526, 4,285,720, 4,681,806, 5,583,090, 6,340,653 6,566,306, 6,730,635, 8,299,011, WO 90/08468, and WO 92/13450.

These isocyanates contain two or more isocyanate (-NCO) groups. Suitable isocyanates include, for example, 1,5-naphthylene diisocyanate, 4,4'-diphenylmethane diisocyanate (MDI), hydrogenated MDI (H12MDI), xylylene diisocyanate (XDI), tetramethylxylol diisocyanate (TMXDI), 4,4'-diphenyldimethylmethane diisocyanate, di- and tetraalkyldiphenylmethane diisocyanate, 4,4'-dibenzyl diisocyanate, 1,3-phenylene diisocyanate, 1,4-phenylene diisocyanate, the isomers of tolylene diisocyanate (TDI), optionally in a mixture, 1-methyl-2,4-diisocyanatocyclohexane, 1,6-diisocyanato-2,2,4-trimethylhexane, 1,6-diisocyanato-2,4,4-trimethylhexane, 1-isocyanatomethyl-3 - isocyanato-1,5,5-trimethylcyclohexane, chlorinated and brominated diisocyanates, phosphorus-containing diisocyanates, 4,4'-diisocyanatophenylperfluoroethane, tetramethoxybutane 1,4-diisocyanate, butane 1,4-diisocyanate, hexane 1,6-diisocyanate (HDI), dicyclohexylmethane diisocyanate, cyclohexane 1,4-diisocyanate, ethylene diisocyanate, phthalic acid bisisocyanatoethyl ester, also polyisocyanates with reactive halogen atoms, such as 1-chloromethylphenyl 2,4-diisocyanate, 1-bromomethylphenyl 2,6-diisocyanate, and 3,3-bischloromethyl ether 4,4'-diphenyldiisocyanate. Sulfur-containing polyisocyanates are obtained, for example, by reacting hexamethylene diisocyanate with thiodiglycol or dihydroxydihexyl sulfide. Further suitable diisocyanates are trimethylhexamethylene diisocyanate, 1,4-diisocyanatobutane, 1,2-diisocyanatododecane and dimer fatty acid diisocyanate.

The multi-functional amines contains two or more amine groups including -NH₂ and -RNH, R being substituted and unsubstituted C₁-C₂₀ alkyl, C₁-C₂₀ heteroalkyl, C₁-C₂₀ cycloalkyl, 3- to 8-membered heterocycloalkyl, aryl, and heteroaryl.

Water soluble diamines are one class of amines useful to prepare polyurea capsules as the amines are usually present in the aqueous phase. One class of such amine is of the type:

H₂N(CH₂)ₙNH₂,

where n is ≥ 1. When n is 1, the amine is a diamine, ethylene diamine. When n is 2, the amine is diamine propane and so on. Exemplary amines of this type include, but are not limited to, ethylenediamine, 1,3-diaminopropane, 1,4-diaminobutane, hexanethylene diamine, hexamethylene diamine, and pentaethylenehexamine. In particular embodiments of this invention, the preferred n is 6, where the amine is a hexamethylene diamine.

Amines that have a functionality greater than 2, but less than 3 and which may provide a degree of cross linking in the shell wall are the polyalykylene polyamines of the type: 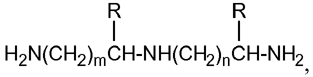 where R equals hydrogen or -CH₃, m is 1-5 and n is 1-5, *e.g.,* diethylene triamine, triethylene tetraamine and the like. Exemplary amines of this type include, but are not limited to diethylenetriamine, bis(3-aminopropyl)amine, bis(hexamethylene)triamine.

Another class of amine that can be used is polyetheramines. They contain primary amino groups attached to the end of a polyether backbone. The polyether backbone is normally based on either propylene oxide (PO), ethylene oxide (EO), or mixed PO/EO. The ether amine can be monoamine, diamine, or triamine, based on this core structure. An example is: 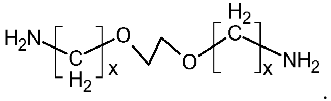 Exemplary polyetheramines include 2,2'-ethylenedioxy)bis (ethylamine) and 4,7,10-trioxa-1,13-tridecanediamine.

Other suitable amines include, but are not limited to, tris(2-aminoethyl)amine, triethylenetetramine, N,N'-bis(3 -aminopropyl)-1,3 -propanediamine, tetraethylene pentamine, 1,2-diaminopropane, N,N,N',N'-tetrakis(2-hydroxyethyl)ethylene diamine, N,N,N',N'-tetrakis(2-hydroxypropyl)ethylene diamine, branched polyethylenimine, 2,4-diamino-6-hydroxypyrimidine and 2,4,6-triaminopyrimidine.

Amphoteric amines, *i.e.,* amines that can react as an acid as well as a base, are another class of amines of use in this invention. Examples of amphoteric amines include proteins and amino acids such as gelatin, L-lysine, L-arginine, L-lysine monohydrochloride, arginine monohydrochloride and ornithine monohydrochloride.

Guanidine amines and guanidine salts are yet another class of amines of use in this invention. Exemplary guanidine amines and guanidine salts include, but are not limited to, 1,3-diaminoguanidine monohydrochloride, 1,1-dimethylbiguanide hydrochloride, guanidine carbonate and guanidine hydrochloride.

Commercially available examples of amines include JEFFAMINE EDR-148 (where x=2), JEFFAMINE EDR-176 (where x=3) (from Huntsman). Other polyether amines include the JEFFAMINE ED Series, and JEFFAMINE TRIAMINES.

Alcohols of use as cross-linking agents typically have at least two nucleophilic centers. Exemplary alcohols include, but are not limited to, ethylene glycol, hexylene glycol, pentaerythritol, glucose, sorbitol, and 2-aminoethanol.

The preparation of polyurethane capsules can be carried out by reacting one or more of the above-referenced isocyanates with a diol or polyol in the presence of a catalyst. Diols or polyols of use in the present invention have a molecular weight in the range of 200-2000. Exemplary diols include, but are not limited to, ethylene glycol, diethylene glycol, propylene glycol, 1,4-butane diol, 1,4 hexane diol, dipropylene glycol, cyclohexyl 1,4 dimethanol, and 1,8 octane diol. Exemplary polyols include, but are not limited to, poly (ethylene glycols), poly (propylene glycols), and poly (tetramethylene glycols).

Suitable catalysts include amino or organometallic compounds and include, for example, 1,4-diazabicyclo[2.2.2]octane (*e.g.,* DABCO, Air Products, Allentown, PA), N,N-dimethylaminoethanol, N,N-dimethylcyclohexylamine, bis-(2-dimethylaminoethyl) ether, N,N dimethylacetylamine, stannous octoate and dibutyltin dilaurate.

### Aminoplasts and Gelatin

A representative process used for aminoplast encapsulation is disclosed in US 3,516,941, though it is recognized that many variations with regard to materials and process steps are possible. Another encapsulation process, i.e., gelatin encapsulation, is disclosed in US 2,800,457. Both processes are discussed in the context of fragrance encapsulation for use in consumer products in US Patent Nos. 4,145,184 and 5,112,688 respectively. Polymer systems are well-known in the art and non-limiting examples of these include aminoplast capsules and encapsulated particles as disclosed in GB 2006709 A; the production of micro-capsules having walls comprising styrene-maleic anhydride reacted with melamine-formaldehyde precondensates as disclosed in US 4,396,670; an acrylic acid-acrylamide copolymer, cross-linked with a melamine-formaldehyde resin as disclosed in US 5,089,339; capsules composed of cationic melamine-formaldehyde condensates as disclosed in US 5,401,577; melamine formaldehyde microencapsulation as disclosed in US 3,074,845; amido-aldehyde resin in-situ polymerized capsules disclosed in EP 0 158 449 A1; etherified urea-formaldehyde polymer as disclosed in US 5,204,185; melamine-formaldehyde microcapsules as described in US 4,525,520; cross-linked oil-soluble melamine-formaldehyde precondensate as described in US 5,011,634; capsule wall material formed from a complex of cationic and anionic melamine-formaldehyde precondensates that are then cross-linked as disclosed in US 5,013,473; polymeric shells made from addition polymers such as condensation polymers, phenolic aldehydes, urea aldehydes or acrylic polymer as disclosed in US 3,516,941; urea-formaldehyde capsules as disclosed in EP 0 443 428 A2; melamine-formaldehyde chemistry as disclosed in GB 2 062 570 A; and capsules composed of polymer or copolymer of styrene sulfonic acid in acid of salt form, and capsules cross-linked with melamine-formaldehyde as disclosed in US 4,001,140.

### Urea-formaldehyde and melamine-formaldehyde Capsules

Urea-formaldehyde and melamine-formaldehyde pre-condensate microcapsule shell wall precursors are prepared by means of reacting urea or melamine with formaldehyde where the mole ratio of melamine or urea to formaldehyde is in the range of from about 10:1 to about 1:6, preferably from about 1:2 to about 1:5. For purposes of practicing this invention, the resulting material has a molecular weight in the range of from 156 to 3000. The resulting material may be used 'as-is' as a cross-linking agent for the aforementioned substituted or un-substituted acrylic acid polymer or copolymer or it may be further reacted with a C₁-C₆ alkanol, *e.g.,* methanol, ethanol, 2-propanol, 3-propanol, 1-butanol, 1-pentanol or 1-hexanol, thereby forming a partial ether where the mole ratio of melamine/urea : formaldehyde : alkanol is in the range of 1: (0.1-6) : (0.1-6). The resulting ether moiety-containing product may be used 'as-is' as a cross-linking agent for the aforementioned substituted or un-substituted acrylic acid polymer or copolymer, or it may be self-condensed to form dimers, trimers and/or tetramers which may also be used as cross-linking agents for the aforementioned substituted or unsubstituted acrylic acid polymers or co-polymers. Methods for formation of such melamine-formaldehyde and urea-formaldehyde pre-condensates are set forth in US Patent Nos. 3,516,846 and 6,261,483, and Lee et al. (2002) J. Microencapsulation 19, 559-569.

Examples of urea-formaldehyde pre-condensates useful in the practice of this invention are URAC 180 and URAC 186, trademarks of Cytec Technology Corp. of Wilmington, DE. Examples of melamine-formaldehyde pre-condensates useful in the practice if this invention, include, but are not limited to, CYMEL U-60, CYMEL U-64 and CYMEL U-65, trademarks of Cytec Technology Corp. of Wilmington, DE. It is preferable to use, as the precondensate for cross-linking, the substituted or un-substituted acrylic acid polymer or co-polymer. In practicing this invention, the range of mole ratios of urea-formaldehyde precondensate/melamine-formaldehyde pre-condensate to substituted/un-substituted acrylic acid polymer/co-polymer is in the range of from 9:1 to 1:9, preferably from 5:1 to 1:5 and most preferably from 2:1 to 1:2.

In one embodiment of the invention, microcapsules with polymer(s) composed of primary and/or secondary amine reactive groups or mixtures thereof and cross-linkers can also be used. See US 2006/0248665. The amine polymers can possess primary and/or secondary amine functionalities and can be of either natural or synthetic origin. Amine-containing polymers of natural origin are typically proteins such as gelatin and albumen, as well as some polysaccharides. Synthetic amine polymers include various degrees of hydrolyzed polyvinyl formamides, polyvinylamines, polyallyl amines and other synthetic polymers with primary and secondary amine pendants. Examples of suitable amine polymers are the LUPAMIN series of polyvinyl formamides available from BASF. The molecular weights of these materials can range from 10,000 to 1,000,000.

Urea-formaldehyde or melamine-formaldehyde capsules can also include formaldehyde scavengers, which are capable of binding free formaldehyde. When the capsules are for use in aqueous media, formaldehyde scavengers such as sodium sulfite, melamine, glycine, and carbohydrazine are suitable. When the capsules are aimed to be used in products having low pH, e.g., fabric care conditioners, formaldehyde scavengers are preferably selected from beta diketones, such as beta-ketoesters, or from 1,3-diols, such as propylene glycol. Preferred beta-ketoesters include alkyl-malonates, alkyl aceto acetates and polyvinyl alcohol aceto acetates.

As indicated, the capsules of this invention can be prepared by conventional methods to encapsulate fragrances. In some embodiments, the fragrance is encapsulated by a polymer in the presence of a capsule formation aid, *e.g.,* a surfactant or dispersant. Classes of protective colloid or emulsifier of use as surfactants or dispersants include maleic-vinyl copolymers such as the copolymers of vinyl ethers with maleic anhydride or acid, sodium lignosulfonates, maleic anhydride/styrene copolymers, ethylene/maleic anhydride copolymers, and copolymers of propylene oxide, ethylenediamine and ethylene oxide, polyvinylpyrrolidone, polyvinyl alcohols, carboxymethyl cellulose, fatty acid esters of polyoxyethylenated sorbitol and sodium dodecylsulfate.

Commercially available surfactants include, but are not limited to, sulfonated naphthalene-formaldehyde condensates such as MORWET D425 (Akzo Nobel); partially hydrolyzed polyvinyl alcohols such as MOWIOLs, *e.g.,* MOWIOL 3-83 (Air Products); ethylene oxide-propylene oxide block copolymers or poloxamers such as PLURONIC, SYNPERONIC or PLURACARE materials (BASF); sulfonated polystyrenes such as FLEXAN II (Akzo Nobel); and ethylene-maleic anhydride polymers such as ZEMAC (Vertellus Specialties Inc.)

Typically, hydrocolloids or adjuvants are used to improve the colloidal stability of the capsule suspension or slurry against coagulation, sedimentation and creaming. As such, such processing aids can also be used in conjunction with the microcapsules of this invention. As used herein, the term "hydrocolloid" refers to a broad class of water-soluble or water-dispersible polymers having anionic, cationic, zwitterionic or nonionic character. In particular embodiments, the capsule suspension includes a nonionic polymer, cationic polymer, anionic polymer, anionic surfactant, or a combination thereof. In certain embodiments, the nonionic polymer is a polyvinylpyrrolidone (PVP), polyvinyl alcohol (PVA) polyethylene glycol (PEG), Polyethylene oxide (PEO), or polyethylene oxide-polypropylene oxide (PEO-PPO), polyethylene oxide-polypropylene oxide-polyethylene oxide (PEO-PPO-PEO). In other embodiments, the cationic polymer is Polyquaterium-6 (polydiallyldimethylammonium chloride), Polyquaternium-11 (vinyl pyrrolidone/dimethylaminoethyl methacrylate copolymer) or Polyquaternium-47 (acrylic acid/methacrylamidopropyl trimethyl ammonium chloride/methyl acrylate terpolymer). In yet other embodiments, the anionic polymer is a polystyrene sulfonic acid, polyacrylic acid, hyaluronic acid, sodium alginate, or sodium carboxymethylcellulose (CMC). In still other embodiments, the anionic surfactant is sodium laureth sulfate (SLS) or a complex ester of phosphoric acid and ethoxylated cosmetic grade oleyl alcohol (*e.g.,* CRODAFOS 010A-SS-(RB)).

Other hydrocolloids useful in the present invention include polycarbohydrates, such as starch, modified starch, dextrin, maltodextrin, and cellulose derivatives, and their quaternized forms; natural gums such as alginate esters, carrageenan, xanthanes, agar-agar, pectins, pectic acid, and natural gums such as gum arabic, gum tragacanth and gum karaya, guar gums and quaternized guar gums; gelatin, protein hydrolysates and their quaternized forms; synthetic polymers and copolymers, such as poly(vinyl pyrrolidone-co-vinyl acetate), poly(vinyl alcohol-co-vinyl acetate), poly((met)acrylic acid), poly(maleic acid), poly(alkyl(meth)acrylate-co-(meth)acrylic acid), poly(acrylic acid-co-maleic acid)copolymer, poly(alkyleneoxide), poly(vinylmethylether), poly(vinylether-co-maleic anhydride), and the like, as well as poly-(ethyleneimine), poly((meth)acrylamide), poly(alkyleneoxide-co-dimethylsiloxane), poly(amino dimethylsiloxane), and their quartenized forms.

The capsule formation aid may also be used in combination with carboxymethyl cellulose and/or a surfactant during processing to facilitate capsule formation. Examples of surfactants that can be used in combination with the capsule formation aid include, but are not limited to, cetyl trimethyl ammonium chloride (CTAC), poloxamers such as PLURONICS (*e.g.,* PLURONIC F127), PLURAFAC (*e.g.,* PLURAFAC F127), or MIRANET-N, saponins such as QNATURALE (National Starch Food Innovation); or a gum Arabic such as Seyal or Senegal. The amount of surfactant present in the capsule slurry can vary depending on the surfactant used. In some embodiments the amount of surfactant is in the range of 0.05 to 0.2 weight percent, in particular when CTAC is employed. In another embodiment, the amount of surfactant is in the range of 1 to 3 weight percent when a saponin or gum arabic is used.
CMC can be used as a dispersant, and at the same time, also as a binder polymer in forming aggregates of this invention. In certain embodiments, the carboxymethyl cellulose polymer has a molecular weight range between 90,000 Daltonss to 1,500,000 Daltonss, more preferably between 250,000 Daltonss to 750,000 Daltonss and most preferably between 400,000 Daltonss to 750,000 Daltonss. The carboxymethyl cellulose polymer has a degree of substitution between 0.1 to 3, preferably between 0.65 to 1.4, and more preferably between 0.8 to 1.0.

The carboxymethyl cellulose polymer is present in the aggregate slurry at a level from 0.1 weight percent to 2 weight percent and more preferably from 0.3 weight percent to 0.7 weight percent.

In some embodiments, CMC-modified aggregates may provide a perceived fragrance intensity increase of greater than 10%, and more preferably an increase of greater than 30% as compared to CMC-modified capsules without coacervation.

The diameter of the capsules useful to produce the aggregates can vary from 100 nanometers to 1000 µm, preferably from 100 nanometers to 50 µm and more preferably from 0.5 to 30 µm. The capsule distribution can be narrow, broad, or multi-modal.

In some embodiments, the capsules are purified before coacervate into aggregates. Purification can be achieved by washing the capsule slurry with water until a neutral pH is achieved. The capsule suspension can be washed using any conventional method including the use of a separatory funnel, filter paper, centrifugation and the like. The capsule suspension can be washed one, two, three, four, five, six, seven, eight, nine, ten or more times until a neutral pH, i.e., pH 7 □ 0.5, is achieved. The pH of the purified capsules can be determined using any conventional method including pH paper, pH indicators, or a pH meter. A capsule suspension is "purified" in that it is 80%, 90%, 95%, 97%, 98% or 99% homogeneous to capsules, from which is removed unwanted impurities and/or starting materials, e.g., polyisocyanate, cross-linking agent and the like. The purification of the capsules can also include the additional step of adding a salt to the capsule suspension prior to the step of washing the capsule suspension with water. Exemplary salts of use in this step of the invention include, but are not limited to, sodium chloride, potassium chloride or bi-sulphite salts.

*Active materials.* Active materials include, but are not limited to, flavors and fragrances. Suitable fragrances include without limitation, any combination of fragrance oil, essential oil, plant extract or mixture thereof that is compatible with, and capable of being encapsulated by a polymer. Individual perfume ingredients that can be included in the capsules of this invention include fragrances containing:
i) hydrocarbons, such as, for example, 3-carene, α-pinene, β-pinene, α-terpinene, γ-terpinene, p-cymene, bisabolene, camphene, caryophyllene, cedrene, farnesene, limonene, longifolene, myrcene, ocimene, valencene, (E,Z)-1,3,5-undecatriene, styrene, and diphenylmethane;
ii) aliphatic alcohols, such as, for example, hexanol, octanol, 3-octanol, 2,6-dimethylheptanol, 2-methyl-2-heptanol, 2-methyl-2-octanol, (E)-2-hexenol, (E)- and (Z)-3-hexenol, 1-octen-3-ol, a mixture of 3,4,5,6,6-pentamethyl-3/4-hepten-2-ol and 3,5,6,6-tetramethyl-4-methyleneheptan-2-ol, (E,Z)-2,6-nonadienol, 3,7-dimethyl-7-methoxy-octan-2-ol, 9-decenol, 10-undecenol, 4-methyl-3-decen-5-ol, aliphatic aldehydes and their acetals such as for example hexanal, heptanal, octanal, nonanal, decanal, undecanal, dodecanal, tridecanal, 2-methyloctanal, 2-methylnonanal, (E)-2-hexenal, (Z)-4-heptenal, 2,6-dimethyl-5-heptenal, 10-undecenal, (E)-4-decenal, 2-dodecenal, 2,6,10-trimethyl-5,9-undecadienal, heptanal-diethylacetal, 1,1-dimethoxy-2,2,5-trimethyl-4-hexene, and citronellyl oxyacetaldehyde;
iii) aliphatic ketones and oximes thereof, such as, for example, 2-heptanone, 2-octanone, 3-octanone, 2-nonanone, 5-methyl-3-heptanone, 5-methyl-3-heptanone oxime, 2,4,4,7-tetramethyl-6-octen-3-one, aliphatic sulfur-containing compounds, such as for example 3-methylthiohexanol, 3-methylthiohexyl acetate, 3-mercaptohexanol, 3-mercaptohexyl acetate, 3-mercaptohexyl butyrate, 3-acetylthiohexyl acetate, 1-menthene-8-thiol, and aliphatic nitriles (*e.g.,* 2-nonenenitrile, 2-tridecenenitrile, 2,12-tridecenenitrile, 3,7-dimethyl-2,6-octadienenitrile, and 3,7-dimethyl-6-octenenitrile);
iv) aliphatic carboxylic acids and esters thereof, such as, for example, (E)- and (Z)-3-hexenylformate, ethyl acetoacetate, isoamyl acetate, hexyl acetate, 3,5,5-trimethylhexyl acetate, 3-methyl-2-butenyl acetate, (E)-2-hexenyl acetate, (E)- and (Z)-3-hexenyl acetate, octyl acetate, 3-octyl acetate, 1-octen-3-yl acetate, ethyl butyrate, butyl butyrate, isoamyl butyrate, hexylbutyrate, (E)- and (Z)-3-hexenyl isobutyrate, hexyl crotonate, ethylisovalerate, ethyl-2-methyl pentanoate, ethyl hexanoate, allyl hexanoate, ethyl heptanoate, allyl heptanoate, ethyl octanoate, ethyl-(E,Z)-2,4-decadienoate, methyl-2-octinate, methyl-2-noninate, allyl-2-isoamyl oxyacetate, and methyl-3,7-dimethyl-2,6-octadienoate;
v) acyclic terpene alcohols, such as, for example, citronellol; geraniol; nerol; linalool; lavandulol; nerolidol; farnesol; tetrahydrolinalool; tetrahydrogeraniol; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol 2,6-dimethyl-2,5,7-octatrien-1-ol; as well as formates, acetates, propionates, isobutyrates, butyrates, isovalerates, pentanoates, hexanoates, crotonates, tiglinates and 3-methyl-2-butenoates thereof;
vi) acyclic terpene aldehydes and ketones, such as, for example, geranial, neral, citronellal, 7-hydroxy-3,7-dimethyloctanal, 7-methoxy-3,7-dimethyloctanal, 2,6,10-trimethyl-9-undecenal, α-sinensal, β-sinensal, geranylacetone, as well as the dimethyl- and diethylacetals of geranial, neral and 7-hydroxy-3,7-dimethyloctanal;
vii) cyclic terpene alcohols, such as, for example, menthol, isopulegol, alpha-terpineol, terpinen-4-ol, menthan-8-ol, menthan-1-ol, menthan-7-ol, borneol, isoborneol, linalool oxide, nopol, cedrol, ambrinol, vetiverol, guaiol, and the formates, acetates, propionates, isobutyrates, butyrates, isovalerates, pentanoates, hexanoates, crotonates, tiglinates and 3-methyl-2-butenoates of alpha-terpineol, terpinen-4-ol, methan-8-ol, methan-1-ol, methan-7-ol, borneol, isoborneol, linalool oxide, nopol, cedrol, ambrinol, vetiverol, and guaiol;
viii) cyclic terpene aldehydes and ketones, such as, for example, menthone, isomenthone, 8-mercaptomenthan-3-one, carvone, camphor, fenchone, α-ionone, β-ionone, α-n-methylionone, β-n-methylionone, α-isomethylionone, β-isomethylionone, alpha-irone, α-damascone, β-damascone, β-damascenone, δ-damascone, γ-damascone, 1-(2,4,4-trimethyl-2-cyclohexen-1-yl)-2-buten-1-one, 1,3,4,6,7,8a-hexahydro-1,1,5,5-tetramethyl-2H-2,4a-methanonaphthalen-8(5H-)-one, nootkatone, dihydronootkatone; acetylated cedarwood oil (cedryl methyl ketone);
ix) cyclic alcohols, such as, for example, 4-tert-butylcyclohexanol, 3,3,5-trimethylcyclohexanol, 3-isocamphylcyclohexanol, 2,6,9-trimethyl-Z2,Z5,E9-cyclododecatrien-1-ol, 2-isobutyl-4-methyltetrahydro-2H-pyran-4-ol;
x) cycloaliphatic alcohols, such as, for example, alpha, 3,3-trimethylcyclo-hexylmethanol, 2-methyl-4-(2,2,3-trimethyl-3-cyclopent-1-yl)butanol, 2-methyl-4-(2,2,3-trimethyl-3-cyclopent-1-yl)-2-buten-1-ol, 2-ethyl-4-(2,2,3-trimethyl-3-cyclopent-1-yl)-2-buten-1-ol, 3 -methyl-5 -(2,2,3 -trimethyl-3 -cyclopent-1-yl)-pentan-2-ol, 3-methyl-5-(2,2,3-trimethyl-3-cyclopent-1-yl)-4-penten-2-ol, 3,3-dimethyl-5-(2,2,3-trimethyl-3-cyclopent-1-yl)-4-penten-2-ol, 1 -(2,2,6-trimethylcyclohexyl)pentan-3-ol, 1-(2,2,6-trimethylcyclohexyl)hexan-3 -ol;
xi) cyclic and cycloaliphatic ethers, such as, for example, cineole, cedryl methyl ether, cyclododecyl methyl ether;
xii) (ethoxymethoxy)cyclododecane; alpha-cedrene epoxide, 3a,6,6,9a-tetramethyldodecahydronaphtho[2,1-b]furan, 3a-ethyl-6,6,9a-trimethyldodecahydronaphtho[2,1-b]furan, 1,5,9-trimethyl-13-oxabicyclo[10.1.0]-trideca-4,8-diene, rose oxide, 2-(2,4-dimethyl-3-cyclohexen-1-yl)-5-methyl-5-(1-methylpropyl)-1,3-dioxan;
xiii) cyclic ketones, such as, for example, 4-tert-butylcyclohexanone, 2,2,5-trimethyl-5-pentylcyclopentanone, 2-heptylcyclopentanone, 2-pentylcyclopentanone, 2-hydroxy-3 -methyl-2-cyclopenten-1-one, 3 -methyl-cis-2-penten-1-yl-2-cyclopenten-1-one, 3-methyl-2-pentyl-2-cyclopenten-1-one, 3-methyl-4-cyclopentadecenone, 3-methyl-5 -cyclopentadecenone, 3 -methylcyclopentadecanone, 4-(1-ethoxyvinyl)-3,3,5,5 -tetra-methylcyclohexanone, 4-tert-pentylcyclohexanone, 5-cyclohexadecen-1-one, 6,7-dihydro-1,1,2,3,3-pentamethyl-4(5H)-indanone, 5-cyclohexadecen-1-one, 8-cyclohexadecen-1-one, 9-cycloheptadecen-1-one, cyclopentadecanone, cycloaliphatic aldehydes, such as, for example, 2,4-dimethyl-3-cyclohexene carbaldehyde, 2-methyl-4-(2,2,6-trimethyl-cyclohexen-1-yl)-2-butenal, 4-(4-hydroxy-4-methylpentyl)-3-cyclohexene carbaldehyde, 4-(4-methyl-3 -penten-1-yl)-3 -cyclohexene carbaldehyde;
xiv) cycloaliphatic ketones, such as, for example, 1-(3,3-dimethylcyclohexyl)-4-penten-1-one, 1 -(5,5-dimethyl-1-cyclohexen-1-yl)-4-penten-1-one, 2,3,8,8-tetramethyl-1,2,3,4,5,6,7,8-octahydro-2-naphtalenyl methyl-ketone, methyl-2,6,10-trimethyl-2,5,9-cyclododecatrienyl ketone, tert-butyl-(2,4-dimethyl-3-cyclohexen-1-yl)ketone;
xv) esters of cyclic alcohols, such as, for example, 2-tert-butylcyclohexyl acetate, 4-tert-butylcyclohexyl acetate, 2-tert-pentylcyclohexyl acetate, 4-tert-pentylcyclohexyl acetate, decahydro-2-naphthyl acetate, 3-pentyltetrahydro-2H-pyran-4-yl acetate, decahydro-2,5,5,8a-tetramethyl-2-naphthyl acetate, 4,7-methano-3a,4,5,6,7,7a-hexahydro-5 or 6-indenyl acetate, 4,7-methano-3a,4,5,6,7,7a-hexahydro-5 or 6-indenyl propionate, 4,7-methano-3a,4,5,6,7,7a-hexahydro-5 or 6-indenyl-isobutyrate, 4,7-methanooctahydro-5 or 6-indenyl acetate;
xvi) esters of cycloaliphatic carboxylic acids, such as, for example, allyl 3-cyclohexyl-propionate, allyl cyclohexyl oxyacetate, methyl dihydrojasmonate, methyl jasmonate, methyl 2-hexyl-3-oxocyclopentanecarboxylate, ethyl 2-ethyl-6,6-dimethyl-2-cyclohexenecarboxylate, ethyl 2,3,6,6-tetramethyl-2-cyclohexenecarboxylate, ethyl 2-methyl-1, 3 -dioxolane-2 -acetate;
xvii) aromatic and aliphatic alcohols, such as, for example, benzyl alcohol, 1-phenylethyl alcohol, 2-phenylethyl alcohol, 3-phenylpropanol, 2-phenylpropanol, 2-phenoxyethanol, 2,2-dimethyl-3-phenylpropanol, 2,2-dimethyl-3-(3-methylphenyl)-propanol, 1,1-dimethyl-2-phenylethyl alcohol, 1,1-dimethyl-3-phenylpropanol, 1-ethyl-1-methyl-3-phenylpropanol, 2-methyl-5-phenylpentanol, 3-methyl-5-phenylpentanol, 3-phenyl-2-propen-1-ol, 4-methoxybenzyl alcohol, 1-(4-isopropylphenyl)ethanol;
xviii) esters of aliphatic alcohols and aliphatic carboxylic acids, such as, for example, benzyl acetate, benzyl propionate, benzyl isobutyrate, benzyl isovalerate, 2-phenylethyl acetate, 2-phenylethyl propionate, 2-phenylethyl isobutyrate, 2-phenylethyl isovalerate, 1-phenylethyl acetate, α-trichloromethylbenzyl acetate, α,α-dimethylphenylethyl acetate, alpha, alpha-dimethylphenylethyl butyrate, cinnamyl acetate, 2-phenoxyethyl isobutyrate, 4-methoxybenzyl acetate, araliphatic ethers, such as for example 2-phenylethyl methyl ether, 2-phenylethyl isoamyl ether, 2-phenylethyl-1-ethoxyethyl ether, phenylacetaldehyde dimethyl acetal, phenylacetaldehyde diethyl acetal, hydratropaaldehyde dimethyl acetal, phenylacetaldehyde glycerol acetal, 2,4,6-trimethyl-4-phenyl-1,3-dioxane, 4,4a,5,9b-tetrahydroindeno[1,2-d]-m-dioxin, 4,4a,5,9b-tetrahydro-2,4-dimethylindeno[1,2-d]-m-dioxin;
xix) aromatic and aliphatic aldehydes, such as, for example, benzaldehyde; phenylacetaldehyde, 3-phenylpropanal, hydratropaldehyde, 4-methylbenzaldehyde, 4-methylphenylacetaldehyde, 3-(4-ethylphenyl)-2,2-dimethylpropanal, 2-methyl-3-(4-isopropylphenyl)propanal, 2-methyl-3-(4-tert-butylphenyl)propanal, 3-(4-tert-butylphenyl)-propanal, cinnamaldehyde, alpha-butylcinnamaldehyde, alpha-amylcinnamaldehyde, alpha-hexylcinnamaldehyde, 3-methyl-5-phenylpentanal, 4-methoxybenzaldehyde, 4-hydroxy-3 -methoxybenzaldehyde, 4-hydroxy-3 -ethoxybenzaldehyde, 3,4-methylenedioxybenzaldehyde, 3,4-dimethoxybenzaldehyde, 2-methyl-3 -(4-methoxyphenyl)-propanal, 2-methyl-3 -(4-methylendioxyphenyl)propanal;
xx) aromatic and aliphatic ketones, such as, for example, acetophenone, 4-methylacetophenone, 4-methoxyacetophenone, 4-tert-butyl-2,6-dimethylacetophenone, 4-phenyl-2-butanone, 4-(4-hydroxyphenyl)-2-butanone, 1-(2-naphthalenyl)ethanone, benzophenone, 1,1,2,3,3,6-hexamethyl-5-indanyl methyl ketone, 6-tert-butyl-1,1-dimethyl-4-indanyl methyl ketone, 1-[2,3-dihydro-1,1,2,6-tetramethyl-3-(1-methylethyl)-1H-5-indenyl]ethanone, 5',6',7',8'-tetrahydro-3',5',5',6',8',8'-hexamethyl-2-acetonaphthone;
xxi) aromatic and araliphatic carboxylic acids and esters thereof, such as, for example, benzoic acid, phenylacetic acid, methyl benzoate, ethyl benzoate, hexyl benzoate, benzyl benzoate, methyl phenylacetate, ethyl phenylacetate, geranyl phenylacetate, phenylethyl phenylacetate, methyl cinnamate, ethyl cinnamate, benzyl cinnamate, phenylethyl cinnamate, cinnamyl cinnamate, allyl phenoxyacetate, methyl salicylate, isoamyl salicylate, hexyl salicylate, cyclohexyl salicylate, cis-3-hexenyl salicylate, benzyl salicylate, phenylethyl salicylate, methyl 2,4-dihydroxy-3,6-dimethylbenzoate, ethyl 3-phenylglycidate, ethyl 3-methyl-3-phenylglycidate;
xxii) nitrogen-containing aromatic compounds, such as, for example, 2,4,6-trinitro-1,3-dimethyl-5-tert-butylbenzene, 3,5-dinitro-2,6-dimethyl-4-tert-butylaceto-phenone, cinnamonitrile, 5-phenyl-3-methyl-2-pentenonitrile, 5-phenyl-3-methyl-pentanonitrile, methyl anthranilate, methy-N-methylanthranilate, Schiff's bases of methyl anthranilate with 7-hydroxy-3,7-dimethyloctanal, 2-methyl-3-(4-tert-butylphenyl)-propanal or 2,4-dimethyl-3-cyclohexene carbaldehyde, 6-isopropylquinoline, 6-isobutylquinoline, 6-sec-butylquinoline, indole, skatole, 2-methoxy-3-isopropylpyrazine, 2-isobutyl-3-methoxypyrazine;
xxiii) phenols, phenyl ethers and phenyl esters, such as, for example, estragole, anethole, eugenol, eugenyl methyl ether, isoeugenol, isoeugenol methyl ether, thymol, carvacrol, diphenyl ether, beta-naphthyl methyl ether, beta-naphthyl ethyl ether, beta-naphthyl isobutyl ether, 1,4-dimethoxybenzene, eugenyl acetate, 2-methoxy-4-methylphenol, 2-ethoxy-5-(1-propenyl)phenol, p-cresyl phenylacetate;
xxiv) heterocyclic compounds, such as, for example, 2,5-dimethyl-4-hydroxy-2H-furan-3-one, 2-ethyl-4-hydroxy-5-methyl-2H-furan-3-one, 3-hydroxy-2-methyl-4H-pyran-4-one, 2-ethyl-3-hydroxy-4H-pyran-4-one;
xxv) lactones, such as, for example, 1,4-octanolide, 3-methyl-1,4-octanolide, 1,4-nonanolide, 1,4-decanolide, 8-decen-1,4-olide, 1,4-undecanolide, 1,4-dodecanolide, 1,5-decanolide, 1,5-dodecanolide, 1,15-pentadecanolide, cis- and trans-11-pentadecen-1,15-olide, cis- and trans-12-pentadecen-1,15-olide, 1,16-hexadecanolide, 9-hexadecen-1,16-olide, 10-oxa-1,16-hexadecanolide, 11-oxa-1,16-hexadecanolide, 12-oxa-1,16-hexadecanolide, ethylene-1,12-dodecanedioate, ethylene-1,13-tridecanedioate, coumarin, 2,3-dihydrocoumarin, and octahydrocoumarin;
xxvi) essential oils, concretes, absolutes, resins, resinoids, balsams, tinctures such as for example ambergris tincture, amyris oil, angelica seed oil, angelica root oil, aniseed oil, valerian oil, basil oil, tree moss absolute, bay oil, armoise oil, benzoe resinoid, bergamot oil, beeswax absolute, birch tar oil, bitter almond oil, savory oil, buchu leaf oil, cabreuva oil, cade oil, calamus oil, camphor oil, cananga oil, cardamom oil, cascarilla oil, cassia oil, cassie absolute, castoreum absolute, cedar leaf oil, cedar wood oil, cistus oil, citronella oil, lemon oil, copaiba balsam, copaiba balsam oil, coriander oil, costus root oil, cumin oil, cypress oil, davana oil, dill weed oil, dill seed oil, eau de brouts absolute, oakmoss absolute, elemi oil, estragon oil, eucalyptus citriodora oil, eucalyptus oil (cineole type), fennel oil, fir needle oil, galbanum oil, galbanum resin, geranium oil, grapefruit oil, guaiacwood oil, gurjun balsam, gurjun balsam oil, helichrysum absolute, helichrysum oil, ginger oil, iris root absolute, iris root oil, jasmine absolute, calamus oil, blue camomile oil, Roman camomile oil, carrot seed oil, cascarilla oil, pine needle oil, spearmint oil, caraway oil, labdanum oil, labdanum absolute, labdanum resin, lavandin absolute, lavandin oil, lavender absolute, lavender oil, lemon-grass oil, lovage oil, lime oil distilled, lime oil expressed, linaloe oil, Litsea cubeba oil, laurel leaf oil, mace oil, marjoram oil, mandarin oil, massoi (bark) oil, mimosa absolute, ambrette seed oil, musk tincture, clary sage oil, nutmeg oil, myrrh absolute, myrrh oil, myrtle oil, clove leaf oil, clove bud oil, neroli oil, olibanum absolute, olibanum oil, opopanax oil, orange flower absolute, orange oil, origanum oil, palmarosa oil, patchouli oil, perilla oil, Peru balsam oil, parsley leaf oil, parsley seed oil, petitgrain oil, peppermint oil, pepper oil, pimento oil, pine oil, pennyroyal oil, rose absolute, rosewood oil, rose oil, rosemary oil, Dalmatian sage oil, Spanish sage oil, sandal-wood oil, celery seed oil: spike-lavender oil, star anise oil, storax oil, tagetes oil, fir needle oil, tea tree oil, turpentine oil, thyme oil, Tolu balsam, tonka bean absolute, tuberose absolute, vanilla extract, violet leaf absolute, verbena oil, vetiver oil, juniperberry oil, wine lees oil, wormwood oil, wintergreen oil, ylang-ylang oil, hyssop oil, civet absolute, cinnamon leaf oil, cinnamon bark oil, and fractions thereof or ingredients isolated therefrom;
(xxvii) flavors including, but are not limited to, acetaldehyde, dimethyl sulfide, ethyl acetate, ethyl propionate, methyl butyrate, and ethyl butyrate. Flavors containing volatile aldehydes or esters include, e.g., cinnamyl acetate, cinnamaldehyde, citral, diethylacetal, dihydrocarvyl acetate, eugenyl formate, and p-methylanisole. Further examples of volatile compounds that may be present in the instant flavor oils include acetaldehyde (apple); benzaldehyde (cherry, almond); cinnamic aldehyde (cinnamon); citral, i.e., alpha citral (lemon, lime); neral, i.e., beta citral (lemon, lime); decanal (orange, lemon); ethyl vanillin (vanilla, cream); heliotropine, i.e., piperonal (vanilla, cream); vanillin (vanilla, cream); alpha-amyl cinnamaldehyde (spicy fruity flavors); butyraldehyde (butter, cheese); valeraldehyde (butter, cheese); citronellal (modifies, many types); decanal (citrus fruits); aldehyde C-8 (citrus fruits); aldehyde C-9 (citrus fruits); aldehyde C-12 (citrus fruits); 2-ethyl butyraldehyde (berry fruits); hexenal, i.e., trans-2 (berry fruits); tolyl aldehyde (cherry, almond); veratraldehyde (vanilla); 2,6-dimethyl-5-heptenal, i.e., melonal (melon); 2-6-dimethyloctanal (green fruit); and 2-dodecenal (citrus, mandarin); cherry; or grape and mixtures thereof. The composition may also contain taste modulators and artificial sweeteners. As used herein, flavor is understood to include spice oleoresins derived from allspice, basil, capsicum, cinnamon, cloves, cumin, dill, garlic, marjoram, nutmeg, paprika, black pepper, rosemary, and turmeric, essential oils, anise oil, caraway oil, clove oil, eucalyptus oil, fennel oil, garlic oil, ginger oil, peppermint oil, onion oil, pepper oil, rosemary oil, spearmint oil, citrus oil, orange oil, lemon oil, bitter orange oil, tangerine oil, alliaceous flavors, garlic, leek, chive, and onion, botanical extracts, arnica flower extract, chamomile flower extract, hops extract, marigold extract, botanical flavor extracts, blackberry, chicory root, cocoa, coffee, kola, licorice root, rose hips, sarsaparilla root, sassafras bark, tamarind and vanilla extracts, protein hydrolysates, hydrolyzed vegetable proteins, meat protein hydrolyzes, milk protein hydrolyzates and compounded flavors both natural and artificial including those disclosed in S. Heath, Source Book of Flavors, Avi Publishing Co., Westport Connecticut, 1981, pages 149-277. Specific preferred flavor adjuvants include, but are not limited to, the following: anise oil; ethyl-2-methyl butyrate; vanillin; cis-3-heptenol; cis-3-hexenol; trans-2-heptenal; butyl valerate; 2,3-diethyl pyrazine; methylcyclopentenolone; benzaldehyde; valerian oil; 3,4-dimeth-oxyphenol; amyl acetate; amyl cinnamate, y-butyryl lactone; furfural; trimethyl pyrazine; phenyl acetic acid; isovaleraldehyde; ethyl maltol; ethyl vanillin; ethyl valerate; ethyl butyrate; cocoa extract; coffee extract; peppermint oil; spearmint oil; clove oil; anethol; cardamom oil; wintergreen oil; cinnamic aldehyde; ethyl-2-methyl valerate; g-hexenyl lactone; 2,4-decadienal; 2,4-heptadienal; methyl thiazole alcohol (4-methyl-5-b-hydroxyethyl thiazole); 2-methyl butanethiol; 4-mercapto-2-butanone; 3-mercapto-2-pentanone; 1-mercapto-2-propane; benzaldehyde; furfural; furfuryl alcohol; 2-mercapto propionic acid; alkyl pyrazine; methyl pyrazine; 2-ethyl-3-methyl pyrazine; tetramethyl pyrazine; polysulfides; dipropyl disulfide; methyl benzyl disulfide; alkyl thiophene; 2,3-dimethyl thiophene; 5-methyl furfural; acetyl furan; 2,4-decadienal; guiacol; phenyl acetaldehyde; b-decalactone; d-limonene; acetoin; amyl acetate; maltol; ethyl butyrate; levulinic acid; piperonal; ethyl acetate; n-octanal; n-pentanal; n-hexanal; diacetyl; monosodium glutamate; monopotassium glutamate; sulfur-containing amino acids, e.g., cysteine; hydrolyzed vegetable protein; 2-methylfuran-3-thiol; 2-methyldihydrofuran-3-thiol; 2,5-dimethylfuran-3-thiol; hydrolyzed fish protein; tetramethyl pyrazine; propylpropenyl disulfide; propylpropenyl trisulfide; diallyl disulfide; diallyl trisulfide; dipropenyl disulfide; dipropenyl trisulfide; 4-methyl-2-[(methylthio)-ethyl]-1,3-dithiolane; 4,5-dimethyl-2-(methylthiomethyl)-1,3 -dithiolane; 4-methyl-2-(methylthiomethyl)-1,3-dithiolane, and the flavor ingredients described in U.S. Patent Nos. 6,110,520 and 6,333,180;
(xxviii) taste masking agents, substances for masking one or more unpleasant taste sensations, in particular a bitter, astringent and/or metallic taste sensation or aftertaste. Examples include lactisol [2O-(4-methoxyphenyl) lactic acid] (cf. U.S. Pat. No. 5,045,336), 2,4-dihydroxybenzoic acid potassium salt (cf. U.S. Pat. No. 5,643,941), ginger extracts (cf. GB 2,380,936), neohesperidine dihydrochalcone (cf. Manufacturing Chemist 2000, July issue, p. 16-17), specific flavones (2-phenylchrom-2-en-4-ones) (cf. U.S. Pat. No. 5,580,545), specific nucleotides, for example cytidine-5'-monophosphates (CMP) (cf. US 2002/0177576), specific sodium salts, such as sodium chloride, sodium citrate, sodium acetate and sodium lactate (cf. Nature, 1997, Vol. 387, p. 563), a lipoprotein of .beta.-lactoglobulin and phosphatidic acid (cf. EPA 635 218), neodiosmine [5,7-dihydroxy-2-(4-methoxy-3-hydroxyphenyl)-7-O-neohesperidosyl-chrom-2-en-4-one] (cf. U.S. Pat. No. 4,154,862), preferably hydroxyflavanones according to EP 1 258 200, in turn preferred in this respect 2-(4-hydroxyphenyl)-5,7-dihydroxychroman-4-one (naringenin), 2-(3,4-dihydroxyphenyl)-5,7-dihydroxychroman-4-one (eriodictyol), 2-(3,4-dihydroxyphenyl)-5-hydroxy-7-methoxychroman-4-one (eriodictyol-7-methylether), 2-(3,4-dihydroxyphenyl)-7-hydroxy-5-methoxychroman-4-one (eriodictyol-5-methylether) and 2-(4-hydroxy-3-methoxyphenyl)-5,7-dihydroxychroman-4-one (homoeriodictyol), the (2S)- or (2R)-enantiomers thereof or mixtures thereof as well as the mono- or polyvalent phenolate salts thereof with Na⁺, K⁺, NH4⁺, Ca²⁺, Mg²⁺ or Al³⁺ as counter cations or .gamma.-aminobutyric acid (4-aminobutyric acid, as the neutral form ("inner salt") or in the carboxylate or ammonium form) according to WO 2005/09684;
(xxix) taste sensates including hot tasting, salivation-inducing substances, substances causing a warmth or tingling feeling, and cooling active ingredients. Examples of hot tasting and/or salivation-inducing substances and/or substances which cause a feeling of warmth and/or a tingling feeling on the skin or on the mucous membranes and which can be a constituent of the products according to the invention are: capsaicin, dihydrocapsaicin, gingerol, paradol, shogaol, piperine, carboxylic acid-N-vanillylamides, in particular nonanoic acid-N-vanillylamide, pellitorin or spilanthol, 2-nonanoic acid amides, in particular 2-nonanoic acid-N-isobutylamide, 2-nonanoic acid-N-4-hydroxy-3-methoxyphenylamide, alkyl ethers of 4-hydroxy-3-methoxybenzyl alcohol, in particular 4-hydroxy-3-methoxybenzyl-n-butylether, alkyl ethers of 4-acyloxy-3-methoxybenzyl alcohol, in particular 4-acetyloxy-3-methoxybenzyl-n-butylether and 4-acetyloxy-3-methoxybenzyl-n-hexylether, alkyl ethers of 3-hydroxy-4-methoxybenzyl alcohol, alkyl ethers of 3,4-dimethoxybenzyl alcohol, alkyl ethers of 3-ethoxy-4-hydroxybenzyl alcohol, alkyl ethers of 3,4-methylene dioxybenzyl alcohol, (4-hydroxy-3-methoxyphenyl)acetic acid amides, in particular (4-hydroxy-3-methoxyphenyl)acetic acid-N-n-octylamide, vanillomandelic acid alkylamides, ferulic acid-phenethylamides, nicotinaldehyde, methylnicotinate, propylnicotinate, 2-butoxyethylnicotinate, benzylnicotinate, 1-acetoxychavicol, polygodial and isodrimeninol, further preferred cis- and/or trans-pellitorin according to WO 2004/000787 or WO 2004/043906, alkenecarboxylic acid-N-alkylamides according to WO 2005/044778, mandelic acid alkylamides according to WO 03/106404 or alkyloxyalkanoic acid amides according to WO 2006/003210. Examples of preferred hot tasting natural extracts and/or natural extracts which cause a feeling of warmth and/or a tingling feeling on the skin or on the mucous membranes and which can be a constituent of the products according to the invention are: extracts of paprika, extracts of pepper (for example capsicum extract), extracts of chili pepper, extracts of ginger roots, extracts of Aframomum melgueta, extracts of Spilanthes-acmella, extracts of Kaempferia galangal or extracts of Alpinia galanga. Suitable cooling active ingredients include the following: 1-menthol, d-menthol, racemic menthol, menthone glycerol acetal (trade name: Frescolat.RTM.MGA), menthyl lactate (trade name: Frescolat.RTM.ML, menthyl lactate preferably being 1-menthyl lactate, in particular 1-menthyl-1-lactate), substituted menthyl-3-carboxamides (for example menthyl-3-carboxylic acid-N-ethylamide), 2-isopropyl-N-2,3-trimethyl-butanamide, substituted cyclohexane carboxamides, 3-menthoxypropane-1,2-diol, 2-hydroxyethyl menthyl carbonate, 2-hydroxypropyl menthyl carbonate, N-acetylglycine menthyl ester, isopulegol, hydroxycarboxylic acid menthyl esters (for example menthyl-3-hydroxybutyrate), monomenthyl succinate, 2-mercaptocyclo-decanone, menthyl-2-pyrrolidin-5-onecarboxylate, 2,3-dihydroxy-p-menthane, 3,3,5-trimethylcyclohexanone glycerol ketal, 3-menthyl-3,6-di- and -trioxaalkanoates, 3-menthyl methoxyacetate and icilin. Cooling active ingredients which are particularly preferred are as follows: 1-menthol, racemic menthol, menthone glycerol acetal (trade name: Frescolat.RTM.MGA), menthyl lactate (preferably 1-menthyl lactate, in particular 1-menthyl-1-lactate, trade name: Frescolat.RTM.ML), 3-menthoxypropane-1,2-diol, 2-hydroxyethyl menthyl carbonate, 2-hydroxypropyl menthyl carbonate.
(xxx) malodor counteracting agents including an α,β-unsaturated carbonyl compounds including but not limited to those disclosed in US 6,610,648 and EP 2,524,704, amyl cinnamaldehyde, benzophenone, benzyl benzoate, benzyl isoeugenol, benzyl phenyl acetate, benzyl salicylate, butyl cinnamate, cinnamyl butyrate, cinnamyl isovalerate, cinnamyl propionate, decyl acetate, ethyl myristate, isobutyl cinnamate, isoamyl salicylate, phenethyl benzoate, phenethyl phenyl acetate, triethyl citrate, tripropylene glycol n-butyl ether, isomers of bicyclo[2.2.1]hept-5-ene-2-carboxylic acid, ethyl ester, nano silver, zinc undecenylate, β-naphthyl methyl ether, β-naphthyl ketone, benzyl acetone. They may include mixture of hexahydro-4,7-methanoinden-5-yl propionate and hexahydro-4,7-methanoinden-6-yl propionate; 4-(2,6,6-trimethyl-2-cyclohexen-1-yl)-3-methyl-3-buten-2-one; 3,7-dimethyl-2,6-nonadien-1-nitrile; dodeca-hydro-3a,6,6,9a-tetramethylnaphtho(2,1-b)furan; ethylene glycol cyclic ester of n-dodecanedioic acid; 1-cyclohexadecen-6-one; 1-cycloheptadecen-10-one; and corn mint oil. They may also include 1-cyclohexylethan-1-yl butyrate; 1-cyclohexylethan-1-yl acetate; 1-cyclohexylethan-1-ol; 1-(4'-methylethyl)cyclohexylethan-1-yl propionate; and 2'-hydroxy-1'-ethyl(2-phenoxy)acetate each of which compound is marketed under the trademark VEILEX by International Flavors & Fragrances Inc. More suitable malodor counteracting agents are polymers containing an α-keto, benzaldehyde, or α,β-unsaturated carbonyl moiety, such as those described in US Application Publications 2012/0294821, 2013/0101544 and 2013/0101545;
(xxxi) vitamins including any vitamin, a derivative thereof and a salt thereof. Examples are as follows: vitamin A and its analogs and derivatives (e.g., retinol, retinal, retinyl palmitate, retinoic acid, tretinoin, and iso-tretinoin, known collectively as retinoids), vitamin E (tocopherol and its derivatives), vitamin C (L-ascorbic acid and its esters and other derivatives), vitamin B3 (niacinamide and its derivatives), alpha hydroxy acids (such as glycolic acid, lactic acid, tartaric acid, malic acid, citric acid, etc.) and beta hydroxy acids (such as salicylic acid and the like);
(xxxii) antibacterials including bisguanidines (e.g., chlorhexidine digluconate), diphenyl compounds, benzyl alcohols, trihalocarbanilides, quaternary ammonium compounds, ethoxylated phenols, and phenolic compounds, such as halo-substituted phenolic compounds, like PCMX (i.e., p-chloro-m-xylenol), triclosan (i.e., 2 , 4, 4 ' -trichloro-2 ' hydroxy-diphenylether), thymol, and triclocarban;
(xxxiii) sunscreen actives including oxybenzone, octylmethoxy cinnamate, butylmethoxy dibenzoyln ethane, p-aminobenzoic acid and octyl dimethyl-p-aminobenzoic acid;
(xxxiv) antioxidants such as beta-carotene, vitamin C (Ascorbic Acid) or an ester thereof, vitamin A or an ester thereof, vitamin E or an ester thereof, lutein or an ester thereof, lignan, lycopene, selenium, flavonoids, vitamin-like antioxidants such as coenzyme Q10 (CoQ10) and glutathione, and antioxidant enzymes such as superoxide dismutase (SOD), catalase, and glutathione peroxidase;
(xxxv) anti-inflammatory agents including, e.g., methyl salicylate, aspirin, ibuprofen, and naproxen. Additional anti-inflammatories useful in topical applications include corticosteroids, such as, but not limited to, flurandrenolide, clobetasol propionate, halobetasol propionate, fluticasone propionate, betamethasone dipropionate, betamethasone benzoate, betamethasone valerate, desoximethasone, dexamethasone, diflorasone diacetate, mometasone furoate, amcinodine, halcinonide, fluocinonide, fluocinolone acetonide, desonide, triamcinolone acetonide, hydrocortisone, hydrocortisone acetate, fluoromethalone, methylprednisolone, and predinicarbate;
(xxxvi) anesthetics that can be delivered locally including benzocaine, butamben, butamben picrate, cocaine, procaine, tetracaine, lidocaine and pramoxine hydrochloride;
(xxxvii) analgesics such as ibuprofen, diclofenac, capsaicin, and lidocaine;
(xxxviii) antifungal agents. Non-limiting examples are micanazole, clotrimazole, butoconazole, fenticonasole, tioconazole, terconazole, sulconazole, fluconazole, haloprogin, ketonazole, ketoconazole, oxinazole, econazole, itraconazole, torbinafine, nystatin and griseofulvin;
(xxxix) antibiotics such as erythromycin, clindamycin, synthomycin, tetracycline, metronidazole and the like;
(xl) anti-viral agents including famcyclovir, valacyclovir and acyclovir;
(xli) anti-parasitic agents such as scabicedes, such as permethrin, crotamiton, lindane and ivermectin;
(xii) anti-infectious and anti-acne agents including benzoyl peroxide, sulfur, resorcinol and salicylic acid;
(xliii) dermatological active ingredients useful in topical applications including, e.g., jojoba oil and aromatic oils such as methyl salicylate, wintergreen, peppermint oil, bay oil, eucalyptus oil and citrus oils, as well as ammonium phenolsulfonate, bismuth subgallate, zinc phenolsulfonate and zinc salicylate;
(xliv) enzymes and co-enzymes useful for topical application including coenzyme Q10, papain enzyme, lipases, proteases, superoxide dismutase, fibrinolysin, desoxyribonuclease, trypsin, collagenase and sutilains;
(xlv) skin whitening agents such as hydroquinone and monobenzone;
(xlvi) anti-histamines including chlorpheniramine, brompheniramine, dexchlorpheniramine, tripolidine, clemastine, diphenhydramine, prometazine, piperazines, piperidines, astemizole, loratadine and terfonadine;
(xlvii) chemotherapeutic agents such as 5-fluorouracil, masoprocol, mechlorethamine, cyclophosphamide, vincristine, chlorambucil, streptozocin, methotrexate, bleomycin, dactinomycin, daunorubicin, coxorubicin and tamoxifen; and
(xlviii) insect repellents including pediculicides for treatment of lice, such as pyrethrins, permethrin, malathion, lindane and the like.

In addition to the active materials listed above, the products of this invention can also contain, for example, the following dyes, colorants or pigments: lactoflavin (riboflavin), beta-carotene, riboflavin-5'-phosphate, alpha-carotene, gamma-carotene, cantaxanthin, erythrosine, curcumin, quinoline yellow, yellow orange S, tartrazine, bixin, norbixin (annatto, orlean), capsanthin, capsorubin, lycopene, beta-apo-8'-carotenal, beta-apo-8'-carotenic acid ethyl ester, xantophylls (flavoxanthin, lutein, cryptoxanthin, rubixanthin, violaxanthin, rodoxanthin), fast carmine (carminic acid, cochineal), azorubin, cochineal red A (Ponceau 4 R), beetroot red, betanin, anthocyanins, amaranth, patent blue V, indigotine I (indigo-carmine), chlorophylls, copper compounds of chlorophylls, acid brilliant green BS (lissamine green), brilliant black BN, vegetable carbon, titanium dioxide, iron oxides and hydroxides, calcium carbonate, aluminum, silver, gold, pigment rubine BK (lithol rubine BK), methyl violet B, victoria blue R, victoria blue B, acilan brilliant blue FFR (brilliant wool blue FFR), naphthol green B, acilan fast green 10 G (alkali fast green 10 G), ceres yellow GRN, sudan blue II, ultramarine, phthalocyanine blue, phthalocayanine green, fast acid violet R. Further naturally obtained extracts (for example paprika extract, black carrot extract, red cabbage extract) can be used for coloring purposes. Goods results are also achieved with the colors named in the following, the so-called aluminum lakes: FD & C Yellow 5 Lake, FD & C Blue 2 Lake, FD & C Blue 1 Lake, Tartrazine Lake, Quinoline Yellow Lake, FD & C Yellow 6 Lake, FD & C Red 40 Lake, Sunset Yellow Lake, Carmoisine Lake, Amaranth Lake, Ponceau 4R Lake, Erythrosyne Lake, Red 2G Lake, Allura Red Lake, Patent Blue V Lake, Indigo Carmine Lake, Brilliant Blue Lake, Brown HT Lake, Black PN Lake, Green S Lake and mixtures thereof.

Flavor oils may contain the following solvents/diluents: ethanol, vegetable oil triglycerides, 1,2-propylene glycol, benzyl alcohol, triacetin (glycerol triacetate), diacetin (glycerol diacetate), triethyl citrate, glycerol.

In some embodiments, the amount of encapsulated fragrance or flavor is from 0.5% to 80% of the total aggregate suspension or aggregate slurry, preferably from 5% to 60%, and most preferably from 20% to 50%.

In addition to the fragrances and flavors, the present invention contemplates the incorporation of solvent materials into one or more of the capsules. The solvent materials are hydrophobic materials that are miscible with the fragrances or flavors. The solvent materials serve to increase the compatibility of various active materials, increase the overall hydrophobicity of the mixture containing the active materials, influence the vapor pressure, or serve to structure the mixture. Suitable solvents are those having reasonable affinity for the active materials and a ClogP greater than 2, (e.g., greater than 2.5, preferably greater than 3.5 and more preferably greater than 5.5). In some embodiments, the solvent is combined with the active materials that have ClogP values as set forth above. It should be noted that selecting a solvent and active material with high affinity for each other will result in improvement in stability. Suitable solvents include triglyceride oil, mono and diglycerides, mineral oil, silicone oil, diethyl phthalate, polyalpha olefins, castor oil, isopropyl myristate, mono-, di- and tri-esters and mixtures thereof, fatty acids, and glycerine. The fatty acid chain can range from C₄-C₂₆ and can have any level of unsaturation. For instance, one of the following solvents can be used: capric/caprylic triglyceride known as NEOBEE M5 (Stepan Corporation); the CAPMUL series by Abitec Corporation (e.g., CAPMUL MCM); isopropyl myristate; fatty acid esters of polyglycerol oligomers, *e.g.,* R2CO-[OCH₂-CH(OCOR1)-CH₂O-]ₙ, where R1 and R2 can be H or C₄-C₂₆ aliphatic chains, or mixtures thereof, and n ranges between 2 and 50, preferably 2 and 30; nonionic fatty alcohol alkoxylates like the NEODOL surfactants by BASF; the dobanol surfactants by Shell Corporation or the BIO-SOFT surfactants by Stepan, wherein the alkoxy group is ethoxy, propoxy, butoxy, or mixtures thereof and said surfactants can be end-capped with methyl groups in order to increase their hydrophobicity; di- and tri-fatty acid chain containing nonionic, anionic and cationic surfactants, and mixtures thereof; fatty acid esters of polyethylene glycol, polypropylene glycol, and polybutylene glycol, or mixtures thereof; polyalphaolefins such as the EXXONMOBIL PURESYM PAO line; esters such as the EXXONMOBIL PURESYN esters; mineral oil; silicone oils such polydimethyl siloxane and polydimethylcyclosiloxane; diethyl phthalate; di-octyl adipate and di-isodecyl adipate.

While no solvent is needed in the core, it is preferable that the level of solvent in the core of the microcapsule product is 80 wt% or less, preferably 50 wt% or less (e.g., 0-20 wt%).

When the active material is a fragrance, in some embodiments, a fragrance having a high weighted ClogP is encapsulated in the capsules of the aggregates of this invention, e.g., 3 to 8. In other embodiment, a fragrance having a low weighted ClogP is used, e.g., 0.5 to 2. For instance, the ingredients having a ClogP value between 2 and 7 (e.g., between 2 and 6, and between 2 and 5) are 25 % or greater (e.g., 50 % or greater and 90 % or greater) by the weight of the fragrance. Those skilled in the art will appreciate that many fragrances can be created employing various solvents and fragrance ingredients. The use of relatively low to intermediate ClogP fragrance ingredients will result in fragrances that are suitable for encapsulation. These fragrances are generally water-insoluble, to be delivered through the aggregates of this invention onto consumer products in different stages such as damp and dry fabric. Without encapsulation, the free fragrances would normally have evaporated or dissolved in water during use, e.g., wash; and without coacervation, capsules enclosing a fragrance would have been unstable and/or unable to deposit onto consumer products especially in an ionic environment. Not to be bounded by any theory, the ions in a formulation base (i.e., the environment) can interact with anionic, cationic, and amphoteric capsules, and also with deposition polymer, which is generally cationic. This interaction not only diminishes the bonding between the deposition polymer and the capsules resulting poor capsule deposition, but also damages the capsule walls and/or induces leakage of fragrance before use.

High ClogP materials have excellent encapsulation properties they are generally well delivered from a regular (non-encapsulated) fragrance in a consumer product. Such fragrance chemicals would generally need encapsulation for overall fragrance character purposes, very long-lasting fragrance delivery, or overcoming incompatibility with the consumer product, e.g., fragrance materials that would otherwise be instable, cause thickening or discoloration of the product or otherwise negatively affect desired consumer product properties.

The active material to be encapsulated can be dispersed in aqueous solutions in the absence/presence of polymers, pre-condensates, surfactants, scavengers and the like prior to formation of capsules. In certain embodiments, the capsules include formaldehyde scavengers, which can be used from effective trace amounts up to 100 times the stoichiometric amount. The stoichiometric amount is the amount of scavenger required to theoretically bind or react all the formaldehyde added in the form of an aminoplast crosslinker (bound and free formaldehyde). This amount of scavenger can be added either to the slurry or afterward to the final product formulation. For instance, an unscavenged slurry can be added to the formulation, followed by a certain amount of scavenger. The particular quantity of a formaldehyde-based cross-linker that is used to create the capsule slurry contains a percentage of free formaldehyde and bound formaldehyde. The total combined moles of free and bound formaldehyde will determine the amount of moles of scavenger that is needed to react with all the formaldehyde. To drive this reaction to completion, about a 10X molar excess of scavenger is used, preferably about a 5X molar excess of scavenger. By moles here is meant moles of scavenging groups. Therefore, if the scavenger molecule is multifunctional (*i.e*., polymeric) less moles of this molecule need to be added.

The minimum level of scavenger required is the amount that removes only the free formaldehyde in the slurry. This level is determined analytically. The minimum amount of moles of scavenger required is equal to the moles of analytically determined formaldehyde (1:1). Exemplary formaldehyde scavengers include β-dicarbonyl compounds; mono or di-amide scavengers; amines that form imines by reaction with formaldehyde; and formaldehyde reducers and sulfur containing compounds, such as those disclosed in US 2009/0258042.

Useful β-dicarbonyl compounds react with formaldehyde. Examples include, but are not limited to, acetoacetamide (BKB; Eastman), ethyl acetoacetate (EAA; Eastman), N,N-dimethyleneacetamide (DMAA; Eastman), acetoacetone, dimethyl-1,3-acetonedicarboxylate, 1,3-acetonedicarboxylic acid, malonic acid, resorcinol, 1,3-cyclohexadione, barbituric acid, 5,5-dimethyl-1,3-cyclohexanedione (dimedone), 2,2-dimethyl-1,3-dioxane-4,6-dione (Meldrum's acid), salicylic acid, methyl acetoacetate (MAA; Eastman), ethyl-2-methyl acetoacetate, 3-methyl-acetoacetone, dimethyl malonate, diethyl malonate, 1,3-dimethyl barbituric acid, resorcinol, phloroglucinol, orcinol, 2,4-dihydroxy benzoic acid, 3,5-dihydroxy benzoic acid, malonamide and β-dicarbonyl scavengers listed in US 5,194,674 and US 5,446,195, as well as in Tomasino, et al. (1984) Textile Chemist and Colorist Vol. 16, No. 12.

Examples of useful mono- and di-amide scavengers are urea, ethylene urea, propylene urea, epsilon-caprolactam, glycouril, hydantoin, 2-oxazolidinone, 2-pyrrolidinone, uracil, barbituric acid, thymine, uric acid, allantoin, polyamides, 4,5-dihydroxyethylene urea, monomethylol-4-hydroxy-4-methoxy-5,5-dimethyl propylurea, nylon 2-hydroxyethyl ethylene urea (SR-511, SR-512; Sartomer), 2-hydroxyethyl urea (HYDROVANCE; National Starch), L-citrulline, biotin, N-methyl urea, N-ethyl urea, N-butyl urea, N-phenyl urea, 4,5-dimethoxy ethylene urea and succinimide.

Amines useful as scavengers include, but are not limited to, poly(vinyl amine) (LUPAMIN; BASF), arginine, lysine, asparagines, proline, tryptophan, 2-amino-2-methyl-1-propanol (AMP); proteins such as casein, gelatin, collagen, whey protein, soy protein, and albumin; melamine, benzoguanamine, 4-aminobenzoic acid (PABA), 3-aminobenzoic acid, 2-aminobenzoic acid (anthranilic acid), 2-aminophenol, 3-aminophenol, 4-aminophenol, creatine, 4-aminosalicylic acid, 5-aminosalicylic acid, methyl anthranilate, methoxylamine HCl, anthranilamide, 4-aminobenzamide, p-toluidine, p-anisidine, sulfanilic acid, sulfanilamide, methyl-4-aminobenzoate, ethyl-4-aminobenzoate (benzocain), beta-diethylaminoethyl-4-aminobenzoate (procain), 4-aminobenzamide, 3,5-diaminobenzoic acid and 2,4-diaminophenol. Other amines as disclosed in US 2006/0248665 and US 6,261,483, and those mentioned in Tomasino, et al. (1984) Textile Chemist and Colorist Vol. 16, No. 12, are also contemplated by the present invention. Other preferred amines can be selected from a non-limiting list of 1,2-phenylenediamine, 1,3-phenylenediamine, and 1,4-phenylenediamine. In addition, aromatic amines, triamines, and aliphatic polyamine may also be used. Examples of these amines may include, but are not limited to, aniline, hexamethylenediamine, bishexamethylenetriamine, triethylaminetriamine, poly(propyleneoxide)triamine, and poly(propyleneglycol)diamines. Hydrazines such as 2,4-dinitrophenzylhydrazine can also be used as scavengers. They react with formaldehyde to give hydrazones. The reaction is pH-dependent and reversible.

Other than scavengers, one or more adjunct material may be used together with the aggregates in the amount of from 0.01% to 25% (e.g., from 0.5% to 10%) by the weight of the composition containing the aggregate and adjunct material.

The adjunct material can be a solubility modifier, an antibacterial, a sunscreen active, an antioxidant, a malodor counteracting agent, a density modifier, a stabilizer, a viscosity modifier, a pH modifier, or any combination thereof. These modifiers can be present in the wall or core of the capsules, or outside the capsules. Preferably, they are in the core as a core modifier.

Nonlimiting examples of a solubility modifier include surfactants (e.g., SLS and Tween 80), acidic compounds (e.g., mineral acids such as sulfuric acid, hydrochloric acid, nitric acid, and phosphoric acid, and carboxylic acids such as acetic acid, citric acid, gluconic acid, glucoheptonic acid, and lactic acid), basic compounds (e.g., ammonia, alkali metal and alkaline earth metal hydroxides, primary, secondary, or tertiary amines, and primary, secondary, or tertiary alkanolamines), ethyl alcohol, glycerol, glucose, galactose, inositol, mannitol, glactitol, adonitol, arabitol, and amino acids.

Exemplary antibacterials include bisguanidines (e.g., chlorhexidine digluconate), diphenyl compounds, benzyl alcohols, trihalocarbanilides, quaternary ammonium compounds, ethoxylated phenols, and phenolic compounds, such as halo-substituted phenolic compounds, like PCMX (i.e., p-chloro-m-xylenol), triclosan (i.e., 2 , 4,4'-trichloro-2'hydroxy-diphenylether), thymol, and triclocarban.

Suitable sunscreen actives include oxybenzone, octylmethoxy cinnamate, butylmethoxy dibenzoyln ethane, p-aminobenzoic acid and octyl dimethyl-p-aminobenzoic acid.

Examples of antioxidants include beta-carotene, vitamin C (Ascorbic Acid) or an ester thereof, vitamin A or an ester thereof, vitamin E or an ester thereof, lutein or an ester thereof, lignan, lycopene, selenium, flavonoids, vitamin-like antioxidants such as coenzyme Q10 (CoQ10) and glutathione, and antioxidant enzymes such as superoxide dismutase (SOD), catalase, and glutathione peroxidase.

Malodor counteracting agents include, but not limited to, an α,β-unsaturated carbonyl compounds including but not limited to those disclosed in US 6,610,648 and EP 2,524,704, amyl cinnamaldehyde, benzophenone, benzyl benzoate, benzyl isoeugenol, benzyl phenyl acetate, benzyl salicylate, butyl cinnamate, cinnamyl butyrate, cinnamyl isovalerate, cinnamyl propionate, decyl acetate, ethyl myristate, isobutyl cinnamate, isoamyl salicylate, phenethyl benzoate, phenethyl phenyl acetate, triethyl citrate, tripropylene glycol n-butyl ether, isomers of bicyclo[2.2.1]hept-5-ene-2-carboxylic acid, ethyl ester, nano silver, and zinc undecenylate. More suitable malodor counteracting agents can be found in US 2013/0101544 and 2013/0101545.

The density of an aggregate slurry and/or an oil core can be adjusted so that a composition containing both has a substantially uniform distribution of the aggregates using known density modifiers or technologies such as those described in Patent Application Publications WO 2000/059616, EP 1 502 646, and EP 2 204 155. Suitable density modifiers include hydrophobic materials and materials having a desired molecular weight (e.g., higher than about 12,000), such as silicone oils, petrolatums, vegetable oils, especially sunflower oil and rapeseed oil, and hydrophobic solvents having a desired density (e.g., less than about 1,000 Kg/m³ at 25°C, such as limonene and octane.

In some embodiments, a stabilizer (e.g., a colloidal stabilizer) is added to aggregate compositions to stabilize the composition. Examples of colloidal stabilizers are polyvinyl alcohol, cellulose derivatives such as hydroxyethyl cellulose, polyethylene oxide, copolymers of polyethylene oxide and polyethylene or polypropylene oxide, cetyl trimethyl ammonium-based surfactants, or copolymers of acrylamide and acrylic acid.

Viscosity control agents (*e.g*., suspending agents), which may be polymeric or colloidal (*e.g*., modified cellulose polymers such as methylcellulose, hydoxyethylcellulose, hydrophobically modified hydroxyethylcellulose, and cross-linked acrylate polymers such as Carbomer, hydrophobically modified polyethers) can be included in aggregate compositions described above, either in the oil core or capsule wall, or in the aggregate slurry outside the capsules. Optionally, silicas, either hydrophobic or hydrophilic, can be included at a concentration from 0.01 % to 20 %, more preferable from 0.5 % to 5 %, by the weight of the capsule compositions. Examples of hydrophobic silicas include silanols, surfaces of which are treated with halogen silanes, alkoxysilanes, silazanes, and siloxanes, such as SIPERNAT D17, AEROSIL R972 and R974 available from Degussa. Exemplary hydrophilic silicas are AEROSIL 200, SIPERNAT 22S, SIPERNAT 50S (available from Degussa), and SYLOID 244 (available from Grace Davison).

One or more humectants are optionally included to hold water in an aggregate composition for a long period of time. They constitute from 0.01% to 25% (e.g., 1% to 5%) by weight of the composition. Examples include glycerin, propylene glycol, alkyl phosphate esters, quaternary amines, inorganic salts (e.g., potassium polymetaphosphate, sodium chloride, etc.), polyethylene glycols, and the like.

Further suitable humectants, as well as viscosity control/suspending agents, are disclosed in US 4,428,869, 4,464,271, 4,446,032, and 6,930,078. Details of hydrophobic silicas as a functional delivery vehicle of active materials other than a free flow/anticaking agent are disclosed in US 5,500,223 and 6,608,017.

In some embodiments, one or more pH modifiers are included in an aggregate composition to adjust the pH value of the aggregate slurry and/or the oil core. The pH modifiers can also assist in the formation of capsule walls by changing the reaction rate of the crosslinking reactions that form the capsule walls. Exemplary pH modifiers include metal hydroxides (e.g., LiOH, NaOH, KOH, and Mg(OH)₂), metal carbonates and bicarbonates (CsCO₃ Li₂CO₃, K₂CO₃, NaHCO₃, and CaCO₃), metal phosphates/hydrogen phosphates/dihydrogen phosphates, metal sulfates, ammonia, mineral acids (HCl, H₂SO₄, H₃PO₄, and HNO₃), carboxylic acids (e.g., acetic acid, citric acid, lactic acid, benzoic acid, and sulfonic acids), and amino acids.

The aggregates of this invention can be used together with one or more non-confined unencapsulated active materials from 0.01% to 50%, more preferably from 5% to 40% by weight of the composition containing an aggregate and a free fragrance.

Optionally, an emulsifier (*i.e*., nonionic such as polyoxyethylene sorbitan monostearate (e.g., TWEEN 60), anionic such as sodium oleate, zwitterionic such as lecithins) from 0.01 weight % to 25 weight %, more preferably from 5 weight % to 10 weight % can be included.

Additional polymeric core modifiers are also contemplated. It has been found that the addition of hydrophobic polymers to the core can also improve stability by slowing diffusion of the active material from the core. The level of polymer is normally less than 80% of the core by weight, preferably less than 50%, and more preferably less than 20%. The basic requirement for the polymer is that it be miscible or compatible with the other components of the core, namely the active material and other solvent. Preferably, the polymer also thickens or gels the core, thus further reducing diffusion. These additional polymeric core modifiers include copolymers of ethylene; copolymers of ethylene and vinyl acetate (ELVAX polymers by DOW Corporation); copolymers of ethylene and vinyl alcohol (EVAL polymers by Kuraray); ethylene/acrylic elastomers such as VALNAC polymers by Dupont; polyvinyl polymers, such as polyvinyl acetate; alkyl-substituted cellulose, such as ethyl cellulose (ETHOCEL made by DOW Corporation) and hydroxypropyl celluloses (KLUCEL polymers by Hercules); cellulose acetate butyrate available from Eastman Chemical; polyacrylates (*e.g*., AMPHOMER, DEMACRYL LT and DERMACRYL 79, made by National Starch and Chemical Company, the AMERHOLD polymers by Amerchol Corporation, and ACUDYNE 258 by ISP Corporation); copolymers of acrylic or methacrylic acid and fatty esters of acrylic or methacrylic acid such as INTELIMER POLYMERS made by Landec Corporation (see also U.S. Pat. Nos. 4,830,855, 5,665,822, 5,783,302, 6,255,367 and 6,492,462); polypropylene oxide; polybutylene oxide of poly(tetrahydrofuran); polyethylene terephthalate; polyurethanes (DYNAM X by National Starch); alkyl esters of poly(methyl vinyl ether); maleic anhydride copolymers, such as the GANTREZ copolymers and OMNIREZ 2000 by ISP Corporation; carboxylic acid esters of polyamines, *e.g.,* ester-terminated polyamides (ETPA) made by Arizona Chemical Company; polyvinyl pyrrolidone (LUVISKOL series of BASF); block copolymers of ethylene oxide, propylene oxide and/or butylenes oxide including, *e.g.,* PLURONIC and SYNPERONIC polymers/dispersants by BASF. Another class of polymers include polyethylene oxide-co-propyleneoxide-co-butylene oxide polymers of any ethylene oxide/propylene oxide/butylene oxide ratio with cationic groups resulting in a net theoretical positive charge or equal to zero (amphoteric). The general structure is: 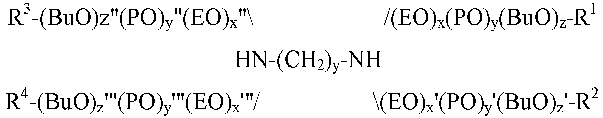 where R¹, R², R³, and R⁴ are independently H or any alkyl or fatty alkyl chain group. Examples of such polymers are the commercially known as TETRONICS by BASF Corporation.

Sacrificial core ingredients can also be included. These ingredients are designed to be lost during or after manufacture and include, but are not limited to, highly water soluble or volatile materials.

In addition to the capsules and adjunct materials described above, the aggregates of this invention can also be used together with one or more other delivery compositions such as polymer-assisted delivery compositions (see US 8,187,580), fiber-assisted delivery compositions (US 2010/0305021), cyclodextrin host guest complexes (US 6,287,603 and US 2002/0019369), pro-fragrances (WO 2000/072816 and EP 0 922 084), membrane delivery systems (US 4,948,047), and any combination thereof.

As used herein olfactory effective amount is understood to mean the amount of compound in the aggregates the individual components will contribute to its particular olfactory characteristics, but the olfactory effect of the delivery system will be the sum of the effects of each of the individual components. Thus, the aggregate of this invention can be used to alter the aroma characteristics of a consumer product, e.g., a fine perfume, by modifying the olfactory reaction contributed by another ingredient in the consumer product. The amount will vary depending on many factors including other ingredients, their relative amounts and the effect that is desired.

Before coacervating into an aggregate, the capsules can be cured at a temperature in the range of, *e.g.,* 55°C to 130°C (e.g., 55°C to 90°C, 55°C to 75°C, and 90°C to 130°C) for 1 minute to 10 hours (e.g., 0.1 hours to 5 hours, 0.2 hours to 4 hours and 0.5 hours to 3 hours). A skilled person in the art can determine, without undue experiments, the curing temperature, duration, and the heating rate.

Not to be bound by any theory, it is believed that there is a direct relationship between higher cure temperature and less leaching of active material from the capsule. Accordingly, the capsules can be cured at a temperature at or above 100°C (e.g., above 110°C and 120°C) to improve the retention capabilities of the capsules.

To obtain capsules with more leaching of the active material, the capsules are cured at or less than 100°C (e.g., at or less than 90°C and at or less than 80°C).

The rate at which the capsules are heated, i.e., cured, also affect the fragrance release profile of the capsules. For instance, the capsules are heated to a target cure temperature at a linear rate of 0.5 to 2 °C per minute (e.g., 1 to 5 °C per minute, 2 to 8 °C per minute, and 2 to 10°C per minute) over a period of 1 to 60 minutes (e.g., 1 to 30 minutes). The following heating methods may be used: conduction for example via oil, steam radiation via infrared, and microwave, convection via heated air, steam injection and other methods known by those skilled in the art. The target cure temperature used herein refers to the minimum temperature in degrees Celsius at which the capsules may be cured to retard leaching.

***Applications.*** The delivery systems of the present invention are well-suited for use, without limitation, in the following products:
a) Household products
   i. Liquid or Powder Laundry Detergents which can use the present invention include those systems described in U.S. Pat. Nos. 5,929,022, 5,916,862, 5,731,278, 5,565,145, 5,470,507, 5,466,802, 5,460,752, 5,458,810, 5,458,809, 5,288,431, 5,194,639, 4,968,451, 4,597,898, 4,561,998, 4,550,862, 4,537,707, 4,537,706, 4,515,705, 4,446,042, and 4,318,818
   ii. Unit Dose Pouches, Tablets and Capsules such as those described in EP 1 431 382 A1, US 2013/0219996 A1, US 2013/0284637 A1, and US 6,492,315. These unit dose formulations can contain high concentrations of a functional material (*e.g*., 5-100% fabric softening agent or detergent active), fragrance (*e.g*., 0.5-100%, 0.5-40%, and 0.5-15%), and flavor (*e.g*., 0.1-100%, 0.1-40%, and 1-20%). They can contain no water to limit the water content as low as less than 30% (*e.g*., less than 20%, less than 10%, and less than 5%).
   iii. Scent Boosters such as those described in US 7,867,968, US 7,871,976, US 8,333,289, US 2007/0269651 A1, and US2014/0107010 A1.
   iv. Fabric Care Products such as Rinse Conditioners (containing 1 to 30 weight % of a fabric conditioning active), Fabric Liquid Conditioners (containing 1 to 30 weight % of a fabric conditioning active), Tumble Drier Sheets, Fabric Refreshers, Fabric Refresher Sprays, Ironing Liquids, and Fabric Softener Systems such as those described in U.S. Pat. Nos. 6,335,315, 5,674,832, 5,759,990, 5,877,145, 5,574,179, 5,562,849, 5,545,350, 5,545,340, 5,411,671, 5,403,499, 5,288,417, 4,767,547 and 4,424,134
      Liquid fabric softeners/fresheners contains at least one fabric softening agent present, preferably at a concentration of 1 to 30% (e.g., 4 to 20%, 4 to 10%, and 8 to 15%). The ratio between the active material and the fabric softening agent can be 1 : 500 to 1 : 2 (e.g., 1 : 250 to 1 : 4 and 1 : 100 to 1 :8). As an illustration, when the fabric softening agent is 5% by weight of the fabric softener, the active material is 0.01 to 2.5%, preferably 0.02 to 1.25% and more preferably 0.1 to 0.63%. As another example, when the fabric softening agent is 20% by weight of the fabric softener, the active material is 0.04 to 10%, preferably 0.08 to 5% and more preferably 0.4 to 2.5%. The active material is a fragrance, malodor counteractant or mixture thereof. The liquid fabric softener can have 0.15 to 15% of capsules (e.g., 0.5 to 10%, 0.7 to 5%, and 1 to 3%). When including capsules at these levels, the neat oil equivalent (NOE) in the softener is 0.05 to 5% (e.g., 0.15 to 3.2%, 0.25 to 2%, and 0.3 to 1%).
      Suitable fabric softening agents include cationic surfactants. Non-limiting examples are quaternary ammonium compounds such as alkylated quaternary ammonium compounds, ring or cyclic quaternary ammonium compounds, aromatic quaternary ammonium compounds, diquaternary ammonium compounds, alkoxylated quaternary ammonium compounds, amidoamine quaternary ammonium compounds, ester quaternary ammonium compounds, and mixtures thereof. Fabric softening compositions, and components thereof, are generally described in US 2004/0204337 and US 2003/0060390. Suitable softening agents include esterquats such as Rewoquat WE 18 commercially available from Evonik Industries and Stepantex SP-90 commercially available from Stepan Company.
   v. Liquid dish detergents such as those described in U.S. Pat. Nos. 6,069,122 and 5,990,065
   vi. Automatic Dish Detergents such as those described in U.S. Pat. Nos. 6,020,294, 6,017,871, 5,968,881, 5,962,386, 5,939,373, 5,914,307, 5,902,781, 5,705,464, 5,703,034, 5,703,030, 5,679,630, 5,597,936, 5,581,005, 5,559,261, 4,515,705, 5,169,552, and 4,714,562
   vii. All-purpose Cleaners including bucket dilutable cleaners and toilet cleaners
   viii. Bathroom Cleaners
   ix. Bath Tissue
   x. Rug Deodorizers
   xi. Candles
   xii. Room Deodorizers
   xiii. Floor Cleaners
   xiv. Disinfectants
   xv. Window Cleaners
   xvi. Garbage bags/trash can liners
   xvii. Air Fresheners including room deodorizer and car deodorizer, scented candles, sprays, scented oil air freshener, Automatic spray air freshener, and neutralizing gel beads
   xviii. Moisture absorber
   xix. Household Devices such as paper towels and disposable Wipes
   xx. Moth balls/traps/cakes
b) Baby Care Products
   i. Diaper Rash Cream/Balm
   ii. Baby Powder
c) Baby Care Devices
   i. Diapers
   ii. Bibs
   iii. Wipes
d) Oral Care Products. Tooth care products (as an example of preparations according to the invention used for oral care) generally include an abrasive system (abrasive or polishing agent), for example silicic acids, calcium carbonates, calcium phosphates, aluminum oxides and/or hydroxylapatites, surface-active substances, for example sodium lauryl sulfate, sodium lauryl sarcosinate and/or cocamidopropylbetaine, humectants, for example glycerol and/or sorbitol, thickening agents, for example carboxymethyl cellulose, polyethylene glycols, carrageenan and/or Laponite.RTM., sweeteners, for example saccharin, taste correctors for unpleasant taste sensations, taste correctors for further, normally not unpleasant taste sensations, taste-modulating substances (for example inositol phosphate, nucleotides such as guanosine monophosphate, adenosine monophosphate or other substances such as sodium glutamate or 2-phenoxypropionic acid), cooling active ingredients, for example menthol derivatives, (for example L-menthyllactate, L-menthylalkylcarbonates, menthone ketals, menthane carboxylic acid amides), 2,2,2-trialkylacetic acid amides (for example 2,2-diisopropylpropionic acid methyl amide), icilin and icilin derivatives, stabilizers and active ingredients, for example sodium fluoride, sodium monofluorophosphate, tin difluoride, quaternary ammonium fluorides, zinc citrate, zinc sulfate, tin pyrophosphate, tin dichloride, mixtures of various pyrophosphates, triclosan, cetylpyridinium chloride, aluminum lactate, potassium citrate, potassium nitrate, potassium chloride, strontium chloride, hydrogen peroxide, flavorings and/or sodium bicarbonate or taste correctors.
   i. Tooth Paste. An exemplary formulation as follows:
      1.calcium phosphate 40-55%
      2.carboxymethyl cellulose 0.8-1.2%
      3.sodium lauryl sulfate 1.5-2.5%
      4.glycerol 20-30%
      5.saccharin 0.1-0.3%
      6.flavor oil 1.0-2.5%
      7. water q.s. to 100%
         A typical procedure for preparing the formulation includes the steps of (i) mixing by a blender according to the foregoing formulation to provide a toothpaste, and (ii) adding a composition of this invention and blending the resultant mixture till homogeneous.
   ii. Tooth Powder
   iii. Oral Rinse
   iv. Tooth Whiteners
   v. Denture Adhesive
e) Health Care Devices
   i. Dental Floss
   ii. Toothbrushes
   iii. Respirators
   iv. Scented/flavored condoms
f) Feminine Hygiene Products such as Tampons, Feminine Napkins and Wipes, and Pantiliners
g) Personal Care Products: Cosmetic or pharmaceutical preparations, e.g., a "water-in-oil" (W/O) type emulsion, an "oil-in-water" (O/W) type emulsion or as multiple emulsions, for example of the water-in-oil-in-water (W/O/W) type, as a PIT emulsion, a Pickering emulsion, a micro-emulsion or nano-emulsion; and emulsions which are particularly preferred are of the "oil-in-water" (O/W) type or water-in-oil-in-water (W/O/W) type. More specifically,
   i. Personal Cleansers (bar soaps, body washes, and shower gels)
   ii. In-shower conditioner
   iii. Sunscreen ant tattoo color protection (sprays, lotions, and sticks)
   iv. Insect repellants
   v. Hand Sanitizer
   vi. Antiinflammatory balms, ointments, and sprays
   vii. Antibacterial ointments and creams
   viii. Sensates
   ix. Deodorants and Antiperspirants including aerosol and pump spray antiperspirant, stick antiperspirant, roll-on antiperspirant, emulsion spray antiperspirant, clear emulsion stick antiperspirant, soft solid antiperspirant, emulsion roll-on antiperspirant, clear emulsion stick antiperspirant, opaque emulsion stick antiperspirant, clear gel antiperspirant, clear stick deodorant, gel deodorant, spray deodorant, roll-on, and cream deordorant.
   x. Wax-based Deodorant. An exemplary formulation as follows:
      1.Parafin Wax 10-20%
      2.Hydrocarbon Wax 5-10%
      3.White Petrolatum 210-15%
      4.Acetylated Lanolin Alcohol 2-4%
      5.Diisopropyl Adipate 4-8%
      6.Mineral Oil 40-60%
      7.Preservative (as needed)
         The formulation is prepared by (i) mixing the above ingredients, (ii) heating the resultant composition to 75 °C until melted, (iii) with stirring, adding 4% cryogenically ground polymer containing a fragrance while maintaining the temperature 75 °C, and (iv) stirring the resulting mixture in order to ensure a uniform suspension while a composition of this invention is added to the formulation.
   xi. Glycol/Soap Type Deodorant. An exemplary formulation as follows:
      1.Propylene Glycol 60-70%
      2.Sodium Stearate 5-10%
      3.Distilled Water 20-30%
      4.2,4,4-Trichloro-2'- Hydroxy Diphenyl Ether, manufactured by the Ciba-Geigy Chemical Company and a Trademark of the Ciba-Geigy Chemical Company) 0.01-0.5%
         The ingredients are combined and heated to 75 °C with stirring until the sodium stearate has dissolved. The resulting mixture is cooled to 40 °C followed by addition of a composition of this invention.
   xii. Lotion including body lotion, facial lotion, and hand lotion
   xiii. Body powder and foot powder
   xiv. Toiletries
   xv. Body Spray
   xvi. Shave cream and male grooming products
   xvii. Bath Soak
   xviii. Exfoliating Scrub
h) Personal Care Devices
   i. Facial Tissues
   ii. Cleansing wipes
i) Hair Care Products
   i. Shampoos (liquid and dry powder)
   ii. Hair Conditioners (Rinse-out conditioners, leave-in conditioners, and cleansing conditioners)
   iii. Hair Rinses
   iv. Hair Refreshers
   v. Hair perfumes
   vi. Hair straightening products
   vii. Hair styling products, Hair Fixative and styling aids
   viii. Hair combing creams
   ix. Hair wax
   x. Hair foam, hair gel, nonaerosol pump spray
   xi. Hair Bleaches, Dyes and Colorants
   xii. Perming agents
   xiii. Hair wipes
j) Beauty Care
   i. Fine Fragrance - Alcoholic. Compositions and methods for incorporating fragrance capsules into alcoholic fine fragrances are described in US 4,428,869. Alcoholic fine fragrances may contain the following:
      1.Ethanol (1-99%)
      2.Water (0-99%)
      3.A suspending aide including but not limited to: hydroxypropyl cellulose, ethyl cellulose, silica, microcrystalline cellulose, carrageenan, propylene glycol alginate, methyl cellulose, sodium carboxymethyl cellulose or xanthan gum (0.-1-%)
      4.Optionally an emulsifier or an emollient may be included including but not limited to those listed above
   ii. Solid Perfume
   iii. Lipstick/lip balm
   iv. Make-up cleanser
   v. Skin care cosmetic such as foundation, pack, sunscreen, skin lotion, milky lotion, skin cream, emollients, skin whitening
   vi. Make-up cosmetic including manicure, mascara, eyeliner, eye shadow, liquid foundation, powder foundation, lipstick and cheek rouge
k) Consumer goods packaging such as fragranced cartons, fragranced plastic bottles/boxes
l) Pet care products
   i. Cat litter
   ii. Flea and tick treatment products
   iii. Pet grooming products
   iv. Pet shampoos
   v. Pet toys, treats, and chewables
   vi. Pet training pads
   vii. Pet carriers and crates
m) Confectionaries confectionery, preferably selected from the group consisting of chocolate, chocolate bar products, other products in bar form, fruit gums, hard and soft caramels and chewing gum
   i. Gum
      1.Gum base (natural latex chicle gum, most current chewing gum bases also presently include elastomers, such as polyvinylacetate (PVA), polyethylene, (low or medium molecular weight) polyisobutene (PIB), polybutadiene, isobutene-isoprene copolymers (butyl rubber), polyvinylethylether (PVE), polyvinylbutyether, copolymers of vinyl esters and vinyl ethers, styrene-butadiene copolymers (styrene-butadiene rubber, SBR), or vinyl elastomers, for example based on vinylacetate/vinyllaurate, vinylacetate/vinylstearate or ethylene/vinylacetate, as well as mixtures of the mentioned elastomers, as described for example in EP 0 242 325, U.S. Pat. No. 4,518,615, U.S. Pat. No. 5,093,136, U.S. Pat. No. 5,266,336, U.S. Pat. No. 5,601,858 or U.S. Pat. No. 6,986,709.) 20-25%
      2.Powdered sugar 45-50%
      3.glucose 15-17%
      4.starch syrup 10-13%
      5.plasticizer 0.1%
      6.flavor 0.8-1.2%
         The components described above were kneaded by a kneader according to the foregoing formulation to provide a chewing gum. Encapsulated Flavor or sensate is then added and blended till homogeneous.
   ii. Breath Fresheners
   iii. Orally Dissolvable Strips
   iv. Chewable Candy
   v. Hard Candy
n) Baked products, preferably selected from the group consisting of bread, dry biscuits, cakes and other cookies;
o) snack foods, preferably selected from the group consisting of baked or fried potato chips or potato dough products, bread dough products and corn or peanut-based extrudates;
   i. Potato, tortilla, vegetable or multigrain chips
   ii. Popcorn
   iii. Pretzels
   iv. Extruded stacks
p) Cereal Products preferably selected from the group consisting of breakfast cereals, muesli bars and precooked finished rice products
q) Alcoholic and non-alcoholic beverages, preferably selected from the group consisting of coffee, tea, wine, beverages containing wine, beer, beverages containing beer, liqueurs, schnapps, brandies, sodas containing fruit, isotonic beverages, soft drinks, nectars, fruit and vegetable juices and fruit or vegetable preparations; instant beverages, preferably selected from the group consisting of instant cocoa beverages, instant tea beverages and instant coffee beverages
   i. Ready to drink liquid drinks
   ii. Liquid Drink Concentrates
   iii. Powder Drinks
   iv. Coffee: Instant Cappucino
      1.Sugar 30-40%
      2.Milk Powder 24-35%
      3.Soluble Coffee 20-25%
      4.Lactose 1-=15%
      5.Food Grade Emulsifier 1-3%
      6.Encapsulated Volatile Flavor .01-0.5%
   v. Tea
   vi. Alcoholic
r) Spice blends and consumer prepared foods
   i. Powder gravy, sauce mixes
   ii. Condiments
   iii. Fermented Products
s)Ready to heat foods: ready meals and soups, preferably selected from the group consisting of powdered soups, instant soups, precooked soups
   i. Soups
   ii. Sauces
   iii. Stews
   iv. Frozen entrees
t) Dairy Products milk products, preferably selected from the group consisting of milk beverages, ice milk, yogurt, kefir, cream cheese, soft cheese, hard cheese, powdered milk, whey, butter, buttermilk and partially or fully hydrolyzed milk protein-containing products Flavored milk beverages
   i. Yoghurt
   ii. Ice cream
   iii. Bean Curd
   iv. Cheese
u) Soya protein or other soybean fractions, preferably selected from the group consisting of soya milk and products produced therefrom, soya lecithin-containing preparations, fermented products such as tofu or tempeh or products produced therefrom and soy sauces;
v) Meat products, preferably selected from the group consisting of ham, fresh or raw sausage preparations, and seasoned or marinated fresh or salt meat products
w) Eggs or egg products, preferably selected from the group consisting of dried egg, egg white and egg yolk
x) Oil-based products or emulsions thereof, preferably selected from the group consisting of mayonnaise, remoulade, dressings and seasoning preparations
y) fruit preparations, preferably selected from the group consisting of jams, sorbets, fruit sauces and fruit fillings; vegetable preparations, preferably selected from the group consisting of ketchup, sauces, dried vegetables, deep-frozen vegetables, precooked vegetables, vegetables in vinegar and preserved vegetables
z) Flavored pet foods.

The above-listed applications are all well known in the art. For example, fabric softener systems are described in US Patent Nos. 6,335,315, 5,674,832, 5,759,990, 5,877,145, 5,574,179; 5,562,849, 5,545,350, 5,545,340, 5,411,671, 5,403,499, 5,288,417, and 4,767,547, 4,424,134. Liquid laundry detergents include those systems described in U.S. Patent Nos. 5,929,022, 5,916,862, 5,731,278, 5,565,145, 5,470,507, 5,466,802, 5,460,752, 5,458,810, 5,458,809, 5,288,431, 5,194,639, 4,968,451, 4,597,898, 4,561,998, 4,550,862, 4,537,707, 4,537,706, 4,515,705, 4,446,042, and 4,318,818. Liquid dish detergents are described in U.S. Patent Nos. 6,069,122 and 5,990,065. Shampoo and conditioners that can employ the present invention include those described in US Patent Nos. 6,162,423, 5,968,286, 5,935,561, 5,932,203, 5,837,661, 5,776,443, 5,756,436, 5,661,118, 5,618,523, 5,275,755, 5,085,857, 4,673,568, 4,387,090 and 4,705,681. Automatic Dish Detergents are described in U.S. Pat. Nos. 6,020,294, 6,017,871, 5,968,881, 5,962,386, 5,939,373, 5,914,307, 5,902,781, 5,705,464, 5,703,034, 5,703,030, 5,679,630, 5,597,936, 5,581,005, 5,559,261, 4,515,705, 5,169,552, and 4,714,562.

All parts, percentages and proportions refer to herein and in the claims are by weight unless otherwise indicated.

The values and dimensions disclosed herein are not to be understood as being strictly limited to the exact numerical values recited. Instead, unless otherwise specified, each such value is intended to mean both the recited value and a functionally equivalent range surrounding that value. For example, a value disclosed as "50%" is intended to mean "about 50%."

The terms "capsule" and "microcapsule" herein are used interchangeably.

All publications cited herein are incorporated by reference in their entirety.

The invention is described in greater detail by the following non-limiting examples. Without further elaboration, it is believed that one skilled in the art can, based on the description herein, utilize the present invention to its fullest extent.

### EXAMPLE 1

Three aggregates of this invention, i.e., Aggregates 1-3, were prepared in this example and Examples 2 and 3 below.

### Preparing of Polyurea (PU) capsule

PU capsules for forming Aggregates 1 and 2 were prepared following the procedure below.

Fragrance Apple (25.8 g; International Flavors and Fragrance Inc., Union Beach) was mixed with NEOBEE oil (6.4 g; Stepan) and Lupranate M20 (2.6 g; polymeric methylene diphenyl diisocyanate; purchased from BASF), to form an oil phase. Subsequently, the oil phase was emulsified into an aqueous solution of 1.9% Morwet D425 (42.8 g; a sodium salt of naphthalene sulfonate condensate; AkzoNobel) under high shearing (IKA - ULTRA TURRAX, T25 Basic) at 9500 rpm for three minutes. The resultant fragrance emulsion was heated to 35 °C and 2.9 g of 40% hexamethylene diamine was added under constant mixing with an overhead mixer. After 15 minutes of stirring at 35 °C, the capsule slurry was cured at 55 °C for two hours and then cooled to room temperature to obtain a capsule dispersion.

### Mixing the PU capsule with a deposition polymer and a binder polymer

To the above freshly prepared PU capsule dispersion (80.5 g) was added 9.5 g of Lupamin 9095 (Polyvinylamine commercially available from BASF, a deposition polymer) under agitation until a homogeneous mixture was obtained. Subsequently, 10 g of 5% Walocel CRT 50000 PA (carboxymethylcellulose commercially available from Dow, a binder polymer) was added and homogenized a Silverson shear mixer at 4000 - 6000 rpm for one minute to obtain a polymeric mixture.

### Coacervating the PU capsule

While the above freshly prepared polymeric mixture (100 g) was stirred at 200 - 600 rpm, 2.6 g of 50% lactic acid aqueous solution was added during a period of 1 to 3 minutes. The resultant slurry was stirred for additional 15 minutes to obtain an aggregate of this invention, i.e., Aggregate 1.

The particle size was measured by microscope imaging. The aggregate has a size of about 20 to 120 µm. See Figure 1a.

Aggregate 1 was formulated in a hair conditioner base and tested for its perfumery benefit. See Example 7 below.

### EXAMPLE 2

Another aggregate of this invention, Aggregate 2, was prepared following a procedure similar to that described in Example 1.

More specifically, to a PU capsule dispersion (73.8 g) was added 9.5 g of Lupamin 9095 under agitation until a homogeneous mixture was obtained. Subsequently, 16.7 g of 3% alginate polymer (RF-6650; commercially available from FMC BioPolymer) was added and homogenized using a Silverson shear mixer at 4000 - 6000 rpm for one minute to obtain a polymeric mixture. Under agitation at 200 - 600 rpm, 10 g of 0.05% sodium sulfate was added and the mixture was stirred using an overhead mixer at 200 - 250 rpm. 2.1 g of 50% of lactic acid solution was added during a period of 1 to 3 minutes under agitation. The resultant slurry was stirred for additional 15 minutes to obtain Aggregate 2.

The particle size was determined by microscope imaging to be 10 to 50 µm. See Figure 1b.

The performance test of Aggregate 2 was evaluated following the procedure in Example 7 in a hair conditioner base.

### EXAMPLE 3

A third aggregate, i.e., Aggregate 3, was prepared following the procedure below. Unlike in Examples 1 and 2, the binder polymer Walocel CRT 50000 PA was used as a dispersant for preparing the PU capsule incorporated in the aggregate.

More specifically, 25.9 g of fragrance Apple (International Flavors and Fragrance Inc., Union Beach) was mixed with 6.5 g of NEOBEE oil (Stepan) and 3.2 g of polyisocyanate Lupranate M20 to form an oil phase. In a separate beaker, an aqueous phase was prepared by dissolving 5 g of 10% Morwet D425 and 15 g of 2% Walocel CRT 50000 PA in 21.1 g of water. The oil phase was then emulsified into the aqueous phase to form a fragrance emulsion under high shearing at 9500 rpm for three minutes. After the fragrance emulsion was heated to 35 °C, 3.6 g of 40% hexamethylene diamine was added under constant stirring. The resultant slurry was stirring for additional 15 minutes and then cured at 55 °C for two hours to obtain a capsule dispersion.

At 55 °C, 9.5 g of Lupamin 9095 was added to the capsule dispersion under stirring, followed by the addition of 10 g of 2% Walocel CRT 50000 PA. The resultant polymeric mixture was homogenized at 4000 - 6000 rpm for one minute and then cooled to room temperature. After 1 hour, the polymeric mixture was stirred at 200 - 600 rpm and 2.8 g of 50% lactic acid aqueous solution was added during a period of 1 to 3 minutes. Continuous stirring for additional 15 minutes resulted in a homogeneous mixture to obtain Aggregate 3.

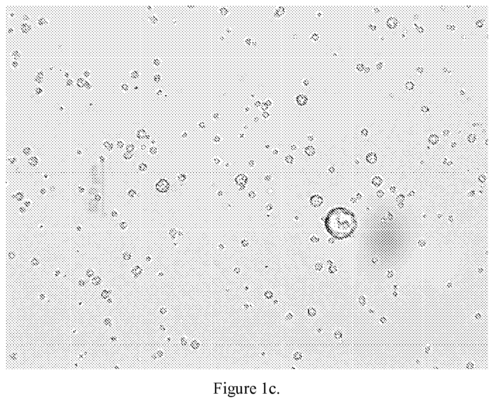

The particle size of the aggregate was determined by microscope imaging to be 20 to 120 µm. See Figure 1c.

The performance of Aggregate 3 in a hair conditioner base was evaluated following the procedure in Example 7.

### EXAMPLE 4

A fourth aggregate, i.e., Aggregate 4, was prepared following the procedure below.

A PU capsule dispersion (82.8 g) was first prepared as shown in Example 1 using an aqueous solution of 1.9% Morwet D425 and Luviksol K90. To the capsule dispersion was added 0.5 g of aminated polydimethylsiloxane (commercially available as Silamine T-SA from SILTECH CORPORATION) under agitation until a homogeneous mixture was obtained. Subsequently, 16.7 g of 3% FMC RF-6650 was added and homogenized a Silverson shear mixer at 4000 - 6000 rpm for one minute to obtain a polymeric mixture. Under agitation at 200 - 600 rpm, 10 g of 0.05% sodium sulfate was added and the mixture was stirred using an overhead mixer at 200 - 250 rpm. 1.7 g of 50% of lactic acid solution was added during a period of 1 to 3 minutes under agitation. The resultant slurry was stirred for additional 15 minutes to obtain Aggregate 4.

The performance of Aggregate 4 in a hair conditioner base was evaluated following the procedure in Example 7.

### EXAMPLE 5

A fifth aggregate, i.e., Aggregate 5, was prepared following the procedure below. To a PU capsule dispersion (83.8 g) prepared using an aqueous solution of 1.9% Morwet D425 and Luviksol K90 was added 2.4 g of Lupamin 9095 and 0.5 g of Silamine T-SA under agitation until a homogeneous mixture was obtained. Subsequently, 13.3 g of 3% FMC RF-6650 was added and homogenized a Silverson shear mixer at 4000 - 6000 rpm for one minute to obtain a polymeric mixture. Under agitation at 200 - 600 rpm, 10 g of 0.05% sodium sulfate was added and the mixture was stirred using an overhead mixer at 200 - 250 rpm. 2.4 g of 50% of lactic acid solution was added during a period of 1 to 3 minutes under agitation. The resultant slurry was stirred for additional 15 minutes to obtain Aggregate 5.

The performance of Aggregate 5 in a hair conditioner base was evaluated following the procedure in Example 7.

### EXAMPLE 6

Another aggregate of this invention, Aggregate 6, was prepared following a procedure similar to that described in Example 1.

To a PU capsule dispersion (73.8 g) was added 9.5 g of Lupamin 9095 under agitation until a homogeneous mixture was obtained. Subsequently, 16.7 g of 3% FMC RF-6650 was added and homogenized a Silverson shear mixer at 4000 - 6000 rpm for one minute to obtain a polymeric mixture. Under agitation at 200 - 600 rpm, 10 g of 0.05% sodium sulfate was added and the mixture was stirred using an overhead mixer at 200 - 250 rpm. 2.1 g of 50% of lactic acid solution was added during a period of 1 to 3 minutes under agitation. The resultant slurry was stirred for additional 15 minutes and then was added further 3.3 g of 30% cetyl trimethyl ammonium chloride solution (commercially available as Maquat CTAC from Pilot Chemical Company) under constant mixing to obtain Aggregate 6.

The performance of Aggregate 6 in a hair conditioner base was evaluated following the procedure in Example 7.

### EXAMPLE 7

Aggregates 1-6 were each formulated in a hair conditioner base to prepare Samples 1-6 for performance evaluation.

Aggregate 1 was blended into a model Hair Conditioner (commercially available from Magick Botanical) at high shear, 4000-6000 rpm for 1 - 2 minutes. The amount of the aggregate added was 0.5% fragrance oil equivalent.

Sample 1 thus prepared (2 g) was added to 2 bundles hair swatch (8 strands) that was wetted under water, with excess water squeezed lightly. After the hair was lathered, the hair swatches were rinsed under a stream of water (38 °C, 1 gal/min) for 45 seconds. Excess water from hair was removed. Hair swatches were then line-dried for 24 hours followed by sensory evaluation by a panel of judges. The fragrance intensity was rated on a scale ranging from 0 to 10. A numerical value of 5 indicated the hair swatches produced a strong intensity, while a value of 10 indicated the hair swatches generated a very strong smell. One hair swatch was evaluated without brushing with a comb to obtain the pre-brush fragrance intensity and the other was used to obtain the post-brush fragrance intensity after brushing it with a brush.

Aggregates 2 - 6 were also formulated into Samples 2 - 6, respectively, and evaluated following the same procedures above except that different aggregates were used.

Seven comparative samples, *i.e.,* Comparative 1-7, were also prepared following the procedure above except that PU capsules, instead of capsule aggregates, were used.

Two comparative samples (i.e., Comparatives 1 and 2) were also prepared and evaluated following the same procedures described above except that capsules, not aggregates were used.

In Comparative 1, the PU capsule prepared in Example 1 above was used.

In Comparative 2, the pH of the PU capsule was adjusted with the same amount of lactic acid used to prepare Aggregate 1.

In Comparative 3, the same amount of binder polymer Walocel CRT 50000 PA was added to the PU capsule.

In Comparative 4, to the PU capsule were added the same amounts of the binder polymer and lactic acid as used to prepare Aggregate 1.

In Comparative 5, to the PU capsule was added the same amount of deposition polymer Lupamin 9095 as used to prepare Aggregate 1.

In Comparative 6, to the PU capsule were added the same amounts of deposition polymer Lupamin 9095 and lactic acid as used to prepare Aggregate 1.

In Comparative 7, to the PU capsule were added the same amounts of binder polymer Walocel CRT 50000 PA and deposition polymer Lupamin 9095 as used to prepare Aggregate 1. No lactic acid was added to induce the controlled coacervation to form the aggregate of this invention.

These seven comparative samples were evaluated together with Samples 1-6.

Summarized in Table 2 below are post-brush intensity obtained from the evaluation results.

**TABLE 2. Post-brush intensity**

| **Sample** | Post-brush intensity |
|---|---|
| Sample 1 | 5.0 |
| Sample 2 | 6.3 |
| Sample 3 | 5.4 |
| Sample 4 | 4.4 |
| Sample 5 | 4.4 |
| Sample 6 | 5.8 |
| Comparative 1 | 2 |
| Comparative 2 | 1.4 |
| Comparative 3 | 1.7 |
| Comparative 5 | 1.9 |
| Comparative 4 | 2.1 |
| Comparative 6 | 2.3 |
| Comparative 7 | 2.8 |

As shown in the table above, Samples 1, 2, 3, 4, 5 and 6 each showed post-brush fragrance intensities of 6.3, 5.6, 5.4, 4.4, 4.4, 5.8 respectively. By contrast, Comparatives 1-7 each had a post-brush fragrance 2.8 or less, respectively. Samples 1-6 of this invention had, unexpectedly, much higher post-brush fragrance intensity than both the capsule itself and the simple mixture of the capsule, binder polymer and deposition polymer without controlled coacervation.

The pre-brush fragrance intensities were also determined. Again, Samples 1-6 had higher pre-brush fragrance intensities than Comparatives 1 and 2. For example, Sample 2 had a pre-brush fragrance intensity of 0.9 and Comparative 1 had an intensity of 0.2.

Additionally, to evaluate storage stability, viscosity of Samples 2 and 6 were measured by placing 2-3 g of the Aggregate 1-6 on Haake RheoStress 1. Viscosity readings of Sample 2 and Sample 6 were taken at 2 s⁻¹, 21 s⁻¹ and 95 s⁻¹ measured in mPa·S initially (T = 0) and after two weeks aged at room temperature. See Table 3 below for the results.

**TABLE 3. Viscosity of Samples 2 and 6 of this invention**

| **Sample** | **Viscosity** (mPa·S) | | |
|---|---|---|---|
| | **2 s⁻¹** | **21 s⁻¹** | **95 s⁻¹** |
| Sample 2 at T = 0 | 17 | 17 | 19 |
| Sample 2 at T = 2 weeks at RT | 24900 | 2474 | 1027 |
| Sample 6 at T = 0 | 2235 | 470 | 129 |
| Sample 6 at T = 2 weeks at RT | 308 | 86 | 64 |

As shown in the table above the viscosity of Sample 2, although initially low, dramatically increased overtime after just 2 weeks. Sample 6 unexpectedly with the addition of Maquat CTAC solution showed very low viscosity overtime for 2 weeks at room temperature.

### OTHER EMBODIMENTS

All of the features disclosed in this specification may be combined in any combination.

Indeed, to achieve the purpose of preparing an aggregate containing multiple capsules, one or more binder polymers, and one or more deposition polymers, one skilled in the art can design and prepare an aggregate by using different capsules, binder polymers, and deposition polymers, and acids, varying the concentrations of the capsules, binder polymers, and deposition polymers to achieve desirable organoleptic or release profiles in a consumer product. Further, the ratios among the capsules, the binder polymers, and the deposition polymers can also be determined by a skilled artisan through assays described in this application or those known in the art. The particle size of the aggregate can be controlled by adjusting the addition rate of the acid to form coacervation and/or by using different acids, capsules, binder polymers, deposition polymers, and different combinations thereof, which can be readily determined by a skilled artisan after certain optimization.

From the above description, a skilled artisan can easily ascertain the essential characteristics of the present invention, and without departing from the scope thereof, can make various changes and modifications of the invention to adapt it to various usages and conditions. Thus, other embodiments are also within the claims.

## Claims

1. An aggregate comprising:
two or more benefit particles each containing an active material and a polymeric material that immobilizes the active material;
one or more binder polymers each having an anionic chemical group that is negatively charged or capable of being negatively charged; and
one or more deposition polymers each having a cationic chemical group that is positively charged or capable of being positively charged,
wherein the aggregate, carrying one or more net positive charges or being neutral, has a particle size of 1 to 200 µm; each of the binder polymers and the deposition polymers has a molecular weight of 5,000 to 10,000,000 Daltons; each of the benefit particles, having a particle size of 0.1 to 50 µm, bonds to at least one of the binder polymer and the deposition polymer, or to both via a covalent or non-covalent bond; and at least one of the binder polymers bonds to one of the deposition polymers via a covalent or non-covalent bond.

2. The aggregate of claim 1, wherein the covalent bond is via a cross-linker selected from the group consisting of a urea-formaldehyde precondensate, a melamine-formaldehyde precondensate, an aldehyde-based cross-linker, a water soluble carbodiimide, a polyfunctional aziridine, a polyfunctional epoxy, a polyfunctional oxazoline, a polyfunctional acrylate, a polyfunctional methacrylate, a polyfunctional cyano acrylate, a polyfunctional isocyanate, a polyfunctional acrylamide, a polyfunctional acetyl acetonate, a polyfunctional levulinate, and a combination thereof, and wherein the weight ratio of the cross-linker and the benefit particles is 0.00002 : 1 to 10 : 1, and preferably, 0.01 : 1 to 5 : 1.

3. The aggregate of claims 1 or 2, wherein the polymer combination theoretical charge-to-weight ratio is 0 to +0.2, more preferably +0.01 to +0.1.

4. The aggregate of any one of claims 1-3, wherein each of the binder polymers and the deposition polymers has a molecular weight of 10,000 to 1,000,000 Daltons, and the active material is selected from the group consisting of a fragrance, flavoring agent, fungicide, brightener, antistatic agent, wrinkle control agent, fabric softener active, hard surface cleaning active, skin or hair conditioning agent, antimicrobial active, UV protection agent, insect repellent, animal repellent, vermin repellent, flame retardant, and combination thereof.

5. The aggregate of any one of claims 1-4, wherein each one of the one or more binder polymers, independently, is a polymer or copolymer of a monomer selected from the group consisting of acrylic acid, crotonic acid, methacrylic acid, maleic anhydride, ethylene maleic anhydride, an acrylate, a methacrylate, an acrylamide, a sulfonated monomer, a sulfated monomer, and a phenolic monomer.

6. The aggregate of any one of claims 1-5, wherein the one or more binder polymers are selected from the group consisting of a carboxyl methyl cellulose, an alginic acid, xanthan gum, carrageenan, agar, carboxyl ethyl cellulose, gum arabic, bacterial alginate, fucogalactan, fucoidan, gellan gum, gum ghatti, gum karaya, gum tragacanth, pectin, propylene glycol alginate, psyllium seed gum, sodium alginate, welan gum, and a combination thereof.

7. The aggregate of any one of claims 1-6, wherein the anionic chemical group is a carboxyl group, a phenolic group, a sulfonic group, a sulfinic group, or an organophosphorus group, the cationic chemical group is an amine group, a pyridine group, an imidazole group, an ethylenimine group, or an amide group, and the aggregate has a particle size of 1 to 120 µm and has one or more positive charges.

8. The aggregate of any one of claims 1-7, wherein each of the one or more deposition polymers, independently, is a polymer or copolymer of a monomer selected from the group consisting of diallyldimethylammonium chloride, methacrylamidopropyl trimethylammonium chloride, N,N-dimethylaminoethyl methacrylate, vinyl pyridine, quaternized vinyl pyridine, vinyl amine, allyl amine, vinyl imidazoline, vinyl imidazole, vinyl imidazolinium, dimethylaminoethyl methacrylate, dimethylaminopropyl, methacryloylaminopropyl lauryldimonium chloride, amino-functionalized silicone, and ethylenimine.

9. The aggregate of any one of claims 1-8, wherein each of the benefit particles is a capsule having a core that contains an active material and a wall that encapsulates the active material, the active material is a fragrance, the capsule has a particle size of 0.1 to 50 µm, and the wall of the capsule is formed of a urea-formaldehyde polymer, a melamine-formaldehyde polymer, a phenolic-formaldehyde polymer, a urea-glutaraldehyde polymer, a melamine-glutaraldehyde polymer, a phenolic-glutaraldehyde polymer, polyurea, polyurethane, polyacrylate, polyamide, polyester, an epoxy cross-linked polymer, a polyfunctional carbodiimide cross-linked polymer, silica, a silica-derived material, or a combination thereof.

10. The aggregate of claim 9, wherein each of the capsules is negatively charged and bonds to one of the deposition polymers via a hydrogen bond, a dipolar interaction, or an ionic interaction, and one of the binder polymers bonds to one of the deposition polymers via an ionic interaction.

11. The aggregate of claim 9, wherein the capsule is positively charged and bonds to one of the binder polymers via ionic interactions, and one of the binder polymers bonds to one of the deposition polymers also via an ionic interaction.

12. The aggregate of any one of the preceding claims, further comprising a viscosity stabilizer, and wherein the binder or deposition polymer is a part of the capsule wall.

13. A process of preparing an aggregate, the process comprising:
(a) providing a benefit particle dispersion containing benefit particles in water, each of the benefit particles containing an active material and a polymeric material that immobilizes the active material,
(b) adding one or more binder polymers and one or more deposition polymers to obtain a polymeric mixture, each of the binder polymers having an anionic chemical group that is negatively charged or capable of being negatively charged, and each of the deposition polymers having a cationic chemical group that is positively charged or capable of being positively; and
(c) causing formation of the aggregate in the polymeric mixture induced by an coacervation event, the coacervation event being pH adjusting, heating, UV irradiating, sonicating, radio irradiating, ionizing, exposing to an enzyme or bacterium, addition of salt or non-solvent, a chemical reaction, or a combination thereof,
wherein each of the benefit particles is a capsule, the active material is selected from the group consisting of a fragrance, flavoring agent, fungicide, brightener, antistatic agent, wrinkle control agent, fabric softener active, hard surface cleaning active, skin conditioning agent, hair conditioning agent, antimicrobial active, UV protection agent, insect repellent, animal repellent, vermin repellent, flame retardant, and a mixture thereof; each of the binder polymers and the deposition polymers has a molecular weight of 10,000 to 1,000,000 Daltons; each of the one or more binder polymers, independently, is a polymer or copolymer of a monomer selected from the group consisting of acrylic acid, acrylate, methacrylic acid, methacrylate, acrylamide, polyacrylic acid, polyacrylate, polymethacrylic, polymethacrylate, polyacrylamide, maleic anhydride, a sulphated monomer, and a phenolic monomer; and each of the one or more deposition polymers is, independently, a polymer or copolymer of a monomer selected from the group consisting of diallyldimethylammonium chloride, methacrylamidopropyl trimethylammonium chloride, N,N-dimethylaminoethyl methacrylate, vinyl pyridine, quaternized vinyl pyridine, vinyl amine, allyl amine, vinyl imidazoline, vinyl imidazole, vinyl imidazolinium, dimethylaminoethyl methacrylate, dimethylaminopropyl, methacryloylaminopropyl lauryldimonium chloride, amino-functionalized silicone, and ethylenimine.

14. A consumer product containing an aggregate of any one of claims 1-12, optionally the consumer product further comprising one or more different aggregate, a free active material, one or more free capsules, or a combination thereof, and wherein the consumer product is a shampoo, hair conditioner, personal wash, fabric detergent, fabric softener, fabric conditioner, hard surface cleaner, body wash, soap bar, scenter booster, liquid detergent, or powder detergent.

## Patentansprüche

1. Aggregat, umfassend:
zwei oder mehr Wirkstoffpartikel, die jeweils ein aktives Material und ein Polymermaterial, das das aktive Material immobilisiert, enthalten;
ein oder mehrere Bindepolymere, die jeweils eine anionische chemische Gruppe aufweisen, die negativ geladen ist oder fähig ist, negativ geladen zu sein; und
ein oder mehrere Abscheidungspolymere, die jeweils eine kationische chemische Gruppe aufweisen, die positiv geladen ist oder fähig ist, positiv geladen zu sein;
wobei das Aggregat, das eine oder mehrere positive Nettoladungen trägt oder neutral ist, eine Partikelgröße von 1 bis 200 µm aufweist; jedes der Bindepolymere und der Abscheidungspolymere ein Molekulargewicht von 5.000 bis 10.000.000 Dalton aufweist; jedes der Wirkstoffpartikel, das eine Partikelgröße von 0,1 bis 50 µm aufweist, über eine kovalente oder nichtkovalente Bindung an wenigstens eines von dem Bindepolymer und dem Abscheidungspolymer oder beide gebunden ist; und
wenigstens eines der Bindepolymere über eine kovalente oder nichtkovalente Bindung an eines der Abscheidungspolymere gebunden ist.

2. Aggregat gemäß Anspruch 1, wobei die kovalente Bindung über einen Vernetzer ausgewählt aus der Gruppe bestehend aus einem Harnstoff-Formaldehyd-Präkondensat, einem Melamin-Formaldehyd-Präkondensat, einem Vernetzer auf Aldehydbasis, einem wasserlöslichen Carbodiimid, einem polyfunktionellen Aziridin, einem polyfunktionellen Epoxid, einem polyfunktionellen Oxazolin, einem polyfunktionellen Acrylat, einem polyfunktionellen Methacrylat, einem polyfunktionellen Cyanoacrylat, einem polyfunktionellen Isocyanat, einem polyfunktionellen Acrylamid, einem polyfunktionellen Acetylacetonat, einem polyfunktionellen Levulinat und einer Kombination davon vorliegt und wobei das Gewichtsverhältnis des Vernetzers und des Wirkstoffpartikels 0,00002:1 bis 10:1 beträgt, vorzugsweise 0,01:1 bis 5:1.

3. Aggregat gemäß Ansprüchen 1 oder 2, wobei das theoretische Ladung-zu-Gewicht-Verhältnis der Polymerkombination 0 bis +0,2 beträgt, bevorzugter +0,01 bis +0,1.

4. Aggregat gemäß einem der Ansprüche 1-3, wobei jedes der Bindepolymere und der Abscheidungspolymere ein Molekulargewicht von 10.000 bis 10.000.000 Dalton aufweist und das aktive Material ausgewählt ist aus der Gruppe bestehend aus einem Duftstoff, Aromastoff, Fungizid, Aufheller, antistatischen Mittel, Faltenbeherrschungsmittel, Gewebeweichmacherwirkstoff, harte-Oberflächen-Reinigungswirkstoff, Haut- oder Haarkonditionierungsmittel, antimikrobiellen Wirkstoff, UV-Schutzmittel, Insektenabwehrmittel, Tierabwehrmittel, Schädlingsabwehrmittel, Flammhemmer und Kombinationen davon.

5. Aggregat gemäß einem der Ansprüche 1-4, wobei jedes von dem einen oder den mehreren Bindepolymeren unabhängig ein Polymer oder Copolymer eines Monomers ausgewählt aus der Gruppe bestehend aus Acrylsäure, Crotonsäure, Methacrylsäure, Maleinsäureanhydrid, Ethylenmaleinsäureanhydrid, einem Acrylat, einem Methacrylat, einem Acrylamid, einem sulfonierten Monomer, einem sulfatierten Monomer und einem phenolischen Monomer ist.

6. Aggregat gemäß einem der Ansprüche 1-5, wobei das eine oder die mehreren Bindepolymere ausgewählt sind aus der Gruppe bestehend aus einer Carboxymethylcellulose, einer Alginsäure, Xanthangummi, Carrageenan, Agar, Carboxyethylcellulose, Gummi arabicum, bakteriellem Alginat, Fucogalactan, Fucoidan Gellangummi, Ghattigummi, Karayagummi, Tragacanthgummi, Pektin, Propylenglycolalginat, Psylliumsamengummi, Natriumalginat, Welangummi und einer Kombination davon.

7. Aggregat gemäß einem der Ansprüche 1-6, wobei die anionische chemische Gruppe eine Carboxygruppe, eine Phenolgruppe, eine Sulfongruppe, eine Sulfingruppe oder eine Organophosphorgruppe ist, die kationische chemische Gruppe eine Amingruppe, eine Pyridingruppe, eine Imidazolgruppe, eine Ethylenimingruppe oder eine Amidgruppe ist und das Aggregat eine Partikelgröße von 1 bis 120 µm aufweist und eine oder mehrere positive Ladungen aufweist.

8. Aggregat gemäß einem der Ansprüche 1-7, wobei jedes von dem einen oder den mehreren Abscheidungspolymeren unabhängig ein Polymer oder Copolymer eines Monomers ausgewählt aus der Gruppe bestehend aus Diallyldimethylammoniumchlorid, Methacrylamidopropyltrimethylammoniumchlorid, N,N-Dimethylaminoethylmethacrylat, Vinylpyridin, quaternisiertem Vinylpyridin, Vinylamin, Allylamin, Vinylimidazolin, Vinylimidazol, Vinylimidazolinium, Dimethylaminoethylmethacrylat, Dimethylaminopropyl, Methacryloylaminopropyllauryldimoniumchlorid, aminofunktionalisiertem Silicon und Ethylenimin ist.

9. Aggregat gemäß einem der Ansprüche 1-8, wobei jedes der Wirkstoffpartikel eine Kapsel ist, die einen Kern, der ein aktives Material enthält, und eine Wand, die das aktive Material einkapselt, aufweist, wobei das aktive Material ein Duftstoff ist, die Kapsel eine Partikelgröße von 0,1 bis 50 µm aufweist und die Wand der Kapsel aus einem Harnstoff-Formaldehyd-Polymer, einem Melanin-Formaldehyd-Polymer, einem Phenol-Formaldehyd-Polymer, einem Harnstoff-Glutaraldehyd-Polymer, einem Melamin-Glutaraldehyd-Polymer, einem Phenol-Glutaraldehyd-Polymer, Polyharnstoff, Polyurethan, Polyacrylat, Polyamid, Polyester, einem epoxyvernetzten Polymer, einem mit polyfunktionellem Carbodiimid vernetzten Polymer, Siliciumdioxid, einem Siliciumdioxid-abgeleiteten Material oder einer Kombination davon besteht.

10. Aggregat gemäß Anspruch 9, wobei jede der Kapseln negativ geladen ist und über eine Wasserstoffbrücke, eine Dipolwechselwirkung oder eine ionische Wechselwirkung an eines der Abscheidungspolymere gebunden ist und eines der Bindepolymere über eine ionische Wechselwirkung an eines der Abscheidungspolymere gebunden ist.

11. Aggregat gemäß Anspruch 9, wobei die Kapsel positiv geladen ist und über ionische Wechselwirkungen an eines der Abscheidungspolymere gebunden ist und eines der Bindepolymere ebenfalls über eine ionische Wechselwirkung an eines der Abscheidungspolymere gebunden ist.

12. Aggregat gemäß einem der vorstehenden Ansprüche, ferner umfassend einen Viskositätsstabilisator, und wobei das Binde- oder Abscheidungspolymer ein Teil der Kapselwand ist.

13. Verfahren zur Herstellung eines Aggregats, wobei das Verfahren umfasst:
(a) Bereitstellen einer Wirkstoffpartikeldispersion, die Wirkstoffpartikel enthält, in Wasser, wobei jedes der Wirkstoffpartikel ein aktives Material und ein Polymermaterial, das das aktive Material immobilisiert, enthält;
(b) Zugeben eines oder mehrerer Bindepolymere und eines oder mehrerer Abscheidungspolymere, um ein Polymergemisch zu erhalten, wobei jedes der Bindepolymere eine anionische chemische Gruppe aufweist, die negativ geladen ist oder fähig ist, negativ geladen zu sein, und jedes der Abscheidungspolymere eine kationische chemische Gruppe aufweist, die positiv geladen ist oder fähig ist, positiv geladen zu sein; und
(c) Bewirken der Entstehung des Aggregats in dem Polymergemisch, induziert durch ein Koazervationsereignis, wobei das Koazervationsereignis pH-Wert-Einstellung, Erhitzen, UV-Bestrahlung, Ultraschallbehandlung, Radiowellenbestrahlung, Ionisation, Exposition gegenüber einem Enzym oder Bakterium, Zugabe von Salz oder Nichtlösungsmittel, eine chemische Reaktion oder eine Kombination davon ist,
wobei jedes der Wirkstoffpartikel eine Kapsel ist, das aktive Material ausgewählt ist aus der Gruppe bestehend aus einem Duftstoff, Aromastoff, Fungizid, Aufheller, antistatischen Mittel, Faltenbeherrschungsmittel, Gewebeweichmacherwirkstoff, harte-Oberflächen-Reinigungswirkstoff, Hautkonditionierungsmittel, Haarkonditionierungsmittel, antimikrobiellen Wirkstoff, UV-Schutzmittel, Insektenabwehrmittel, Tierabwehrmittel, Schädlingsabwehrmittel, Flammhemmer und einem Gemisch davon; jedes der Bindepolymere und der Abscheidungspolymere ein Molekulargewicht von 10.000 bis 10.000.000 Dalton aufweist; jedes von dem einen oder den mehreren Bindepolymeren unabhängig ein Polymer oder Copolymer eines Monomers ausgewählt aus der Gruppe bestehend aus Acrylsäure, Acrylat, Methacrylsäure, Methacrylat, Acrylamid, Polyacrylsäure, Polyacrylat, Polymethacryl, Polymethacrylat, Polyacrylamid, Maleinsäureanhydrid, einem sulfatierten Monomer und einem phenolischen Monomer ist; und jedes von dem einen oder den mehreren Abscheidungspolymeren unabhängig ein Polymer oder Copolymer eines Monomers ausgewählt aus der Gruppe bestehend aus Diallyldimethylammoniumchlorid, Methacrylamidopropyltrimethylammoniumchlorid, N,N-Dimethylaminoethylmethacrylat, Vinylpyridin, quaternisiertem Vinylpyridin, Vinylamin, Allylamin, Vinylimidazolin, Vinylimidazol, Vinylimidazolinium, Dimethylaminoethylmethacrylat, Dimethylaminopropyl, Methacryloylaminopropyllauryldimoniumchlorid, aminofunktionalisiertem Silicon und Ethylenimin ist.

14. Verbraucherprodukt, das ein Aggregat gemäß einem der Ansprüche 1-12 enthält, wobei das Verbraucherprodukt gegebenenfalls ferner ein oder mehrere andere Aggregate, ein freies aktives Material, eine oder mehrere freie Kapseln oder eine Kombination davon umfasst und wobei das Verbraucherprodukt ein/eine Shampoo, Haarkonditionierungsmittel, Körperspülung, Gewebedetergens, Gewebeweichmacher, Gewebekonditionierungsmittel, Reinigungsmittel für harte Oberflächen, Körperreinigungsmittel, Seifenstück, Duftverstärker, Flüssigdetergens oder Pulverdetergens ist.

## Revendications

1. Agrégat, comprenant :
deux, ou plus, particules bénéfiques contenant chacune une matière active et un matériau polymère qui immobilise la matière active ;
un ou plusieurs polymères liants, ayant chacun un groupement chimique anionique qui est négativement chargé ou capable d'être négativement chargé ; et
un ou plusieurs polymères de dépôt, ayant chacun un groupement chimique cationique qui est positivement chargé ou capable d'être positivement chargé ;
où l'agrégat, portant une ou plusieurs charges positives nettes ou étant neutre, possède une taille de particules allant de 1 à 200 µm ; chacun parmi les polymères liants et les polymères de dépôt possède un poids moléculaire allant de 5000 à 10 000 000 Daltons ; chacune parmi les particules bénéfiques, ayant une taille de particules allant de 0,1 à 50 µm, se lie avec au moins un parmi le polymère liant et le polymère de dépôt, ou les deux, via une liaison covalente ou non covalente ; et au moins l'un parmi les polymères liants se lie à l'un des polymères de dépôt via une liaison covalente ou non covalente.

2. Agrégat selon la revendication 1, dans lequel la liaison covalente se fait via un agent de réticulation choisi dans le groupe constitué par un pré-condensat d'urée-formaldéhyde, un pré-condensat de mélamine-formaldéhyde, un agent de réticulation à base d'aldéhyde, un carbodiimide hydrosoluble, une aziridine polyfonctionnelle, un époxy polyfonctionnel, une oxazoline polyfonctionnelle, un acrylate polyfonctionnel, un méthacrylate polyfonctionnel, un cyanoacrylate polyfonctionnel, un isocyanate polyfonctionnel, un acrylamide polyfonctionnel, un acétonate d'acétyle polyfonctionnel, un lévulinate polyfonctionnel, et une combinaison de ceux-ci, et où le rapport pondéral de l'agent de réticulation et des particules bénéfiques va de 0,00002:1 à 10:1, et préférablement de 0,01:1 à 5:1.

3. Agrégat selon les revendications 1 ou 2, dans lequel le rapport charge-poids théorique de la combinaison de polymères va de 0 à +0,2, plus préférablement de +0,01 à +0,1.

4. Agrégat selon l'une quelconque des revendications 1-3, dans lequel chacun parmi les polymères liants et les polymères de dépôt possède un poids moléculaire allant de 10 000 à 1 000 000 Daltons, et la matière active est choisie dans le groupe constitué par une fragrance, un agent aromatisant, un fongicide, un azurant optique, un agent antistatique, un agent antirides, un principe actif d'assouplissant textile, un principe actif de nettoyage des surfaces dures, un agent de conditionnement de la peau ou des cheveux, un principe actif antimicrobien, un agent de protection contre les UV, un répulsif pour insectes, un répulsif pour animaux, un répulsif pour vermines, un agent ignifuge, et une combinaison de ceux-ci.

5. Agrégat selon l'une quelconque des revendications 1-4, dans lequel chacun parmi les un ou plusieurs polymères liants, indépendamment, est un polymère ou copolymère d'un monomère choisi dans le groupe constitué par l'acide acrylique, l'acide crotonique, l'acide méthacrylique, l'anhydride maléique, l'anhydride maléique-éthylène, un acrylate, un méthacrylate, un acrylamide, un monomère sulfoné, un monomère sulfaté, et un monomère phénolique.

6. Agrégat selon l'une quelconque des revendications 1-5, dans lequel les un ou plusieurs polymères liants sont choisis dans le groupe constitué par la carboxyméthylcellulose, l'acide alginique, la gomme xanthane, un carraghénane, l'agar-agar, la carboxyéthylcellulose, la gomme arabique, un alginate bactérien, un fucogalactane, un fucoïdane, la gomme gellane, la gomme ghatti, la gomme de karaya, la gomme adragante, une pectine, un alginate-propylène glycol, la gomme de graines de psyllium, l'alginate de sodium, la gomme welane, et une combinaison de ceux-ci.

7. Agrégat selon l'une quelconque des revendications 1-6, dans lequel le groupement chimique anionique est un groupement carboxyle, un groupement phénolique, un groupement sulfonique, un groupement sulfinique, ou un groupement organophosphoré, le groupement chimique cationique est un groupement amine, un groupement pyridine, un groupement imidazole, un groupement éthylèneimine, ou un groupement amide, et l'agrégat possède une taille de particules allant de 1 à 120 µm et possède une ou plusieurs charges positives.

8. Agrégat selon l'une quelconque des revendications 1-7, dans lequel chacun parmi les un ou plusieurs polymères de dépôt, indépendamment, est un polymère ou copolymère d'un monomère choisi dans le groupe constitué par le chlorure de diallyldiméthylammonium, le chlorure de méthacrylamidopropyl triméthylammonium, le méthacrylate de N,N-diméthylaminoéthyle, la vinylpyridine, la vinylpyridine quaternisée, la vinylamine, l'allylamine, la vinylimidazoline, le vinylimidazole, le vinylimidazolinium, le méthacrylate de diméthylaminoéthyle, le diméthylaminopropyle, le chlorure de méthacryloylaminopropyl lauryldimonium, une silicone à fonctionnalisation amino, et l'éthylèneimine.

9. Agrégat selon l'une quelconque des revendications 1-8, dans lequel chacune parmi les particules bénéfiques est une capsule ayant un coeur qui contient une matière active et une paroi qui encapsule la matière active, la matière active étant une fragrance, la capsule ayant une taille de particule allant de 0,1 à 50 µm, et la paroi de la capsule étant formée par un polymère d'urée-formaldéhyde, un polymère de mélamine-formaldéhyde, un polymère phénolique-formaldéhyde, un polymère d'urée-glutaraldéhyde, un polymère de mélamine-glutaraldéhyde, un polymère phénolique-glutaraldéhyde, une polyurée, un polyuréthane, un polyacrylate, un polyamide, un polyester, un polymère à réticulation époxy, un polymère à réticulation carbodiimide polyfonctionnel, de la silice, un matériau dérivé de silice, ou une combinaison de ceux-ci.

10. Agrégat selon la revendication 9, dans lequel chacune parmi les capsules est négativement chargée et se lie à l'un des polymères de dépôt via une liaison hydrogène, une interaction dipolaire, ou une interaction ionique, et l'un des polymères liants se lie à l'un des polymères de dépôt via une interaction ionique.

11. Agrégat selon la revendication 9, dans lequel la capsule est positivement chargée et se lie à l'un des polymères liants via des interactions ioniques, et l'un des polymères liants se lie à l'un des polymères de dépôt de même via une interaction ionique.

12. Agrégat selon l'une quelconque des revendications précédentes, comprenant en outre un stabilisateur de viscosité, et où le polymère liant ou de dépôt fait partie de la paroi de la capsule.

13. Procédé de préparation d'un agrégat, le procédé comprenant les étapes consistant à :
(a) fournir une dispersion de particules bénéfiques contenant des particules bénéfiques dans de l'eau, chacune parmi les particules bénéfiques contenant une matière active et un matériau polymère qui immobilise la matière active ;
(b) ajouter un ou plusieurs polymères liants et un ou plusieurs polymères de dépôt afin d'obtenir un mélange polymère, chacun parmi les polymères liants ayant un groupement chimique anionique qui est négativement chargé ou capable d'être négativement chargé, et chacun parmi les polymères de dépôt ayant un groupement chimique cationique qui est positivement chargé ou capable d'être positivement chargé ; et
(c) provoquer la formation de l'agrégat dans le mélange polymère induite par un évènement de coacervation, l'évènement de coacervation étant un ajustement de pH, un chauffage, une irradiation par des UV, un traitement ultrasonore, une radio-irradiation, une ionisation, une exposition à une enzyme ou une bactérie, une addition de sel ou de non-solvant, une réaction chimique, ou une combinaison de ceux-ci,
où chacune parmi les particules bénéfiques est une capsule, la matière active est choisie dans le groupe constitué par une fragrance, un agent aromatisant, un fongicide, un azurant optique, un agent antistatique, un agent antirides, un principe actif d'assouplissant textile, un principe actif de nettoyage des surfaces dures, un agent de conditionnement de la peau, un agent de conditionnement des cheveux, un principe actif antimicrobien, un agent de protection contre les UV, un répulsif pour insectes, un répulsif pour animaux, un répulsif pour vermines, un agent ignifuge, et un mélange de ceux-ci ; chacun parmi les polymères liants et les polymères de dépôt possède un poids moléculaire allant de 10 000 à 1 000 000 Daltons ; chacun parmi les un ou plusieurs polymères liants, indépendamment, est un polymère ou copolymère d'un monomère choisi dans le groupe constitué par l'acide acrylique, un acrylate, l'acide méthacrylique, un méthacrylate, l'acrylamide, l'acide polyacrylique, un polyacrylate, l'acide polyméthacrylique, un polyméthacrylate, le polyacrylamide, l'anhydride maléique, un monomère sulfaté, et un monomère phénolique ; et chacun parmi les un ou plusieurs polymères de dépôt, indépendamment, est un polymère ou copolymère d'un monomère choisi dans le groupe constitué par le chlorure de diallyldiméthylammonium, le chlorure de méthacrylamidopropyl triméthylammonium, le méthacrylate de N,N-diméthylaminoéthyle, la vinylpyridine, la vinylpyridine quaternisée, la vinylamine, l'allylamine, la vinylimidazoline, le vinylimidazole, le vinylimidazolinium, le méthacrylate de diméthylaminoéthyle, le diméthylaminopropyle, le chlorure de méthacryloylaminopropyl lauryldimonium, une silicone à fonctionnalisation amino, et l'éthylèneimine.

14. Bien de consommation contenant un agrégat selon l'une quelconque des revendications 1-12, éventuellement le bien de consommation comprenant en outre un ou plusieurs agrégats différents, une matière active libre, une ou plusieurs capsules libres, ou une combinaison de ceux-ci ; et où le bien de consommation est un shampooing, un après-shampooing, un produit de toilette, un détergent pour textiles, un assouplissant textile, un conditionneur pour textiles, un nettoyant pour surfaces dures, une lotion nettoyante pour le corps, un pain de savon, un amplificateur de senteur, un détergent liquide, ou un détergent en poudre.
